# EUROPÄISCHE PATENTSCHRIFT

(11) **EP 0 967 867 B1**
(45) Veröffentlichungstag und Bekanntmachung des Hinweises auf die Patenterteilung: **02.01.2003**
(21) Anmeldenummer: 98905421.8
(22) Anmeldetag: 12.02.1998
(51) Int. Cl.: A01N 33/24, C07C 259/02

(54) **HYDROXIMSÄUREHALOGENIDE, VERFAHREN ZU IHRER HERSTELLUNG UND IHRE VERWENDUNG**
HYDROXIMIC ACID HALOGENIDES, METHOD FOR THE PRODUCTION AND USE THEREOF
HALOGENURES DE L'ACIDE HYDROXIMIQUE, LEUR FABRICATION ET LEUR UTILISATION

(30) Priorität: 05.03.1997 DE 19708940
(43) Veröffentlichungstag der Anmeldung: 05.01.2000
(73) Patentinhaber: BASF AKTIENGESELLSCHAFT, 67056 Ludwigshafen (DE)
(72) Erfinder: BAYER, Herbert, D-68159 Mannheim (DE); SAUTER, Hubert, D-68167 Mannheim (DE); MÜLLER, Bernd, D-67227 Frankenthal (DE); GRAMMENOS, Wassilios, D-67063 Ludwigshafen (DE); KIRSTGEN, Reinhard, D-67434 Neustadt (DE); GYPSER, Andreas, D-68159 Mannheim (DE); PTOCK, Arne, D-67067 Ludwigshafen (DE); GROTE, Thomas, D-67105 Schifferstadt (DE); RÖHL, Franz, D-67105 Schifferstadt (DE); RACK, Michael, D-69123 Heidelberg (DE); GÖTZ, Roland, D-91541 Rothenburg (DE); LORENZ, Gisela, D-67434 Neustadt (DE); AMMERMANN, Eberhard, D-64646 Heppenheim (DE); STRATHMANN, Siegfried, D-67117 Limburgerhof (DE); HARRIES, Volker, D-67227 Frankenthal (DE)
(74) Vertreter: Fitzner, Ulrich, Dr.
(86) Internationale Anmeldenummer: EP9800782
(87) Internationale Veröffentlichungsnummer: WO98038857

(56) Entgegenhaltungen:
- WO-A-95/21153
- WO-A-95/21154

## Beschreibung

Die vorliegende Erfindung betrifft Hydroximsäurehalogenide der Formel I 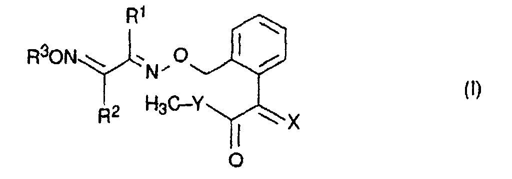 in der die Substituenten die folgende Bedeutung haben:
- X: NOCH₃, CHOCH₃ oder CHCH₃;
- Y: O oder NH;
- R¹: Halogen;
- R²: C₂-C₆-Alkyl, C₂-C₆-Alkenyl und C₂-C₆-Alkinyl, wobei diese Gruppen partiell oder vollständig halogeniert sein können und/oder einen oder zwei der folgenden Reste tragen können: Cyano, C₁-C₄-Alkoxy, C₁-C₄-Halogenalkoxy und Phenyl, welches seinerseits partiell oder vollständig halogeniert sein kann und/oder eine bis drei der folgenden Gruppen tragen kann: Cyano, Nitro, C₁-C₄-Alkyl, C₁-C₄-Halogenalkyl, C₁-C₄-Alkoxy und C₁-C₄-Halogenalkoxy;
Methyl, welches partiell oder vollständig halogeniert ist und/oder einen der folgenden Reste trägt: Cyano, C₁-C₄-Alkoxy oder C₁-C₄-Halogenalkoxy;
C₅-C₆-Cycloalkyl, welches partiell oder vollständig halogeniert sein kann und/oder eine bis drei C₁-C₄-Alkylgruppen tragen kann;
Aryl oder Arylmethylen, welches im Arylteil partiell oder vollständig halogeniert sein kann und/oder einen bis drei der folgenden Reste tragen kann: Cyano, Nitro, C₁-C₄-Alkyl, C₁-C₄-Halogenalkyl, C₁-C₄-Alkoxy und C₁-C₄-Halogenalkoxy;
- R³: C₁-C₆-Alkyl, C₁-C₃-Alkoxy-C₁-C₆-alkyl, C₃-C₆-Cycloalkyl-C₁-C₆-alkyl, C₃-C₆-Alkenyl und C₃-C₆-Alkinyl, wobei diese Gruppen partiell oder vollständig halogeniert sein können und wobei die cycloalkylgruppen außerdem eine bis drei C₁-C₄-Alkylreste tragen können,
sowie ihre Salze.

Außerdem betrifft die Erfindung Verfahren zur Herstellung der Verbindungen I, sie enthaltende Mittel sowie deren Verwendung zur Bekämpfung von tierischen Schädlingen und Schadpilzen.

Aus der Literatur sind Phenylessigsäurederivate mit Wirkung gegen tierische Schädlinge und Schadpilze bekannt, deren allgemeine Struktur die Struktur der vorliegenden Verbindungen umfaßt (WO-A 95/21,153; WO-A 95/21,154). Außerdem werden in der WO-A 95/18,789 Verbindungen ähnlicher Strukturen mit Wirkung gegen tierische Schädlinge und Schadpilze beschrieben.

Der vorliegenden Erfindung lagen demgegenüber Verbindungen mit verbesserten Wirkungseigenschaften als Aufgabe zugrunde.

Demgemäß wurden die eingangs definierten Verbindungen I gefunden. Außerdem wurden Verfahren und Zwischenprodukte zu ihrer Herstellung sowie sie enthaltende Mittel und ihre Verwendung gegen tierische Schädlinge und Schadpilze gefunden.

Die vorliegenden Verbindungen I unterscheiden sich von den aus WO-A 95/21,153 und WO-A 95/21,154 bekannten Verbindungen durch die besondere Kombination der Gruppen R¹ bis R³. Insbesondere wurde gefunden, daß Verbindungen des bekannten Strukturtyps eine verbesserte Wirkung zeigen, wenn in der Position des Restes R¹ ein Halogenatom und in der Position des Restes R² eine sterisch anspruchsvolle Gruppe gebunden ist, die die Lipophilie der Verbindung erhöht, und die Position des Restes R³ nicht von Wasserstoff eingenommen wird.

Die Verbindungen I können im allgemeinen nach den in der eingangs zitierten Literatur beschriebenen Verfahren erhalten werden.

Besonders vorteilhaft erhält man die Verbindungen I dadurch, daß man einen Carbonsäureester IIa zunächst mit Hydroxylamin in die entsprechende Hydroxamsäure IIc überführt, IIc anschließend mit einer Benzylverbindung IIIa zum entsprechenden Hydroxamsäureester IV umsetzt und IV mit einem Halogenierungsmittel *[HAL]* in I überführt.

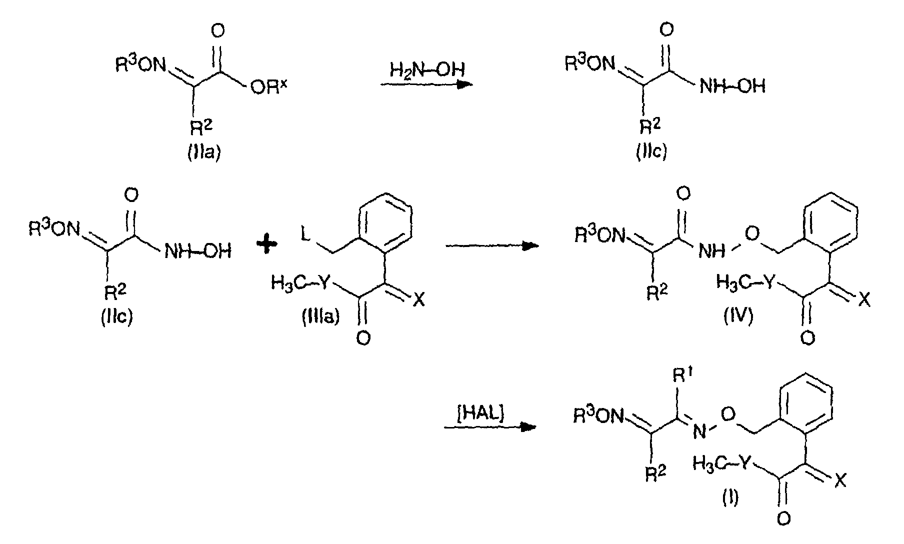

R^{x} in der Formel IIa steht für den Rest einer üblichen Abgangsgruppe. Unter üblichen Abgangsgruppen im Sinne dieser Umsetzung sind besonders die folgenden Gruppen zu verstehen: C₁-C₄-Alkyl (besonders Methyl oder Ethyl) oder Phenyl.

L in der Formel IIIa steht für eine nucleofuge Abgangsgruppe. Im Sinne dieser Umsetzung sind darunter besonders die Folgenden zu verstehen: Halogen oder Alkyl- oder Arylsulfonat, besonders Chlor, Brom, Iod, Mesylat, Tosylat und Triflat.

Die Umsetzung des Carbonsäureesters IIa mit Hydroxylamin erfolgt üblicherweise bei Temperaturen von -20°C bis 50°C, vorzugsweise 0°C bis 20°C, in einem inerten organischen Lösungsmittel, bevorzugt in Gegenwart einer Base (vgl. Lit. Houben-Weyl, 4. Auflage, Bd. ES, S. 1141 ff.).

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Alkohole wie Methanol und Ethanol. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetallund Erdalkalimetalloxide wie Lithiumoxid, Natriumoxid, Calziumoxid und Magnesiumoxid, Alkalimetall- und Erdalkalimetallcarbonate wie Lithiumcarbonat und Calziumcarbonat sowie Alkalimetallund Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Alkalimetallhydroxide wie Natrium- und Kaliumhydroxid, sowie Alkalimetallalkoholate wie Natriummethanolat und Natriumethanolat.

Die Basen werden im allgemeinen äquimolar oder im Überschuß verwendet.

Die Edukte werden im allgemeinen in äquimolaren Mengen miteinander umgesetzt. Es kann für die Ausbeute vorteilhaft sein, Hydroxylamin in einem Überschuß bezogen auf IIa einzusetzen.

Die für die Herstellung der Verbindungen I benötigten Carbonsäureester IIa sind in der Literatur bekannt [DE-A 28 08 317; DE-A 22 65 234; J. Chem. Soc. PT 1, 2340 ff. (1975); Chem. Ber. 16, 2987 ff. (1883); J. Org. Chem. 37, 139 (1972)] oder können gemäß der zitierten Literatur hergestellt werden.

Die Umsetzung der Hydroxamsäure IIc mit der Benzylverbindung IIIa erfolgt üblicherweise bei Temperaturen von 0°C bis 130°C, vorzugsweise 10°C bis 60°C, in einem inerten organischen Lösungsmittel in Gegenwart einer Base [vgl. Lit. Liebigs Ann. Chem. 1992, 997 ff.; Synth. Commun. 19, 339 ff. (1989)].

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid, Dimethylformamid und Dimethylacetamid, besonders bevorzugt Tetrahydrofuran, Acetonitril und Dimethylformamid. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetallund Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat, Kaliumcarbonat und Calziumcarbonat sowie Alkalimetall hydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriummethanolat, Kaliumcarbonat und Natriumhydrid.

Die Basen werden im allgemeinen äquimolar, im Überschuß oder gegebenenfalls als Lösungsmittel verwendet.

Die Edukte werden im allgemeinen in äquimolaren Mengen miteinander umgesetzt. Es kann für die Ausbeute vorteilhaft sein, IIc in einem Überschuß bezogen auf IIIa einzusetzen.

Die für diese Umsetzung benötigten Benzylverbindungen sind aus der eingangs zitierten Literatur bekannt oder können gemäß dieser Literatur hergestellt werden.

Diese Halogenierung der Hydroxamsäureester IV erfolgt üblicherweise bei Temperaturen von -20°C bis 100°C, vorzugsweise -10°C bis 80°C, in einem inerten organischen Lösungsmittel [vgl. Lit. Houben-Weyl, 4. Aufl. Bd. E5, S. 631 ff.; J. Org. Chem. 36, 233 (1971); Synthesis 9, 750 ff. (1991); Tetrahedron 52(1), 233 ff. (1996)].

Als Halogenierungsmittel bei dieser Umsetzung eignen sich die üblichen anorganischen und organischen Halogenierungsmittel, z.B. Thionylchlorid, Oxalylchlorid, Phosphortribromid, Phosphortrichlorid, Phosphorpentachlorid, Phosphoroxychlorid, Phosphortriiodid, Triphenylphosphin/CCl₄, Triphenylphosphin/CBr₄, Triphenylphosphin/Iod, vorzugsweise Thionylchlorid oder die genannten Triphenylphosphin-Reagentien.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid, Dimethylformamid und Dimethylacetamid, besonders bevorzugt Acetonitril, Toluol und Tetrahydrofuran. Es können auch Gemische der genannten Lösungsmittel verwendet werden.

Die Halogenierungsmittel werden im allgemeinen mindestens in äquimolaren Mengen eingesetzt. Es kann für die Ausbeute vorteilhaft sein, sie in einem Überschuß von bis zu 10 mol bezogen auf 1 mol IV, vorzugsweise bis zu 5 mol, insbesondere bis zu 3 mol, einzusetzen.

Die Verbindungen IV können alternativ auch dadurch erhalten werden, daß man eine Carbonsäure IIb mit einem Benzylhydroxylamin IIIb umsetzt.

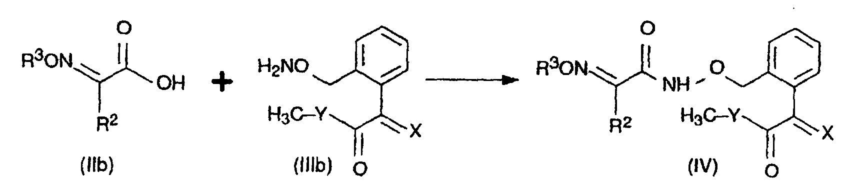

Diese Umsetzung erfolgt üblicherweise bei Temperaturen von -10°C bis 120°C, vorzugsweise 0°C bis 50°C, in einem inerten organischen Lösungsmittel in Gegenwart eines Aktivierungsreagens [vgl. Lit. Houben-Weyl, 4. Aufl. Bd. E5 S. 1141 ff.; J. Antibiot. 39, 1382 (1986)].

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid, Dimethylformamid und Dimethylacetamid, besonders bevorzugt Tetrahydrofuran und Methylenchlorid. Es können auch Gemische der genannten Lösungsmittel verwendet werden.

Als Aktivierungsreagentien eignen sich Säurehalogenidbildner wie Phosgen, Phosphortribromid, Phosphortrichlorid, Phosphorpentachlorid, Phosphoroxychlorid, Thionylchlorid oder Oxalylchlorid; Anhydridbildner wie Chlorameisensäureethylester oder Methansulfonylchlorid; Carbodiimide wie N,N'-Dicyclohexylcarbodiimid oder andere übliche Mittel wie N,N'-Carbonyldiimidazol oder Triphenylphosphin in CCl₄. Besonders bevorzugt werden Thionylchlorid, Oxalylchlorid und N,N'-Carbonyldiimidazol.

Die Aktivierungsreagentien werden im allgemeinen äquimolar oder im Überschuß verwendet.

Die Edukte werden im allgemeinen in äquimolaren Mengen miteinander umgesetzt. Es kann für die Ausbeute vorteilhaft sein, IIb in einem Überschuß bezogen auf IIIb einzusetzen.

Die für diese Umsetzung benötigten Carbonsäuren IIb sind aus der Literatur bekannt [J. Pharm. Sci. 57, 688 ff. (1968); DE-A 22 23 375; DE-A 22 65 234] oder können gemäß der zitierten Literatur hergestellt werden.

Außerdem erhält man die Verbindungen IV auch dadurch, daß man einen Carbonsäureester der Formal IIa unter den vorstehend für die Umsetzung von IIa zu IIc beschriebenen Bedingungen mit dem Benzylhydroxylamin IIIb umsetzt.

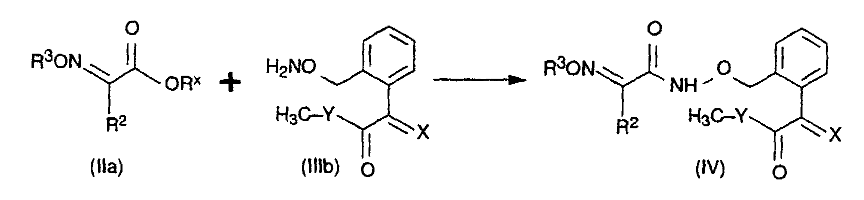

Nach einem weiteren Verfahren erhält man die Verbindungen I vorteilhaft, indem man ein Amidoxim IId mit einer Benzylverbindung IIIa in die entsprechende Verbindung der Formel v überführt und die Aminogruppe von V im Wege einer Diazotierung gegen Halogen austauscht.

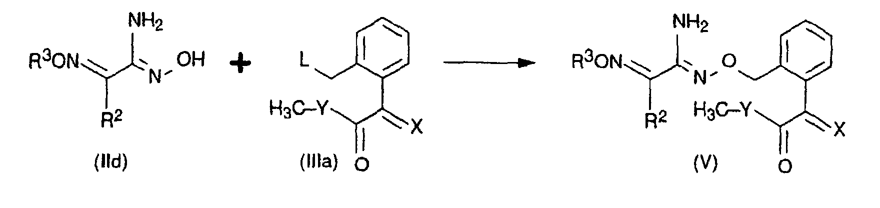

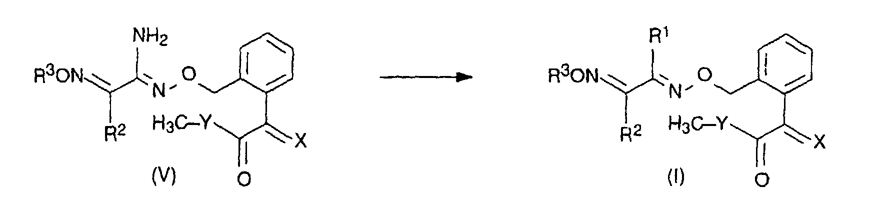

Die Umsetzung des Amidoxims IId mit der Benzylverbindung IIIa erfolgt üblicherweise bei Temperaturen von 0°C bis 130°C, vorzugsweise 10°C bis 60°C, in einem inerten organischen Lösungsmittel in Gegenwart einer Base [vgl. Lit. Heterocycles 36, 1027 ff. (1993)].

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Tetrahydrofuran, Acetonitril und Dimethylformamid. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetallund Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat, Kaliumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriummethanolat, Kaliumcarbonat und Natriumhydrid.

Die Basen werden im allgemeinen äquimolar oder im Überschuß verwendet.

Die Edukte werden im allgemeinen in äquimolaren Mengen miteinander umgesetzt. Es kann für die Ausbeute vorteilhaft sein, IId in einem Überschuß bezogen auf IIIa einzusetzen.

Die für diese Herstellung der Verbindungen I benötigten Amidoxime IId sind in der Literatur bekannt [DE-A 44 42 732; Gazz. Chim. Ital. 55, 327 (1925)] oder können gemäß der zitierten Literatur hergestellt werden.

Die Diazotierung und Halogenierung von V zu I erfolgt üblicherweise bei Temperaturen von -20°C bis 50°C, vorzugsweise 0°C bis 20°C, in Wasser oder in einem wäßrigen inerten organischen Lösungsmittel [vgl. Lit. J. Org. Chem. 45, 4144 ff. (1980); Chem. Ber. 26, 1567 ff. (1893)].

Als Halogenierungsmittel dienen in dieser Umsetzung Fluorwaserstoff. Chlorwasserstoff, Bromwasserstoff und Iodwasserstoff, insbesondere Chlorwasserstoff.

Die Halogenierungsmittel werden im allgemeinen im Überschuß oder gegebenenfalls als Lösungsmittel verwendet.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt sind neben Wasser Gemische von Dioxan und Wasser und/oder Tetrahydrofuran und Wasser.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Verbindungen I können bei ihrer Herstellung aufgrund ihrer C=C- und C=N-Doppelbingungen als E/Z-Isomerengemische anfallen, wobei diese Gemische in üblicher Weise, z.B. durch Kristallisation oder Chromatographie, in die reinen Isomere aufgetrennt werden können.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder dem tierischen Schädling erfolgen.

In Bezug auf die C=X-Doppelbindung werden hinsichtlich ihrer Wirksamkeit die E-Isomere der Verbindungen I bevorzugt (Konfiguration bezogen auf die OCH₃ bzw. CH₃-Gruppe im Verhältnis zur COYCH₃-Gruppe).

In Bezug auf die CR²=NOR³ Doppelbindung werden im allgemeinen hinsichtlich ihrer Wirksamkeit die cis-Isomere der Verbindungen I (Konfiguration bezogen auf den Rest R² im Verhältnis zur OR³-Gruppe) bevorzugt.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**Alkyl:** gesättigte, geradkettige oder verzweigte Kohlenwasserstoffreste mit 1 bis 4 oder 6 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 2,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Halogenalkyl:** geradkettige oder verzweigte Alkylgruppen mit 1 bis 4 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 3 oder 4 Kohlenstoffatomen (wie vorstehend genannt), welche über ein Sauerstoffatom (-O-) an das Gerüst gebunden sind;
**Halogenalkoxy:** geradkettige oder verzweigte Halogenalkylgruppen mit 1 bis 4 Kohlenstoffatomen (wie vorstehend genannt), welche über ein Sauerstoffatom (-O-) an das Gerüst gebunden sind;
**Alkenyl:** ungesättigte, geradkettige oder verzweigte Kohlenwasserstoffreste mit 2 bis 6 Kohlenstoffatomen und einer Doppelbindung in einer beliebigen Position, z.B. Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-lpropenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-lpropenyl und l-Ethyl-2-methyl-2-propenyl;
**Alkinyl:** geradkettige oder verzweigte Kohlenwasserstoffgruppen mit 2 bis 6 Kohlenstoffatomen und einer Dreifachbindung in einer beliebigen Position, z.B. Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Cycloalkyl:** monocyclische, gesättigte Kohlenwasserstoffgruppen mit 3 bis 6 oder 5 bis 6 Kohlenstoffririggliedern, z.B. Cyclopropyl, Cyclobutyl, Cyclopentyl und Cyclohexyl;
**Aryl:** ein ein- bis dreikerniges aromatisches Ringsystem enthaltend 6 bis 14 Kohlenstoffringglieder, z.B. Phenyl, Naphthyl und Anthracenyl.

Die Angabe *"partiell oder vollständig halogeniert"* soll zum Ausdruck bringen, daß in den entsprechend charakterisierten Gruppen die Wasserstoffatome der Kohelnwasserstoffreste zum Teil oder vollständig durch Halogenatome wie vorstehend genannt, besonders Fluor, Chlor oder Brom, insbesondere Fluor oder Chlor, ersetzt sein können.

Im Hinblick auf ihre biologische Wirkung sind Verbindungen I bevorzugt, in denen Y Sauerstoff bedeutet und X für NOCH₃, CHOCH₃ oder CHCH₃ steht.

Gleichermaßen sind Verbindungen I bevorzugt, in denen Y NH bedeutet und X für NOCH₃ steht.

Außerdem werden Verbindungen I bevorzugt, in denen R¹ für Chlor oder Brom, insbesondere Chlor, steht.

Desweiteren werden Verbindungen I bevorzugt, in denen R² für eine der folgenden Gruppen steht:
- C₂-C₆-Alkyl, welches partiell oder vollständig halogeniert sein kann und/oder einen oder zwei der folgenden Reste tragen kann: Cyano, C₁-C₄-Alkoxy, C₁-C₄-Halogenalkoxy und Phenyl, welches seinerseits partiell oder vollständig halogeniert sein kann und/oder eine bis drei der folgenden Gruppen tragen kann: Cyano, Nitro, C₁-C₄-Alkyl, C₁-C₄-Halogenalkyl, C₁-C₄-Alkoxy und C₁-C₄-Halogenalkoxy;
- Phenyl oder Benzyl, welches im Arylteil partiell oder vollständig halogeniert sein kann und/oder einen bis drei der folgenden Reste tragen kann: Cyano, Nitro, C₁-C₄-Alkyl, C₁-C₄-Halogenalkyl, C₁-C₄-Alkoxy und C₁-C₄-Halogenalkoxy.

Insbesondere werden Verbindungen I bevorzugt, in denen R² für eine der folgenden Gruppen steht:
- C₂-C₆-Alkyl, welches partiell oder vollständig halogeniert sein kann und/oder einen oder zwei C₁-C₄-Alkoxyreste tragen kann;
- Phenyl oder Benzyl, welches im Arylteil partiell oder vollständig halogeniert sein kann und/oder einen bis drei der folgenden Reste tragen kann: Cyano, C₁-C₄-Alkyl, C₁-C₄-Halogenalkyl, C₁-C₄-Alkoxy und C₁-C₄-Halogenalkoxy.

Desweiteren werden Verbindungen I bevorzugt, in denen R³ für eine der folgenden Gruppen steht:
- C₁-C₆-Alkyl, C₁-C₃-Alkoxy-C₁-C₂-alkyl, C₃-C₆-Alkenyl und C₃-C₆-Alkinyl, wobei diese Gruppen partiell oder vollständig halogeniert sein können.

Insbesondere werden Verbindungen I bevorzugt, in denen R³ für eine der folgenden Gruppen steht:
- C₁-C₆-Alkyl, C₁-C₃-Alkoxy-C₁-C₂-alkyl, C₃-C₆-Alkenyl und C₃-C₆-Alkinyl.

Besonders bevorzugt sind insbesondere Verbindungen I, in denen R³ für C₁-C₃-Alkyl, C₁-C₃-Alkoxyethyl, C₃-C₄-Alkenyl oder C₃-C₄-Alkinyl steht,
Insbesondere werden Verbindungen I im Hinblick auf die biologische Wirkung bevorzugt, in denen die Substituenten die folgende Bedeutung haben:
- X: NOCH₃, CHOCH₃ oder CHCH₃;
- Y: O oder NH;
- R¹: Chlor oder Brom;
- R²: C₂-C₆-Alkyl;
Phenyl oder Benzyl, welches im Phenylteil partiell oder vollständig halogeniert sein kann und/oder einen bis drei der folgenden Reste tragen kann: Cyano, C₁-C₄-Alkyl, C₁-C₄-Halogenalkyl, C₁-C₄-Alkoxy und C₁-C₄-Halogenalkoxy;
- R³: C₁-C₆-Alkyl, C₃-C₆-Alkenyl und C₃-C₆-Alkinyl, wobei diese Gruppen partiell halogeniert sein können.

Im Hinblick auf ihre biologische Aktivität werden insbesondere die in den folgenden Tabellen zusammengestellten Verbindungen I bevorzugt. Die in den Tabellen für einen Substituenten genannten Gruppen stellen außerdem, für sich betrachtet, unabhängig von der Kombination in der sie genannt sind, eine besonders bevorzugte Ausgestaltung des betreffenden Substituenten dar.

### Tabelle 1

Verbindungen der allgemeinen Formel I.A, in denen R¹ für Chlor steht, R³ Methyl bedeutet und der Rest R² für eine Verbindung einer Gruppe der Tabelle A entspricht

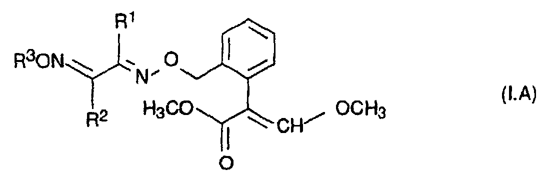

### Tabelle 2

Verbindungen der allgemeinen Formel I.B, in denen R¹ für Chlor steht, R³ Methyl bedeutet und der Rest R² für eine Verbindung einer Gruppe der Tabelle A entspricht

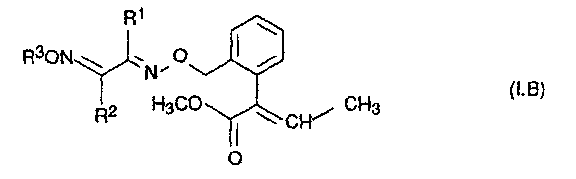

### Tabelle 3

Verbindungen der allgemeinen Formel I.C, in denen R¹ für Chlor steht, R³ Methyl bedeutet und der Rest R² für eine Verbindung einer Gruppe der Tabelle A entspricht

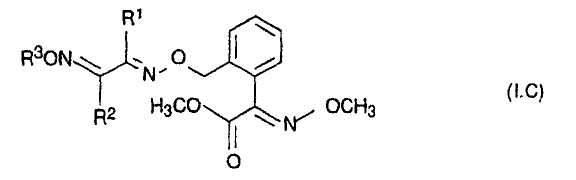

### Tabelle 4

Verbindungen der allgemeinen Formel I.D, in denen R¹ für Chlor steht, R³ Methyl bedeutet und der Rest R² für eine Verbindung einer Gruppe der Tabelle A entspricht

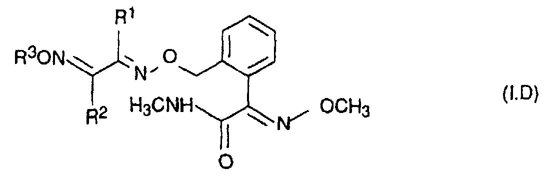

### Tabelle 5

Verbindungen der allgemeinen Formel I.A, in denen R¹ für Fluor steht, R³ Methyl bedeutet und der Rest R² für eine Verbindung einer Gruppe der Tabelle B entspricht

### Tabelle 6

Verbindungen der allgemeinen Formel I.B, in denen R¹ für Fluor steht, R³ Methyl bedeutet und der Rest R² für eine Verbindung einer Gruppe der Tabelle B entspricht

### Tabelle 7

Verbindungen der allgemeinen Formel I.C, in denen R¹ für Fluor steht, R³ Methyl bedeutet und der Rest R² für eine Verbindung einer Gruppe der Tabelle B entspricht

### Tabelle 8

Verbindungen der allgemeinen Formel I.D, in denen R¹ für Fluor steht, R³ Methyl bedeutet und der Rest R² für eine Verbindung einer Gruppe der Tabelle B entspricht

### Tabelle 9

Verbindungen der allgemeinen Formel I.A, in denen R¹ für Brom steht, R³ Methyl bedeutet und der Rest R² für eine Verbindung einer Gruppe der Tabelle A entspricht

### Tabelle 10

Verbindungen der allgemeinen Formel I.B, in denen R¹ für Brom steht, R³ Methyl bedeutet und der Rest R² für eine Verbindung einer Gruppe der Tabelle A entspricht

### Tabelle 11

Verbindungen der allgemeinen Formel I.C, in denen R¹ für Brom steht, R³ Methyl bedeutet und der Rest R² für eine Verbindung einer Gruppe der Tabelle A entspricht

### Tabelle 12

Verbindungen der allgemeinen Formel I.D, in denen R¹ für Brom steht, R³ Methyl bedeutet und der Rest R² für eine Verbindung einer Gruppe der Tabelle A entspricht

### Tabelle 13

Verbindungen der allgemeinen Formel I.A, in denen R¹ für Iod steht, R³ Methyl bedeutet und der Rest R² für eine Verbindung einer Gruppe der Tabelle B entspricht

### Tabelle 14

Verbindungen der allgemeinen Formel I.B, in denen R¹ für Iod steht, R³ Methyl bedeutet und der Rest R² für eine Verbindung einer Gruppe der Tabelle B entspricht

### Tabelle 15

Verbindungen der allgemeinen Formel I.C, in denen R¹ für Iod steht, R³ Methyl bedeutet und der Rest R² für eine Verbindung einer Gruppe der Tabelle B entspricht

### Tabelle 16

Verbindungen der allgemeinen Formel I.D, in denen R¹ für Iod steht, R³ Methyl bedeutet und der Rest R² für eine Verbindung einer Gruppe der Tabelle B entspricht

### Tabelle 17

Verbindungen der allgemeinen Formel I.A, in denen R¹ für Chlor steht, R³ Ethyl bedeutet und der Rest R² für eine Verbindung einer Gruppe der Tabelle A entspricht

### Tabelle 18

Verbindungen der allgemeinen Formel I.B, in denen R¹ für Chlor steht, R³ Ethyl bedeutet und der Rest R² für eine Verbindung einer Gruppe der Tabelle A entspricht

### Tabelle 19

Verbindungen der allgemeinen Formel I.C, in denen R¹ für Chlor steht, R³ Ethyl bedeutet und der Rest R² für eine Verbindung einer Gruppe der Tabelle A entspricht

### Tabelle 20

Verbindungen der allgemeinen Formel I.D, in denen R¹ für Chlor steht, R³ Ethyl bedeutet und der Rest R² für eine Verbindung einer Gruppe der Tabelle A entspricht

### Tabelle 21

Verbindungen der allgemeinen Formel I.A, in denen R¹ für Fluor steht, R³ Ethyl bedeutet und der Rest R² für eine Verbindung einer Gruppe der Tabelle B entspricht

### Tabelle 22

Verbindungen der allgemeinen Formel I.B, in denen R¹ für Fluor steht, R³ Ethyl bedeutet und der Rest R² für eine Verbindung einer Gruppe der Tabelle B entspricht

### Tabelle 23

Verbindungen der allgemeinen Formel I.C, in denen R¹ für Fluor steht, R³ Ethyl bedeutet und der Rest R² für eine Verbindung einer Gruppe der Tabelle B entspricht

### Tabelle 24

Verbindungen der allgemeinen Formel I.D, in denen R¹ für Fluor steht, R³ Ethyl bedeutet und der Rest R² für eine Verbindung einer Gruppe der Tabelle B entspricht

### Tabelle 25

Verbindungen der allgemeinen Formel I.A, in denen R¹ für Brom steht, R³ Ethyl bedeutet und der Rest R² für eine Verbindung einer Gruppe der Tabelle A entspricht

### Tabelle 26

Verbindungen der allgemeinen Formel I.B, in denen R¹ für Brom steht, R³ Ethyl bedeutet und der Rest R² für eine Verbindung einer Gruppe der Tabelle A entspricht

### Tabelle 27

Verbindungen der allgemeinen Formel I.C, in denen R¹ für Brom steht, R³ Ethyl bedeutet und der Rest R² für eine Verbindung einer Gruppe der Tabelle A entspricht

### Tabelle 28

Verbindungen der allgemeinen Formel I.D, in denen R¹ für Brom steht, R³ Ethyl bedeutet und der Rest R² für eine Verbindung einer Gruppe der Tabelle A entspricht

### Tabelle 29

Verbindungen der allgemeinen Formel I.A, in denen R¹ für Iod steht, R³ Ethyl bedeutet und der Rest R² für eine Verbindung einer Gruppe der Tabelle B entspricht

### Tabelle 30

Verbindungen der allgemeinen Formel I.B, in denen R¹ für Iod steht, R³ Ethyl bedeutet und der Rest R² für eine Verbindung einer Gruppe der Tabelle B entspricht

### Tabelle 31

Verbindungen der allgemeinen Formel I.C, in denen R¹ für Iod steht, R³ Ethyl bedeutet und der Rest R² für eine Verbindung einer Gruppe der Tabelle B entspricht

### Tabelle 32

Verbindungen der allgemeinen Formel I.D, in denen R¹ für Iod steht, R³ Ethyl bedeutet und der Rest R² für eine Verbindung einer Gruppe der Tabelle B entspricht

### Tabelle 33

Verbindungen der allgemeinen Formel I.A, in denen R¹ für Chlor steht, R³ Propyl bedeutet und der Rest R² für eine Verbindung einer Gruppe der Tabelle A entspricht

### Tabelle 34

Verbindungen der allgemeinen Formel I.B, in denen R¹ für Chlor steht, R³ Propyl bedeutet und der Rest R² für eine Verbindung einer Gruppe der Tabelle A entspricht

### Tabelle 35

Verbindungen der allgemeinen Formel I.C, in denen R¹ für Chlor steht, R³ Propyl bedeutet und der Rest R² für eine Verbindung einer Gruppe der Tabelle A entspricht

### Tabelle 36

verbindungen der allgemeinen Formel I.D, in denen R¹ für Chlor steht, R³ Propyl bedeutet und der Rest R² für eine Verbindung einer Gruppe der Tabelle A entspricht

### Tabelle 37

Verbindungen der allgemeinen Formel I.A, in denen R¹ für Fluor steht, R³ Propyl bedeutet und der Rest R² für eine Verbindung einer Gruppe der Tabelle B entspricht

### Tabelle 38

Verbindungen der allgemeinen Formel I.B, in denen R¹ für Fluor steht, R³ Propyl bedeutet und der Rest R² für eine Verbindung einer Gruppe der Tabelle B entspricht

### Tabelle 39

Verbindungen der allgemeinen Formel I.C, in denen R¹ für Fluor steht, R³ Propyl bedeutet und der Rest R² für eine Verbindung einer Gruppe der Tabelle B entspricht

### Tabelle 40

Verbindungen der allgemeinen Formel I.D, in denen R¹ für Fluor steht, R³ Propyl bedeutet und der Rest R² für eine Verbindung einer Gruppe der Tabelle B entspricht

### Tabelle 41

Verbindungen der allgemeinen Formel I.A, in denen R¹ für Brom steht, R³ Propyl bedeutet und der Rest R² für eine Verbindung einer Gruppe der Tabelle A entspricht

### Tabelle 42

Verbindungen der allgemeinen Formel I.B, in denen R¹ für Brom steht, R³ Propyl bedeutet und der Rest R² für eine Verbindung einer Gruppe der Tabelle A entspricht

### Tabelle 43

Verbindungen der allgemeinen Formel I.C, in denen R¹ für Brom steht, R³ Propyl bedeutet und der Rest R² für eine Verbindung einer Gruppe der Tabelle A entspricht

### Tabelle 44

Verbindungen der allgemeinen Formel I.D, in denen R¹ für Brom steht, R³ Propyl bedeutet und der Rest R² für eine Verbindung einer Gruppe der Tabelle A entspricht

### Tabelle 45

Verbindungen der allgemeinen Formel I.A, in denen R¹ für Iod steht, R³ Propyl bedeutet und der Rest R² für eine Verbindung einer Gruppe der Tabelle B entspricht

### Tabelle 46

Verbindungen der allgemeinen Formel I.B, in denen R¹ für Iod steht, R³ Propyl bedeutet und der Rest R² für eine Verbindung einer Gruppe der Tabelle B entspricht

### Tabelle 47

Verbindungen der allgemeinen Formel I.C, in denen R¹ für Iod steht, R³ Propyl bedeutet und der Rest R² für eine Verbindung einer Gruppe der Tabelle B entspricht

### Tabelle 48

Verbindungen der allgemeinen Formel I.D, in denen R¹ für Iod steht, R³ Propyl bedeutet und der Rest R² für eine Verbindung einer Gruppe der Tabelle B entspricht

### Tabelle 49

Verbindungen der allgemeinen Formel I.A, in denen R¹ für Chlor steht, R³ iso-Propyl bedeutet und der Rest R² für eine Verbindung einer Gruppe der Tabelle A entspricht

### Tabelle 50

Verbindungen der allgemeinen Formel I.B, in denen R¹ für Chlor steht, R³ iso-Propyl bedeutet und der Rest R² für eine Verbindung einer Gruppe der Tabelle A entspricht

### Tabelle 51

Verbindungen der allgemeinen Formel I.C, in denen R¹ für Chlor steht, R³ iso-Propyl bedeutet und der Rest R² für eine Verbindung einer Gruppe der Tabelle A entspricht

### Tabelie 52

Verbindungen der allgemeinen Formel I.D, in denen R¹ für Chlor steht, R³ iso-Propyl bedeutet und der Rest R² für eine Verbindung einer Gruppe der Tabelle A entspricht

### Tabelle 53

Verbindungen der allgemeinen Formel I.A, in denen R¹ für Fluor steht, R³ iso-Propyl bedeutet und der Rest R² für eine Verbindung einer Gruppe der Tabelle B entspricht

### Tabelle 54

Verbindungen der allgemeinen Formel I.B, in denen R¹ für Fluor steht, R³ iso-Propyl bedeutet und der Rest R² für eine Verbindung einer Gruppe der Tabelle B entspricht

### Tabelle 55

Verbindungen der allgemeinen Formel I.C, in denen R¹ für Fluor steht, R³ iso-Propyl bedeutet und der Rest R² für eine Verbindung einer Gruppe der Tabelle B entspricht

### Tabelle 56

Verbindungen der allgemeinen Formel I.D, in denen R¹ für Fluor steht, R³ iso-Propyl bedeutet und der Rest R² für eine Verbindung einer Gruppe der Tabelle B entspricht

### Tabelle 57

Verbindungen der allgemeinen Formel I.A, in denen R¹ für Brom steht, R³ iso-Propyl bedeutet und der Rest R² für eine Verbindung einer Gruppe der Tabelle A entspricht

### Tabelle 58

Verbindungen der allgemeinen Formel I.B, in denen R¹ für Brom steht, R³ iso-Propyl bedeutet und der Rest R² für eine Verbindung einer Gruppe der Tabelle A entspricht

### Tabelle 59

Verbindungen der allgemeinen Formel I.C, in denen R¹ für Brom steht, R³ iso-Propyl bedeutet und der Rest R² für eine Verbindung einer Gruppe der Tabelle A entspricht

### Tabelle 60

Verbindungen der allgemeinen Formel I.D, in denen R¹ für Brom steht, R³ iso-Propyl bedeutet und der Rest R² für eine Verbindung einer Gruppe der Tabelle A entspricht

### Tabelle 61

Verbindungen der allgemeinen Formel I.A, in denen R¹ für Iod steht, R³ iso-Propyl bedeutet und der Rest R² für eine Verbindung einer Gruppe der Tabelle B entspricht

### Tabelle 62

Verbindungen der allgemeinen Formel I.B, in denen R¹ für Iod steht, R³ iso-Propyl bedeutet und der Rest R² für eine Verbindung einer Gruppe der Tabelle B entspricht

### Tabelle 63

Verbindungen der allgemeinen Formel I.C, in denen R¹ für Iod steht, R³ iso-Propyl bedeutet und der Rest R² für eine Verbindung einer Gruppe der Tabelle B entspricht

### Tabelle 64

Verbindungen der allgemeinen Formel I.D, in denen R¹ für Iod steht, R³ iso-Propyl bedeutet und der Rest R² für eine Verbindung einer Gruppe der Tabelle B entspricht

### Tabelle 65

Verbindungen der allgemeinen Formel I.A, in denen R¹ für Chlor steht, R³ 2-Methoxyethyl bedeutet und der Rest R² für eine Verbindung einer Gruppe der Tabelle A entspricht

### Tabelle 66

Verbindungen der allgemeinen Formel I.B, in denen R¹ für Chlor steht, R³ 2-Methoxyethyl bedeutet und der Rest R² für eine Verbindung einer Gruppe der Tabelle A entspricht

### Tabelle 67

Verbindungen der allgemeinen Formel I.C, in denen R¹ für Chlor steht, R³ 2-Methoxyethyl bedeutet und der Rest R² für eine Verbindung einer Gruppe der Tabelle A entspricht

### Tabelle 68

Verbindungen der allgemeinen Formel I.D, in denen R¹ für Chlor steht, R³ 2-Methoxyethyl bedeutet und der Rest R² für eine Verbindung einer Gruppe der Tabelle A entspricht

### Tabelle 69

Verbindungen der allgemeinen Formel I.A, in denen R¹ für Fluor steht, R³ 2-Methoxyethyl bedeutet und der Rest R² für eine Verbindung einer Gruppe der Tabelle B entspricht

### Tabelle 70

Verbindungen der allgemeinen Formel I.B, in denen R¹ für Fluor steht, R³ 2-Methoxyethyl bedeutet und der Rest R² für eine Verbindung einer Gruppe der Tabelle B entspricht

### Tabelle 71

Verbindungen der allgemeinen Formel I.C, in denen R¹ für Fluor steht, R³ 2-Methoxyethyl bedeutet und der Rest R² für eine Verbindung einer Gruppe der Tabelle B entspricht

### Tabelle 72

Verbindungen der allgemeinen Formel I.D, in denen R¹ für Fluor steht, R³ 2-Methoxyethyl bedeutet und der Rest R² für eine Verbindung einer Gruppe der Tabelle B entspricht

### Tabelle 73

Verbindungen der allgemeinen Formel I.A, in denen R¹ für Brom steht, R³ 2-Methoxyethyl bedeutet und der Rest R² für eine Verbindung einer Gruppe der Tabelle A entspricht

### Tabelle 74

Verbindungen der allgemeinen Formel I.B, in denen R¹ für Brom steht, R³ 2-Methoxyethyl bedeutet und der Rest R² für eine Verbindung einer Gruppe der Tabelle A entspricht

### Tabelle 75

Verbindungen der allgemeinen Formel I.C, in denen R¹ für Brom steht, R³ 2-Methoxyethyl bedeutet und der Rest R² für eine Verbindung einer Gruppe der Tabelle A entspricht

### Tabelle 76

Verbindungen der allgemeinen Formel I.D, in denen R¹ für Brom steht, R³ 2-Methoxyethyl bedeutet und der Rest R² für eine Verbindung einer Gruppe der Tabelle A entspricht

### Tabelle 77

Verbindungen der allgemeinen Formel I.A, in denen R¹ für Iod steht, R³ 2-Methoxyethyl bedeutet und der Rest R² für eine Verbindung einer Gruppe der Tabelle B entspricht

### Tabelle 78

Verbindungen der allgemeinen Formel I.B, in denen R¹ für Iod steht, R³ 2-Methoxyethyl bedeutet und der Rest R² für eine Verbindung einer Gruppe der Tabelle B entspricht

### Tabelle 79

Verbindungen der allgemeinen Formel I.C, in denen R¹ für Iod steht, R³ 2-Methoxyethyl bedeutet und der Rest R² für eine Verbindung einer Gruppe der Tabelle B entspricht

### Tabelle 80

Verbindungen der allgemeinen Formel I.D, in denen R¹ für Iod steht, R³ 2-Methoxyethyl bedeutet und der Rest R² für eine Verbindung einer Gruppe der Tabelle B entspricht

### Tabelle 81

Verbindungen der allgemeinen Formel I.A, in denen R¹ für Chlor steht, R³ 2-Ethoxyethyl bedeutet und der Rest R² für eine Verbindung einer Gruppe der Tabelle A entspricht

### Tabelle 82

Verbindungen der allgemeinen Formel I.B, in denen R¹ für Chlor steht, R³ 2-Ethoxyethyl bedeutet und der Rest R² für eine Verbindung einer Gruppe der Tabelle A entspricht

### Tabelle 83

Verbindungen der allgemeinen Formel I.C, in denen R¹ für Chlor steht, R³ 2-Ethoxyethyl bedeutet und der Rest R² für eine Verbindung einer Gruppe der Tabelle A entspricht

### Tabelle 84

Verbindungen der allgemeinen Formel I.D, in denen R¹ für Chlor steht, R³ 2-Ethoxyethyl bedeutet und der Rest R² für eine Verbindung einer Gruppe der Tabelle A entspricht

### Tabelle 85

Verbindungen der allgemeinen Formel I.A, in denen R¹ für Fluor steht, R³ 2-Ethoxyethyl bedeutet und der Rest R² für eine Verbindung einer Gruppe der Tabelle B entspricht

### Tabelle 86

Verbindungen der allgemeinen Formel I.B, in denen R¹ für Fluor steht, R³ 2-Ethoxyethyl bedeutet und der Rest R² für eine Verbindung einer Gruppe der Tabelle B entspricht

### Tabelle 87

Verbindungen der allgemeinen Formel I.C, in denen R¹ für Fluor steht, R³ 2-Ethoxyethyl bedeutet und der Rest R² für eine Verbindung einer Gruppe der Tabelle B entspricht

### Tabelle 88

Verbindungen der allgemeinen Formel I.D, in denen R¹ für Fluor steht, R³ 2-Ethoxyethyl bedeutet und der Rest R² für eine Verbindung einer Gruppe der Tabelle B entspricht

### Tabelle 89

Verbindungen der allgemeinen Formel I.A, in denen R¹ für Brom steht, R³ 2-Ethoxyethyl bedeutet und der Rest R² für eine Verbindung einer Gruppe der Tabelle A entspricht

### Tabelle 90

Verbindungen der allgemeinen Formel I.B, in denen R¹ für Brom steht, R³ 2-Ethoxyethyl bedeutet und der Rest R² für eine Verbindung einer Gruppe der Tabelle A entspricht

### Tabelle 91

Verbindungen der allgemeinen Formel I.C, in denen R¹ für Brom steht, R³ 2-Ethoxyethyl bedeutet und der Rest R² für eine Verbindung einer Gruppe der Tabelle A entspricht

### Tabelle 92

Verbindungen der allgemeinen Formel I.D, in denen R¹ für Brom steht, R³ 2-Ethoxyethyl bedeutet und der Rest R² für eine Verbindung einer Gruppe der Tabelle A entspricht

### Tabelle 93

Verbindungen der allgemeinen Formel I.A, in denen R¹ für Iod steht, R³ 2-Ethoxyethyl bedeutet und der Rest R² für eine Verbindung einer Gruppe der Tabelle B entspricht

### Tabelle 94

Verbindungen der allgemeinen Formel I.B, in denen R¹ für Iod steht, R³ 2-Ethoxyethyl bedeutet und der Rest R² für eine Verbindung einer Gruppe der Tabelle B entspricht

### Tabelle 95

Verbindungen der allgemeinen Formel I.C, in denen R¹ für Iod steht, R³ 2-Ethoxyethyl bedeutet und der Rest R² für eine Verbindung einer Gruppe der Tabelle B entspricht

### Tabelle 96

Verbindungen der allgemeinen Formel I.D, in denen R¹ für Iod steht, R³ 2-Ethoxyethyl bedeutet und der Rest R² für eine Verbindung einer Gruppe der Tabelle B entspricht

### Tabelle 97

Verbindungen der allgemeinen Formel I.A, in denen R¹ für Chlor steht, R³ Cyclopropylmethyl bedeutet und der Rest R² für eine Verbindung einer Gruppe der Tabelle A entspricht

### Tabelle 98

Verbindungen der allgemeinen Formel I.B, in denen R¹ für Chlor steht, R³ Cyclopropylmethyl bedeutet und der Rest R² für eine Verbindung einer Gruppe der Tabelle A entspricht

### Tabelle 99

Verbindungen der allgemeinen Formel I.C, in denen R¹ für Chlor steht, R³ Cyclopropylmethyl bedeutet und der Rest R² für eine Verbindung einer Gruppe der Tabelle A entspricht

### Tabelle 100

Verbindungen der allgemeinen Formel I.D, in denen R¹ für Chlor steht, R³ Cyclopropylmethyl bedeutet und der Rest R² für eine Verbindung einer Gruppe der Tabelle A entspricht

### Tabelle 101

Verbindungen der allgemeinen Formel I.A, in denen R¹ für Fluor steht, R³ Cyclopropylmethyl bedeutet und der Rest R² für eine Verbindung einer Gruppe der Tabelle B entspricht

### Tabelle 102

Verbindungen der allgemeinen Formel I.B, in denen R¹ für Fluor steht, R³ Cyclopropylmethyl bedeutet und der Rest R² für eine Verbindung einer Gruppe der Tabelle B entspricht

### Tabelle 103

Verbindungen der allgemeinen Formel I.C, in denen R¹ für Fluor steht, R³ Cyclopropylmethyl bedeutet und der Rest R² für eine Verbindung einer Gruppe der Tabelle B entspricht

### Tabelle 104

Verbindungen der allgemeinen Formel I.D, in denen R¹ für Fluor steht, R³ Cyclopropylmethyl bedeutet und der Rest R² für eine Verbindung einer Gruppe der Tabelle B entspricht

### Tabelle 105

Verbindungen der allgemeinen Formel I.A, in denen R¹ für Brom steht, R³ Cyclopropylmethyl bedeutet und der Rest R² für eine Verbindung einer Gruppe der Tabelle A entspricht

### Tabelle 106

Verbindungen der allgemeinen Formel I.B, in denen R¹ für Brom steht, R³ Cyclopropylmethyl bedeutet und der Rest R² für eine Verbindung einer Gruppe der Tabelle A entspricht

### Tabelle 107

Verbindungen der allgemeinen Formel I.C, in denen R¹ für Brom steht, R³ Cyclopropylmethyl bedeutet und der Rest R² für eine Verbindung einer Gruppe der Tabelle A entspricht

### Tabelle 108

Verbindungen der allgemeinen Formel I.D, in denen R¹ für Brom steht, R³ Cyclopropylmethyl bedeutet und der Rest R² für eine Verbindung einer Gruppe der Tabelle A entspricht

### Tabelle 109

Verbindungen der allgemeinen Formel I.A, in denen R¹ für Iod steht, R³ Cyclopropylmethyl bedeutet und der Rest R² für eine Verbindung einer Gruppe der Tabelle B entspricht

### Tabelle 110

Verbindungen der allgemeinen Formel I.B, in denen R¹ für Iod steht, R³ Cyclopropylmethyl bedeutet und der Rest R² für eine Verbindung einer Gruppe der Tabelle B entspricht

### Tabelle 111

Verbindungen der allgemeinen Formel I.C, in denen R¹ für Iod steht, R³ Cyclopropylmethyl bedeutet und der Rest R² für eine Verbindung einer Gruppe der Tabelle B entspricht

### Tabelle 112

Verbindungen der allgemeinen Formel I.D, in denen R¹ für Iod steht, R³ Cyclopropylmethyl bedeutet und der Rest R² für eine Verbindung einer Gruppe der Tabelle B entspricht

### Tabelle 113

Verbindungen der allgemeinen Formel I.A, in denen R¹ für Chlor steht, R³ Allyl bedeutet und der Rest R² für eine Verbindung einer Gruppe der Tabelle A entspricht

### Tabelle 114

Verbindungen der allgemeinen Formel I.B, in denen R¹ für Chlor steht, R³ Allyl bedeutet und der Rest R² für eine Verbindung einer Gruppe der Tabelle A entspricht

### Tabelle 115

Verbindungen der allgemeinen Formel I.C, in denen R¹ für Chlor steht, R³ Allyl bedeutet und der Rest R² für eine Verbindung einer Gruppe der Tabelle A entspricht

### Tabelle 116

Verbindungen der allgemeinen Formel I.D, in denen R¹ für Chlor steht, R³ Allyl bedeutet und der Rest R² für eine Verbindung einer Gruppe der Tabelle A entspricht

### Tabelle 117

Verbindungen der allgemeinen Formel I.A, in denen R¹ für Fluor steht, R³ Allyl bedeutet und der Rest R² für eine Verbindung einer Gruppe der Tabelle B entspricht

### Tabelle 118

Verbindungen der allgemeinen Formel I.B, in denen R¹ für Fluor steht, R³ Allyl bedeutet und der Rest R² für eine Verbindung einer Gruppe der Tabelle B entspricht

### Tabelle 119

Verbindungen der allgemeinen Formel I.C, in denen R¹ für Fluor steht, R³ Allyl bedeutet und der Rest R² für eine Verbindung einer Gruppe der Tabelle B entspricht

### Tabelle 120

Verbindungen der allgemeinen Formel I.D, in denen R¹ für Fluor steht, R³ Allyl bedeutet und der Rest R² für eine Verbindung einer Gruppe der Tabelle B entspricht

### Tabelle 121

Verbindungen der allgemeinen Formel I.A, in denen R¹ für Brom steht, R³ Allyl bedeutet und der Rest R² für eine Verbindung einer Gruppe der Tabelle A entspricht

### Tabelle 122

Verbindungen der allgemeinen Formel I.B, in denen R¹ für Brom steht, R³ Allyl bedeutet und der Rest R² für eine Verbindung einer Gruppe der Tabelle A entspricht

### Tabelle 123

Verbindungen der allgemeinen Formel I.C, in denen R¹ für Brom steht, R³ Allyl bedeutet und der Rest R² für eine Verbindung einer Gruppe der Tabelle A entspricht

### Tabelle 124

Verbindungen der allgemeinen Formel I.D, in denen R¹ für Brom steht, R³ Allyl bedeutet und der Rest R² für eine Verbindung einer Gruppe der Tabelle A entspricht

### Tabelle 125

Verbindungen der allgemeinen Formel I.A, in denen R¹ für Iod steht, R³ Allyl bedeutet und der Rest R² für eine Verbindung einer Gruppe der Tabelle B entspricht

### Tabelle 126

Verbindungen der allgemeinen Formel I.B, in denen R¹ für Iod steht, R³ Allyl bedeutet und der Rest R² für eine Verbindung einer Gruppe der Tabelle B entspricht

### Tabelle 127

Verbindungen der allgemeinen Formel I.C, in denen R¹ für Iod steht, R³ Allyl bedeutet und der Rest R² für eine Verbindung einer Gruppe der Tabelle B entspricht

### Tabelle 128

Verbindungen der allgemeinen Formel I.D, in denen R¹ für Iod steht, R³ Allyl bedeutet und der Rest R² für eine Verbindung einer Gruppe der Tabelle B entspricht

### Tabelle 129

Verbindungen der allgemeinen Formel I.A, in denen R¹ für Chlor steht, R³ trans-3-Chlorallyl bedeutet und der Rest R² für eine Verbindung einer Gruppe der Tabelle A entspricht

### Tabelle 130

Verbindungen der allgemeinen Formel I.B, in denen R¹ für Chlor steht, R³ trans-3-Chlorallyl bedeutet und der Rest R² für eine Verbindung einer Gruppe der Tabelle A entspricht

### Tabelle 131

Verbindungen der allgemeinen Formel I.C, in denen R¹ für Chlor steht, R³ trans-3-Chlorallyl bedeutet und der Rest R² für eine Verbindung einer Gruppe der Tabelle A entspricht

### Tabelle 132

Verbindungen der allgemeinen Formel I.D, in denen R¹ für Chlor steht, R³ trans-3-Chlorallyl bedeutet und der Rest R² für eine Verbindung einer Gruppe der Tabelle A entspricht

### Tabelle 133

Verbindungen der allgemeinen Formel I.A, in denen R¹ für Fluor steht, R³ trans-3-Chlorallyl bedeutet und der Rest R² für eine Verbindung einer Gruppe der Tabelle B entspricht

### Tabelle 134

Verbindungen der allgemeinen Formel I.B, in denen R¹ für Fluor steht, R³ trans-3-Chlorallyl bedeutet und der Rest R² für eine Verbindung einer Gruppe der Tabelle B entspricht

### Tabelle 135

Verbindungen der allgemeinen Formel I.C, in denen R¹ für Fluor steht, R³ trend-3-Chlorallyl bedeutet und der Rest R² für eine Verbindung einer Gruppe der Tabelle B entspricht

### Tabelle 136

Verbindungen der allgemeinen Formel I.D, in denen R¹ für Fluor steht, R³ trans-3-Chlorallyl bedeutet und der Rest R² für eine Verbindung einer Gruppe der Tabelle B entspricht

### Tabelle 137

Verbindungen der allgemeinen Formel I.A, in denen R¹ für Brom steht, R³ trans-3-Chlorallyl bedeutet und der Rest R² für eine Verbindung einer Gruppe der Tabelle A entspricht

### Tabelle 138

Verbindungen der allgemeinen Formel I.B, in denen R¹ für Brom steht, R³ trans-3-Chlorallyl bedeutet und der Rest R² für eine Verbindung einer Gruppe der Tabelle A entspricht

### Tabelle 139

Verbindungen der allgemeinen Formel I.C, in denen R¹ für Brom steht, R³ trans-3-Chlorallyl bedeutet und der Rest R² für eine Verbindung einer Gruppe der Tabelle A entspricht

### Tabelle 140

Verbindungen der allgemeinen Formel I.D, in denen R¹ für Brom steht, R³ trans-3-Chlorallyl bedeutet und der Rest R² für eine Verbindung einer Gruppe der Tabelle A entspricht

### Tabelle 141

Verbindungen der allgemeinen Formel I.A, in denen R¹ für Iod steht, R³ trans-3-Chlorallyl bedeutet und der Rest R² für eine Verbindung einer Gruppe der Tabelle B entspricht

### Tabelle 142

Verbindungen der allgemeinen Formel I.B, in denen R¹ für Iod steht, R³ trans-3-Chlorallyl bedeutet und der Rest R² für eine Verbindung einer Gruppe der Tabelle B entspricht

### Tabelle 143

Verbindungen der allgemeinen Formel I.C, in denen R¹ für Iod steht, R³ trans-3-Chlorallyl bedeutet und der Rest R² für eine Verbindung einer Gruppe der Tabelle B entspricht

### Tabelle 144

Verbindungen der allgemeinen Formel I.D, in denen R¹ für Iod steht, R³ trans-3-Chlorallyl bedeutet und der Rest R² für eine Verbindung einer Gruppe der Tabelle B entspricht

### Tabelle 145

Verbindungen der allgemeinen Formel I.A, in denen R¹ für Chlor steht, R³ trans-Butenyl bedeutet und der Rest R² für eine Verbindung einer Gruppe der Tabelle A entspricht

### Tabelle 146

Verbindungen der allgemeinen Formel I.B, in denen R¹ für Chlor steht, R³ trans-Butenyl bedeutet und der Rest R² für eine Verbindung einer Gruppe der Tabelle A entspricht

### Tabelle 147

Verbindungen der allgemeinen Formel I.C, in denen R¹ für Chlor steht, R³ trans-Butenyl bedeutet und der Rest R² für eine Verbindung einer Gruppe der Tabelle A entspricht

### Tabelle 148

Verbindungen der allgemeinen Formel I.D, in denen R¹ für Chlor steht, R³ trans-Butenyl bedeutet und der Rest R² für eine Verbindung einer Gruppe der Tabelle A entspricht

### Tabelle 149

Verbindungen der allgemeinen Formel I.A, in denen R¹ für Fluor steht, R³ trans-Butenyl bedeutet und der Rest R² für eine Verbindung einer Gruppe der Tabelle B entspricht

### Tabelle 150

Verbindungen der allgemeinen Formel I.B, in denen R¹ für Fluor steht, R³ trans-Butenyl bedeutet und der Rest R² für eine Verbindung einer Gruppe der Tabelle B entspricht

### Tabelle 151

Verbindungen der allgemeinen Formel I.C, in denen R¹ für Fluor steht, R³ trans-Butenyl bedeutet und der Rest R² für eine Verbindung einer Gruppe der Tabelle B entspricht

### Tabelle 152

Verbindungen der allgemeinen Formel I.D, in denen R¹ für Fluor steht, R³ trans-Butenyl bedeutet und der Rest R² für eine Verbindung einer Gruppe der Tabelle B entspricht

### Tabelle 153

Verbindungen der allgemeinen Formel I.A, in denen R¹ für Brom steht, R³ trans-Butenyl bedeutet und der Rest R² für eine Verbindung einer Gruppe der Tabelle A entspricht

### Tabelle 154

Verbindungen der allgemeinen Formel I.B, in denen R¹ für Brom steht, R³ trans-Butenyl bedeutet und der Rest R² für eine Verbindung einer Gruppe der Tabelle A entspricht

### Tabelle 155

Verbindungen der allgemeinen Formel I.C, in denen R¹ für Brom steht, R³ trans-Butenyl bedeutet und der Rest R² für eine Verbindung einer Gruppe der Tabelle A entspricht

### Tabelle 156

Verbindungen der allgemeinen Formel I.D, in denen R¹ für Brom steht, R³ trans-Butenyl bedeutet und der Rest R² für eine Verbindung einer Gruppe der Tabelle A entspricht

### Tabelle 157

Verbindungen der allgemeinen Formel I.A, in denen R¹ für Iod steht, R³ trans-Butenyl bedeutet und der Rest R² für eine Verbindung einer Gruppe der Tabelle B entspricht

### Tabelle 158

Verbindungen der allgemeinen Formel I.B, in denen R¹ für Iod steht, R³ trans-Butenyl bedeutet und der Rest R² für eine Verbindung einer Gruppe der Tabelle B entspricht

### Tabelle 159

Verbindungen der allgemeinen Formel I.C, in denen R¹ für Iod steht, R³ trans-Butenyl bedeutet und der Rest R² für eine Verbindung einer Gruppe der Tabelle B entspricht

### Tabelle 160

Verbindungen der allgemeinen Formel I.D, in denen R¹ für Iod steht, R³ trans-Butenyl bedeutet und der Rest R² für eine Verbindung einer Gruppe der Tabelle B entspricht

### Tabelle 161

Verbindungen der allgemeinen Formel I.A, in denen R¹ für Chlor steht, R³ Propargyl bedeutet und der Rest R² für eine Verbindung einer Gruppe der Tabelle A entspricht

### Tabelle 162

Verbindungen der allgemeinen Formel I.B, in denen R¹ für Chlor steht, R³ Propargyl bedeutet und der Rest R² für eine Verbindung einer Gruppe der Tabelle A entspricht

### Tabelle 163

Verbindungen der allgemeinen Formel I.C, in denen R¹ für Chlor steht, R³ Propargyl bedeutet und der Rest R² für eine Verbindung einer Gruppe der Tabelle A entspricht

### Tabelle 164

Verbindungen der allgemeinen Formel I.D, in denen R¹ für Chlor steht, R³ Propargyl bedeutet und der Rest R² für eine Verbindung einer Gruppe der Tabelle A entspricht

### Tabelle 165

Verbindungen der allgemeinen Formel I.A, in denen R¹ für Fluor steht, R³ Propargyl bedeutet und der Rest R² für eine Verbindung einer Gruppe der Tabelle B entspricht

### Tabelle 166

Verbindungen der allgemeinen Formel I.B, in denen R¹ für Fluor steht, R³ Propargyl bedeutet und der Rest R² für eine Verbindung einer Gruppe der Tabelle B entspricht

### Tabelle 167

Verbindungen der allgemeinen Formel I.C, in denen R¹ für Fluor steht, R³ Propargyl bedeutet und der Rest R² für eine Verbindung einer Gruppe der Tabelle B entspricht

### Tabelle 168

Verbindungen der allgemeinen Formel I.D, in denen R¹ für Fluor steht, R³ Propargyl bedeutet und der Rest R² für eine Verbindung einer Gruppe der Tabelle B entspricht

### Tabelle 169

Verbindungen der allgemeinen Formel I.A, in denen R¹ für Brom steht, R³ Propargyl bedeutet und der Rest R² für eine Verbindung einer Gruppe der Tabelle A entspricht

### Tabelle 170

Verbindungen der allgemeinen Formel I.B, in denen R¹ für Brom steht, R³ Propargyl bedeutet und der Rest R² für eine Verbindung einer Gruppe der Tabelle A entspricht

### Tabelle 171

Verbindungen der allgemeinen Formel I.C, in denen R¹ für Brom steht, R³ Propargyl bedeutet und der Rest R² für eine Verbindung einer Gruppe der Tabelle A entspricht

### Tabelle 172

Verbindungen der allgemeinen Formel I.D, in denen R¹ für Brom steht, R³ Propargyl bedeutet und der Rest R² für eine Verbindung einer Gruppe der Tabelle A entspricht

### Tabelle 173

Verbindungen der allgemeinen Formel I.A, in denen R¹ für Iod steht, R³ Propargyl bedeutet und der Rest R² für eine Verbindung einer Gruppe der Tabelle B entspricht

### Tabelle 174

Verbindungen der allgemeinen Formel I.B, in denen R¹ für Iod steht, R³ Propargyl bedeutet und der Rest R² für eine Verbindung einer Gruppe der Tabelle B entspricht

### Tabelle 175

Verbindungen der allgemeinen Formel I.C, in denen R¹ für Iod steht, R³ Propargyl bedeutet und der Rest R² für eine Verbindung einer Gruppe der Tabelle B entspricht

### Tabelle 176

Verbindungen der allgemeinen Formel I.D, in denen R¹ für Iod steht, R³ Propargyl bedeutet und der Rest R² für eine Verbindung einer Gruppe der Tabelle B entspricht

### Tabelle 177

Verbindungen der allgemeinen Formel I.A, in denen R¹ für Chlor steht, R³ 3-Brompropargyl bedeutet und der Rest R² für eine Verbindung einer Gruppe der Tabelle A entspricht

### Tabelle 178

Verbindungen der allgemeinen Formel I.B, in denen R¹ für Chlor steht, R³ 3-Brompropargyl bedeutet und der Rest R² für eine Verbindung einer Gruppe der Tabelle A entspricht

### Tabelle 179

Verbindungen der allgemeinen Formel I.C, in denen R¹ für Chlor steht, R³ 3-Brompropargyl bedeutet und der Rest R² für eine Verbindung einer Gruppe der Tabelle A entspricht

### Tabelle 180

Verbindungen der allgemeinen Formel I.D, in denen R¹ für Chlor steht, R³ 3-Brompropargyl bedeutet und der Rest R² für eine Verbindung einer Gruppe der Tabelle A entspricht

### Tabelle 181

Verbindungen der allgemeinen Formel I.A, in denen R¹ für Fluor steht, R³ 3-Brompropargyl bedeutet und der Rest R² für eine Verbindung einer Gruppe der Tabelle B entspricht

### Tabelle 182

Verbindungen der allgemeinen Formel I.B, in denen R¹ für Fluor steht, R³ 3-Brompropargyl bedeutet und der Rest R² für eine Verbindung einer Gruppe der Tabelle B entspricht

### Tabelle 183

Verbindungen der allgemeinen Formel I.C, in denen R¹ für Fluor steht, R³ 3-Brompropargyl bedeutet und der Rest R² für eine Verbindung einer Gruppe der Tabelle B entspricht

### Tabelle 184

Verbindungen der allgemeinen Formel I.D, in denen R¹ für Fluor steht, R³ 3-Brompropargyl bedeutet und der Rest R² für eine Verbindung einer Gruppe der Tabelle B entspricht

### Tabelle 185

Verbindungen der allgemeinen Formel I.A, in denen R¹ für Brom steht, R³ 3-Brompropargyl bedeutet und der Rest R² für eine Verbindung einer Gruppe der Tabelle A entspricht

### Tabelle 186

Verbindungen der allgemeinen Formel I.B, in denen R¹ für Brom steht, R³ 3-Brompropargyl bedeutet und der Rest R² für eine Verbindung einer Gruppe der Tabelle A entspricht

### Tabelle 187

Verbindungen der allgemeinen Formel I.C, in denen R¹ für Brom steht, R³ 3-Brompropargyl bedeutet und der Rest R² für eine Verbindung einer Gruppe der Tabelle A entspricht

### Tabelle 188

Verbindungen der allgemeinen Formel I.D, in denen R¹ für Brom steht, R³ 3-Brompropargyl bedeutet und der Rest R² für eine Verbindung einer Gruppe der Tabelle A entspricht

### Tabelle 189

Verbindungen der allgemeinen Formel I.A, in denen R¹ für Iod steht, R³ 3-Brompropargyl bedeutet und der Rest R² für eine Verbindung einer Gruppe der Tabelle B entspricht

### Tabelle 190

Verbindungen der allgemeinen Formel I.B, in denen R¹ für Iod steht, R³ 3-Brompropargyl bedeutet und der Rest R² für eine Verbindung einer Gruppe der Tabelle B entspricht

### Tabelle 191

Verbindungen der allgemeinen Formel I.C, in denen R¹ für Iod steht, R³ 3-Brompropargyl bedeutet und der Rest R² für eine Verbindung einer Gruppe der Tabelle B entspricht

### Tabelle 192

Verbindungen der allgemeinen Formel I.D, in denen R¹ für Iod steht, R³ 3-Brompropargyl bedeutet und der Rest R² für eine Verbindung einer Gruppe der Tabelle B entspricht

### Tabelle 193

Verbindungen der allgemeinen Formel I.A, in denen R¹ für Chlor steht, R³ But-3-in-1-yl bedeutet und der Rest R² für eine Verbindung einer Gruppe der Tabelle A entspricht

### Tabelle 194

Verbindungen der allgemeinen Formel I.B, in denen R¹ für Chlor steht, R³ But-3-in-1-yl bedeutet und der Rest R² für eine Verbindung einer Gruppe der Tabelle A entspricht

### Tabelle 195

Verbindungen der allgemeinen Formel I.C, in denen R¹ für Chlor steht, R³ But-3-in-1-yl bedeutet und der Rest R² für eine Verbindung einer Gruppe der Tabelle A entspricht

### Tabelle 196

Verbindungen der allgemeinen Formel I.D, in denen R¹ für Chlor steht, R³ But-3-in-1-yl bedeutet und der Rest R² für eine Verbindung einer Gruppe der Tabelle A entspricht

### Tabelle 197

Verbindungen der allgemeinen Formel I.A, in denen R¹ für Fluor steht, R³ But-3-in-1-yl bedeutet und der Rest R² für eine Verbindung einer Gruppe der Tabelle B entspricht

### Tabelle 198

Verbindungen der allgemeinen Formel I.B, in denen R¹ für Fluor steht, R³ But-3-in-1-yl bedeutet und der Rest R² für eine Verbindung einer Gruppe der Tabelle B entspricht

### Tabelle 199

Verbindungen der allgemeinen Formel I.C, in denen R¹ für Fluor steht, R³ But-3-in-1-yl bedeutet und der Rest R² für eine Verbindung einer Gruppe der Tabelle B entspricht

### Tabelle 200

Verbindungen der allgemeinen Formel I.D, in denen R¹ für Fluor steht, R³ But-3-in-1-yl bedeutet und der Rest R² für eine Verbindung einer Gruppe der Tabelle B entspricht

### Tabelle 201

Verbindungen der allgemeinen Formel I.A, in denen R¹ für Brom steht, R³ But-3-in-1-yl bedeutet und der Rest R² für eine Verbindung einer Gruppe der Tabelle A entspricht

### Tabelle 202

Verbindungen der allgemeinen Formel I.B, in denen R¹ für Brom steht, R³ But-3-in-1-yl bedeutet und der Rest R² für eine Verbindung einer Gruppe der Tabelle A entspricht

### Tabelle 203

Verbindungen der allgemeinen Formel I.C, in denen R¹ für Brom steht, R³ But-3-in-1-yl bedeutet und der Rest R² für eine Verbindung einer Gruppe der Tabelle A entspricht

### Tabelle 204

Verbindungen der allgemeinen Formel I.D, in denen R¹ für Brom steht, R³ But-3-in-1-yl bedeutet und der Rest R² für eine Verbindung einer Gruppe der Tabelle A entspricht

### Tabelle 205

Verbindungen der allgemeinen Formel I.A, in denen R¹ für Iod steht, R³ But-3-in-1-yl bedeutet und der Rest R² für eine Verbindung einer Gruppe der Tabelle B entspricht

### Tabelle 206

Verbindungen der allgemeinen Formel I.B, in denen R¹ für Iod steht, R³ But-3-in-1-yl bedeutet und der Rest R² für eine Verbindung einer Gruppe der Tabelle B entspricht

### Tabelle 207

Verbindungen der allgemeinen Formel I.C, in denen R¹ für Iod steht, R³ But-3-in-1-yl bedeutet und der Rest R² für eine Verbindung einer Gruppe der Tabelle B entspricht

### Tabelle 208

Verbindungen der allgemeinen Formel I.D, in denen R¹ für Iod steht, R³ But-3-in-1-yl bedeutet und der Rest R² für eine Verbindung einer Gruppe der Tabelle B entspricht

### Tabelle 209

Verbindungen der allgemeinen Formel I.A, in denen R¹ für Chlor steht, R³ Fluormethyl bedeutet und der Rest R² für eine Verbindung einer Gruppe der Tabelle A entspricht

### Tabelle 210

Verbindungen der allgemeinen Formel I.B, in denen R¹ für Chlor steht, R³ Fluormethyl bedeutet und der Rest R² für eine Verbindung einer Gruppe der Tabelle A entspricht

### Tabelle 211

Verbindungen der allgemeinen Formel I.C, in denen R¹ für Chlor steht, R³ Fluormethyl bedeutet und der Rest R² für eine Verbindung einer Gruppe der Tabelle A entspricht

### Tabelle 212

Verbindungen der allgemeinen Formel I.D, in denen R¹ für Chlor steht, R³ Fluormethyl bedeutet und der Rest R² für eine Verbindung einer Gruppe der Tabelle A entspricht

### Tabelle 213

Verbindungen der allgemeinen Formel I.A, in denen R¹ für Fluor steht, R³ Fluormethyl bedeutet und der Rest R² für eine Verbindung einer Gruppe der Tabelle B entspricht

### Tabelle 214

Verbindungen der allgemeinen Formel I.B, in denen R¹ für Fluor steht, R³ Fluormethyl bedeutet und der Rest R² für eine Verbindung einer Gruppe der Tabelle B entspricht

### Tabelle 215

Verbindungen der allgemeinen Formel I.C, in denen R¹ für Fluor steht, R³ Fluormethyl bedeutet und der Rest R² für eine Verbindung einer Gruppe der Tabelle B entspricht

### Tabelle 216

Verbindungen der allgemeinen Formel I.D, in denen R¹ für Fluor steht, R³ Fluormethyl bedeutet und der Rest R² für eine Verbindung einer Gruppe der Tabelle B entspricht

### Tabelle 217

Verbindungen der allgemeinen Formel I.A, in denen R¹ für Brom steht, R³ Fluormethyl bedeutet und der Rest R² für eine Verbindung einer Gruppe der Tabelle A entspricht

### Tabelle 218

Verbindungen der allgemeinen Formel I.B, in denen R¹ für Brom steht, R³ Fluormethyl bedeutet und der Rest R² für eine Verbindung einer Gruppe der Tabelle A entspricht

### Tabelle 219

Verbindungen der allgemeinen Formel I.C, in denen R¹ für Brom steht, R³ Fluormethyl bedeutet und der Rest R² für eine Verbindung einer Gruppe der Tabelle A entspricht

### Tabelle 220

Verbindungen der allgemeinen Formel I.D, in denen R¹ für Brom steht, R³ Fluormethyl bedeutet und der Rest R² für eine Verbindung einer Gruppe der Tabelle A entspricht

### Tabelle 221

Verbindungen der allgemeinen Formel I.A, in denen R¹ für Iod steht, R³ Fluormethyl bedeutet und der Rest R² für eine Verbindung einer Gruppe der Tabelle B entspricht

### Tabelle 222

Verbindungen der allgemeinen Formel I.B, in denen R¹ für Iod steht, R³ Fluormethyl bedeutet und der Rest R² für eine Verbindung einer Gruppe der Tabelle B entspricht

### Tabelle 223

5 Verbindungen der allgemeinen Formel I.C, in denen R¹ für Iod steht, R³ Fluormethyl bedeutet und der Rest R² für eine Verbindung einer Gruppe der Tabelle B entspricht

### Tabelle 224

Verbindungen der allgemeinen Formel I.D, in denen R¹ für Iod steht, R³ Fluormethyl bedeutet und der Rest R² für eine Verbindung einer Gruppe der Tabelle B entspricht

### Tabelle 225

Verbindungen der allgemeinen Formel I.A, in denen R¹ für Chlor steht, R³ Difluormethyl bedeutet und der Rest R² für eine Verbindung einer Gruppe der Tabelle A entspricht

### Tabelle 226

Verbindungen der allgemeinen Formel I.B, in denen R¹ für Chlor steht, R³ Difluormethyl bedeutet und der Rest R² für eine Verbindung einer Gruppe der Tabelle A entspricht

### Tabelle 227

Verbindungen der allgemeinen Formel I.C, in denen R¹ für Chlor steht, R³ Difluormethyl bedeutet und der Rest R² für eine Verbindung einer Gruppe der Tabelle A entspricht

### Tabelle 228

Verbindungen der allgemeinen Formel I.D, in denen R¹ für Chlor steht, R³ Difluormethyl bedeutet und der Rest R² für eine Verbindung einer Gruppe der Tabelle A entspricht

### Tabelle 229

Verbindungen der allgemeinen Formel I.A, in denen R¹ für Fluor steht, R³ Difluormethyl bedeutet und der Rest R² für eine Verbindung einer Gruppe der Tabelle B entspricht

### Tabelle 230

Verbindungen der allgemeinen Formel I.B, in denen R¹ für Fluor steht, R³ Difluormethyl bedeutet und der Rest R² für eine Verbindung einer Gruppe der Tabelle B entspricht

### Tabelle 231

Verbindungen der allgemeinen Formel I.C, in denen R¹ für Fluor steht, R³ Difluormethyl bedeutet und der Rest R² für eine Verbindung einer Gruppe der Tabelle B entspricht

### Tabelle 232

Verbindungen der allgemeinen Formel I.D, in denen R¹ für Fluor steht, R³ Difluormethyl bedeutet und der Rest R² für eine Verbindung einer Gruppe der Tabelle B entspricht

### Tabelle 233

Verbindungen der allgemeinen Formel I.A, in denen R¹ für Brom steht, R³ Difluormethyl bedeutet und der Rest R² für eine Verbindung einer Gruppe der Tabelle A entspricht

### Tabelle 234

Verbindungen der allgemeinen Formel I.B, in denen R¹ für Brom steht, R³ Difluormethyl bedeutet und der Rest R² für eine Verbindung einer Gruppe der Tabelle A entspricht

### Tabelle 235

Verbindungen der allgemeinen Formel I.C, in denen R¹ für Brom steht, R³ Difluormethyl bedeutet und der Rest R² für eine Verbindung einer Gruppe der Tabelle A entspricht

### Tabelle 236

Verbindungen der allgemeinen Formel I.D, in denen R¹ für Brom steht, R³ Difluormethyl bedeutet und der Rest R² für eine Verbindung einer Gruppe der Tabelle A entspricht

### Tabelle 237

Verbindungen der allgemeinen Formel I.A. in denen R¹ für Iod steht, R³ Difluormethyl bedeutet und der Rest R² für eine Verbindung einer Gruppe der Tabelle B entspricht

### Tabelle 238

Verbindungen der allgemeinen Formel I.B, in denen R¹ für Iod steht, R³ Difluormethyl bedeutet und der Rest R² für eine Verbindung einer Gruppe der Tabelle B entspricht

### Tabelle 239

Verbindungen der allgemeinen Formel I.C, in denen R¹ für Iod steht, R³ Difluormethyl bedeutet und der Rest R² für eine Verbindung einer Gruppe der Tabelle B entspricht

### Tabelle 240

Verbindungen der allgemeinen Formel I.D, in denen R¹ für Iod steht, R³ Difluormethyl bedeutet und der Rest R² für eine Verbindung einer Gruppe der Tabelle B entspricht

### Tabelle 241

Verbindungen der allgemeinen Formel I.A, in denen R¹ für Chlor steht, R³ 2,2,2-Trifluorethyl bedeutet und der Rest R² für eine Verbindung einer Gruppe der Tabelle A entspricht

### Tabelle 242

Verbindungen der allgemeinen Formel I.B, in denen R¹ für Chlor steht, R³ 2,2,2-Trifluorethyl bedeutet und der Rest R² für eine Verbindung einer Gruppe der Tabelle A entspricht

### Tabelle 243

Verbindungen der allgemeinen Formel I.C, in denen R¹ für Chlor steht, R³ 2,2,2-Trifluorethyl bedeutet und der Rest R² für eine Verbindung einer Gruppe der Tabelle A entspricht

### Tabelle 244

Verbindungen der allgemeinen Formel I.D, in denen R¹ für Chlor steht, R³ 2,2,2-Trifluorethyl bedeutet und der Rest R² für eine Verbindung einer Gruppe der Tabelle A entspricht

### Tabelle 245

Verbindungen der allgemeinen Formel I.A, in denen R¹ für Fluor steht, R³ 2,2,2-Trifluorethyl bedeutet und der Rest R² für eine Verbindung einer Gruppe der Tabelle B entspricht

### Tabelle 246

Verbindungen der allgemeinen Formel I.B, in denen R¹ für Fluor steht, R³ 2,2,2-Trifluorethyl bedeutet und der Rest R² für eine Verbindung einer Gruppe der Tabelle B entspricht

### Tabelle 247

Verbindungen der allgemeinen Formel I.C, in denen R¹ für Fluor steht, R³ 2,2,2-Trifluorethyl bedeutet und der Rest R² für eine Verbindung einer Gruppe der Tabelle B entspricht

### Tabelle 248

Verbindungen der allgemeinen Formel I.D, in denen R¹ für Fluor steht, R³ 2,2,2-Trifluorethyl bedeutet und der Rest R² für eine Verbindung einer Gruppe der Tabelle B entspricht

### Tabelle 249

Verbindungen der allgemeinen Formel I.A, in denen R¹ für Brom steht, R³ 2,2,2-Trifluorethyl bedeutet und der Rest R² für eine Verbindung einer Gruppe der Tabelle A entspricht

### Tabelle 250

Verbindungen der allgemeinen Formel I.B, in denen R¹ für Brom steht, R³ 2,2,2-Trifluorethyl bedeutet und der Rest R² für eine Verbindung einer Gruppe der Tabelle A entspricht

### Tabelle 251

Verbindungen der allgemeinen Formel I.C, in denen R¹ für Brom steht, R³ 2,2,2-Trifluorethyl bedeutet und der Rest R² für eine Verbindung einer Gruppe der Tabelle A entspricht

### Tabelle 252

Verbindungen der allgemeinen Formel I.D, in denen R¹ für Brom steht, R³ 2,2,2-Trifluorethyl bedeutet und der Rest R² für eine Verbindung einer Gruppe der Tabelle A entspricht

### Tabelle 253

Verbindungen der allgemeinen Formel I.A, in denen R¹ für Iod steht, R³ 2,2,2-Trifluorethyl bedeutet und der Rest R² für eine Verbindung einer Gruppe der Tabelle B entspricht

### Tabelle 254

Verbindungen der allgemeinen Formel I.B, in denen R¹ für Iod steht, R³ 2,2,2-Trifluorethyl bedeutet und der Rest R² für eine Verbindung einer Gruppe der Tabelle B entspricht

### Tabelle 255

Verbindungen der allgemeinen Formel I.C, in denen R¹ für Iod steht, R³ 2,2,2-Trifluorethyl bedeutet und der Rest R² für eine Verbindung einer Gruppe der Tabelle B entspricht

### Tabelle 256

Verbindungen der allgemeinen Formel I.D, in denen R¹ für Iod steht, R³ 2,2,2-Trifluorethyl bedeutet und der Rest R² für eine Verbindung einer Gruppe der Tabelle B entspricht

**Tabelle A**

| **Nr.** | **R**^{**2**} | |
|---|---|---|
| A.1 | CH₂CH₃ | |
| A.2 | CH₂CH₂-CN | |
| A.3 | CH₂CH₂-O-CH₃ | |
| A.4 | CH₂CH₂-O-CH₂CH₃ | |
| A.5 | CH₂CH₂CH₃ | |
| A.6 | CH(CH₃)₂ | |
| A.7 | CH₂CH₂CH₂CH₃ | |
| A.8 | CH(CH₃)CH₂CH₃ | |
| A.9 | CH₂CH(CH₃)₂ | |
| A.10 | CH=CH₂ | |
| A.11 | CH=CH-CH₃ | (E) |
| A.12 | CH=CH-[C₆H₅] | (E) |
| A.13 | CH=CH-[2-CN-C₆H₄] | (E) |
| A.14 | CH=CH-[3-CN-C₆H₄] | (E) |
| A.15 | CH=CH-[4-CN-C₆H₄] | (E) |
| A.16 | CH=CH-[2-F-C₆H₄] | (E) |
| A.17 | CH=CH-[3-F-C₆H₄] | (E) |
| A.18 | CH=CH-[4-F-C₆H₄] | (E) |
| A.19 | CH=CH-[2,4-F₂-C₆H₃] | (E) |
| A.20 | CH=CH-[3,4-F₂-C₆H₃] | (E) |
| A.21 | CH=CH-[2,4,5-F₃-C₆H₂] | (E) |
| A.22 | CH=CH-[2,4,6-F₃-C₆H₂] | (E) |
| A.23 | CH=CH-[2-Cl-C₆H₄] | (E) |
| A.24 | CH=CH-[3-Cl-C₆H₄] | (E) |
| A.25 | CH=CH-[4-Cl-C₆H₄] | (E) |
| A.26 | CH=CH-[2,4-Cl₂-C₆H₃] | (E) |
| A.27 | CH=CH-[3,4-Cl₂-C₆H₃] | (E) |
| A.28 | CH=CH-[2,4,5-Cl₃-C₆H₂] | (E) |
| A.29 | CH=CH-[2,4,6-Cl₃-C₆H₂] | (E) |
| A.30 | CH=CH-[2-CH₃-C₆H₄] | (E) |
| A.31 | CH=CH-[3-CH₃-C₆H₄] | (E) |
| A.32 | CH=CH-[4-CH₃-C₆H₄] | (E) |
| A.33 | CH=CH-[2-F, 4-CH₃-C₆H₃] | (E) |
| A.34 | CH=CH-[2-CH₃, 4-F-C₆H₃] | (E) |
| A.35 | CH=CH-[3-F, 4-CH₃-C₆H₃] | (E) |
| A.36 | CH=CH-[3-CH₃, 4-F-C₆H₃] | (E) |
| A.37 | CH=CH-[2-Cl, 4-CH₃-C₆H₃] | (E) |
| A.38 | CH=CH-[2-CH₃, 4-Cl-C₆H₃] | (E) |
| A.39 | CH=CH-[3-Cl, 4-CH₃-C₆H₃] | (E) |
| A.40 | CH=CH-[3-CH₃, 4-Cl-C₆H₃] | (E) |
| A.41 | CH=CH-[2,4-(CH₃)₂-C₆H₃] | (E) |
| A.42 | CH=CH-[3,4-(CH₃)₂-C₆H₃] | (E) |
| A.43 | CH=CH-[2,4,5-(CH₃)₃-C₆H₂] | (E) |
| A.44 | CH=CH-[2,4,6-(CH₃)₃-C₆H₂] | (E) |
| A.45 | CH=CH-[2-CF₃-C₆H₄] | (E) |
| A.46 | CH=CH-[3-CF₃-C₆H₄] | (E) |
| A.47 | CH=CH-[4-CF₃-C₆H₄] | (E) |
| A.48 | CH=CH-[2-F, 4-CF₃-C₆H₃] | (E) |
| A.49 | CH=CH-[2-CF₃, 4-F-C₆H₃] | (E) |
| A.50 | CH=CH-[3-F, 4-CF₃-C₆H₃] | (E) |
| A.51 | CH=CH-[3-CF₃, 4-F-C₆H₃] | (E) |
| A.52 | CH=CH-[2-Cl, 4-CF₃-C₆H₃] | (E) |
| A.53 | CH=CH-[2-CF₃, 4-Cl-C₆H₃] | (E) |
| A.54 | CH=CH-[3-Cl, 4-CF₃-C₆H₃] | (E) |
| A.55 | CH=CH-[3-CF₃, 4-Cl-C₆H₃] | (E) |
| A.56 | CH=CH-[2,4-(CF₃)₂-C₆H₃] | (E) |
| A.57 | CH=CH-[3,4-(CF₃)₂-C₆H₃] | (E) |
| A.58 | CH=CH-[2,4,5-(CF₃)₃-C₆H₂] | (E) |
| A.59 | CH=CH-[2,4,6-(CF₃)₃-C₆H₂] | (E) |
| A.60 | CH=CH-[2-OCH₃-C₆H₄] | (E) |
| A.61 | CH=CH-[3-OCH₃-C₆H₄] | (E) |
| A.62 | CH=CH-[4-OCH₃-C₆H₄] | (E) |
| A.63 | CH=CH-[2-F, 4-OCH₃-C₆H₃] | (E) |
| A.64 | CH=CH-[2-OCH₃, 4-F-C₆H₃] | (E) |
| A.65 | CH=CH-[3-F, 4-OCH₃-C₆H₃] | (E) |
| A.66 | CH=CH-[3-OCH₃, 4-F-C₆H₃] | (E) |
| A.67 | CH=CH-[2-Cl, 4-OCH₃-C₆H₃] | (E) |
| A.68 | CH=CH-[2-OCH₃, 4-Cl-C₆H₃] | (E) |
| A.69 | CH=CH-[3-Cl, 4-OCH₃-C₆H₃] | (E) |
| A.70 | CH=CH-[3-OCH₃, 4-Cl-C₆H₃] | (E) |
| A.71 | CH=CH-[2,4-(OCH₃)₂-C₆H₃] | (E) |
| A.72 | CH=CH-[3,4-(OCH₃)₂-C₆H₃] | (E) |
| A.73 | CH=CH-[2,4,5-(OCH₃)₃-C₆H₂] | (E) |
| A.74 | CH=CH-[2,4,6-(OCH₃)₃-C₆H₂] | (E) |
| A.75 | CH=CH-[2-OCHF₂-C₆H₄] | (E) |
| A.76 | CH=CH-[3-OCHF₂-C₆H₄] | (E) |
| A.77 | CH=CH-[4-OCHF₂-C₆H₄] | (E) |
| A.78 | CH=CH-[2-F, 4-OCHF₂-C₆H₃] | (E) |
| A.79 | CH=CH-[2-OCHF₂, 4-F-C₆H₃] | (E) |
| A.80 | CH=CH-[3-F, 4-OCHF₂-C₆H₃] | (E) |
| A.81 | CH=CH-[3-OCHF₂, 4-F-C₆H₃] | (E) |
| A.82 | CH=CH-[2-Cl, 4-OCHF₂-C₆H₃] | (E) |
| A.83 | CH=CH-[2-OCHF₂, 4-Cl-C₆H₃] | (E) |
| A.84 | CH=CH-[3-Cl, 4-OCHF₂-C₆H₃] | (E) |
| A.85 | CH=CH-[3-OCHF₂, 4-Cl-C₆H₃] | (E) |
| A.86 | CH=CH-[2,4-(OCHF₂)₂-C₆H₃] | (E) |
| A.87 | CH=CH-[3,4-(OCHF₂)₂-C₆H₃] | (E) |
| A.88 | CH=CH-[2,4,5-(OCHF₂)₃-C₆H₂] | (E) |
| A.89 | CH=CH-[2,4,6-(OCHF₂)₃-C₆H₂] | (E) |
| A.90 | CH₂CH=CH₂ | |
| A.91 | CH₂CH=CH-Cl | (E) |
| A.92 | CH₂CH=CH-Br | (E) |
| A.93 | CH₂CH=CH-CH₃ | (E) |
| A.94 | C≡CH | |
| A.95 | C≡C-Cl | |
| A.96 | C≡C-Br | |
| A.97 | C≡C-CH₃ | |
| A.98 | C≡C-C₆H₅ | |
| A.99 | C≡C-[2-Cl-C₆H₄] | |
| A.100 | C≡C-[4-Cl-C₆H₄] | |
| A.101 | C≡C-[2,4-Cl₂-C₆H₃] | |
| A.102 | C≡C-[2-CH₃-C₆H₄] | |
| A.103 | C≡C-[4-CH₃-C₆H₄] | |
| A.104 | C≡C-[2,4-(CH₃)₂-C₆H₃] | |
| A.105 | C≡C-[2-Cl, 4-CH₃-C₆H₃] | |
| A.106 | C≡C-[2-CH₃, 4-Cl-C₆H₃] | |
| A.107 | C≡C-[3-CF₃-C₆H₄] | |
| A.108 | C≡C-[3-Cl, 5-CF₃-C₆H₃] | |
| A.109 | C≡C-[2-OCH₃-C₆H₄] | |
| A.110 | C≡C-[4-OCH₃-C₆H₄] | |
| A.111 | C≡C-[2,4-(OCH₃)₂-C₆H₃] | |
| A.112 | C≡C-[2-Cl, 4-OCH₃-C₆H₃] | |
| A.113 | C≡C-[2-OCH₃, 4-Cl-C₆H₃] | |
| A.114 | C≡C-[3-OCHF₂-C₆H₄] | |
| A.115 | C≡C-[3-Cl, 5-OCHF₂-C₆H₃] | |
| A.116 | cyclopentyl | |
| A.117 | 1-CH₃-cyclopentyl | |
| A.118 | 2-CH₃-cyclopentyl | |
| A.119 | 3-CH₃-cyclopentyl | |
| A.120 | 2,3-(CH₃)₂-cyclopentyl | |
| A.121 | 1-Cl-cyclopentyl | |
| A.122 | 2-Cl-cyclopentyl | |
| A.123 | 3-Cl-cyclopentyl | |
| A.124 | 2-CH₃, 3-Cl-cyclopentyl | |
| A.125 | 2,3-Cl₂-cyclopentyl | |
| A.126 | cyclohexyl | |
| A.127 | 1-CH₃-cyclohexyl | |
| A.128 | 2-CH₃-cyclohexyl | |
| A.129 | 3-CH₃-cyclohexyl | |
| A.130 | 2,3-(CH₃)₂-cyclohexyl | |
| A.131 | 3,3-(CH₃)₂-cyclohexyl | |
| A.132 | 1-Cl-cyclohexyl | |
| A.133 | 2-Cl-cyclohexyl | |
| A.134 | 3-Cl-cyclohexyl | |
| A.135 | 2-CH₃, 3-Cl-cyclohexyl | |
| A.136 | 2,3-Cl₂-cyclohexyl | |
| A.137 | C₆H₅ | |
| A.138 | 2-CN-C₆H₄ | |
| A.139 | 3-CN-C₆H₄ | |
| A.140 | 4-CN-C₆H₄ | |
| A.141 | 2-F-C₆H₄ | |
| A.142 | 3-F-C₆H₄ | |
| A.143 | 4-F-C₆H₄ | |
| A.144 | 2,4-F₂-C₆H₃ | |
| A.145 | 3,4-F₂-C₆H₃ | |
| A.146 | 2,4,5-F₃-C₆H₂ | |
| A.147 | 2,4,6-F₃-C₆H₂ | |
| A.148 | 2-Cl-C₆H₄ | |
| A.149 | 3-Cl-C₆H₄ | |
| A.150 | 4-Cl-C₆H₄ | |
| A.151 | 2,4-Cl₂-C₆H₃ | |
| A.152 | 3,4-Cl₂-C₆H₃ | |
| A.153 | 2,4,5-Cl₃-C₆H₂ | |
| A.154 | 2,4,6-Cl₃-C₆H₂ | |
| A.155 | 2-CH₃-C₆H₄ | |
| A.156 | 3-CH₃-C₆H₄ | |
| A.157 | 4-CH₃-C₆H₄ | |
| A.158 | 2-F, 4-CH₃-C₆H₃ | |
| A.159 | 2-CH₃, 4-F-C₆H₃ | |
| A.160 | 3-F, 4-CH₃-C₆H₃ | |
| A.161 | 3-CH₃, 4-F-C₆H₃ | |
| A.162 | 2-Cl, 4-CH₃-C₆H₃ | |
| A.163 | 2-CH₃, 4-Cl-C₆H₃ | |
| A.164 | 3-Cl, 4-CH₃-C₆H₃ | |
| A.165 | 3-CH₃, 4-Cl-C₆H₃ | |
| A.166 | 2,4-(CH₃)₂-C₆H₃ | |
| A.167 | 3,4-(CH₃)₂-C₆H₃ | |
| A.168 | 2,4,5-(CH₃)₃-C₆H₂ | |
| A.169 | 2,4,6-(CH₃)₃-C₆H₂ | |
| A.170 | 2-CF₃-C₆H₄ | |
| A.171 | 3-CF₃-C₆H₄ | |
| A.172 | 4-CF₃-C₆H₄ | |
| A.173 | 2-F, 4-CF₃-C₆H₃ | |
| A.174 | 2-CF₃, 4-F-C₆H₃ | |
| A.175 | 3-F, 4-CF₃-C₆H₃ | |
| A.176 | 3-CF₃, 4-F-C₆H₃ | |
| A.177 | 2-Cl, 4-CF₃-C₆H₃ | |
| A.178 | 2-CF₃, 4-Cl-C₆H₃ | |
| A.179 | 3-Cl, 4-CF₃-C₆H₃ | |
| A.180 | 3-CF₃, 4-Cl-C₆H₃ | |
| A.181 | 2,4-(CF₃)₂-C₆H₃ | |
| A.182 | 3,4-(CF₃)₂-C₆H₃ | |
| A.183 | 2,4,5-(CF₃)₃-C₆H₂ | |
| A.184 | 2,4,6-(CF₃)₃-C₆H₂ | |
| A.185 | 2-OCH₃-C₆H₄ | |
| A.186 | 3-OCH₃-C₆H₄ | |
| A.187 | 4-OCH₃-C₆H₄ | |
| A.188 | 2-F, 4-OCH₃-C₆H₃ | |
| A.189 | 2-OCH₃, 4-F-C₆H₃ | |
| A.190 | 3-F, 4-OCH₃-C₆H₃ | |
| A.191 | 3-OCH₃, 4-F-C₆H₃ | |
| A.192 | 2-Cl, 4-OCH₃-C₆H₃ | |
| A.193 | 2-OCH₃, 4-Cl-C₆H₃ | |
| A.194 | 3-Cl 4-OCH₃-C₆H₃ | |
| A.195 | 3-OCH₃, 4-Cl-C₆H₃ | |
| A.196 | 2,4-(OCH₃)₂-C₆H₃ | |
| A.197 | 3,4-(OCH₃)₂-C₆H₃ | |
| A.198 | 2,4,5-(OCH₃)₃-C₆H₂ | |
| A.199 | 2,4,6-(OCH₃)₃-C₆H₂ | |
| A.200 | 2-OCHF₂-C₆H₄ | |
| A.201 | 3-OCHF₂-C₆H₄ | |
| A.202 | 4-OCHF₂-C₆H₄ | |
| A.203 | 2-F, 4-OCHF₂-C₆H₃ | |
| A.204 | 2-OCHF₂, 4-F-C₆H₃ | |
| A.205 | 3-F, 4-OCHF₂-C₆H₃ | |
| A.206 | 3-OCHF₂, 4-F-C₆H₃ | |
| A.207 | 2-Cl, 4-OCHF₂-C₆H₃ | |
| A.208 | 2-OCHF₂, 4-Cl-C₆H₃ | |
| A.209 | 3-Cl, 4-OCHF₂-C₆H₃ | |
| A.210 | 3-OCHF₂, 4-Cl-C₆H₃ | |
| A.211 | 2,4-(OCHF₂)₂-C₆H₃ | |
| A.212 | 3,4-(OCHF₂)₂-C₆H₃ | |
| A.213 | 2,4,5-(OCHF₂)₃-C₆H₂ | |
| A.214 | 2,4,6-(OCHF₂)₃-C₆H₂ | |
| A.215 | CH₂-C₆H₅ | |
| A.216 | CH₂-[2-CN-C₆H₄] | |
| A.217 | CH₂-[3-CN-C₆H₄] | |
| A.218 | CH₂-[4-CN-C₆H₄] | |
| A.219 | CH₂-[2-F-C₆H₄] | |
| A.220 | CH₂-[3-F-C₆H₄] | |
| A.221 | CH₂-[4-F-C₆H₄] | |
| A.222 | CH₂-[2,4-F₂-C₆H₃] | |
| A.223 | CH₂-[3,4-F₂-C₆H₃] | |
| A.224 | CH₂-[2,4,5-F₃-C₆H₂] | |
| A.225 | CH₂-[2,4,6-F₃-C₆H₂] | |
| A.226 | CH₂-[2-Cl-C₆H₄] | |
| A.227 | CH₂-[3-Cl-C₆H₄] | |
| A.228 | CH₂-[4-Cl-C₆H₄] | |
| A.229 | CH₂-[2,4-Cl₂-C₆H₃] | |
| A.230 | CH₂-[3,4-Cl₂-C₆H₃] | |
| A.231 | CH₂-[2,4,5-Cl₃-C₆H₂] | |
| A.232 | CH₂-[2,4,6-Cl₃-C₆H₂] | |
| A.233 | CH₂-[2-CH₃-C₆H₄) | |
| A.234 | CH₂-[3-CH₃-C₆H₄] | |
| A.235 | CH₂-[4-CH₃-C₆H₄] | |
| A. 236 | CH₂-[2-F, 4-CH₃-C₆H₃] | |
| A.237 | CH₂-[2-CH₃, 4-F-C₆H₃] | |
| A.238 | CH₂-[3-F, 4-CH₃-C₆H₃] | |
| A.239 | CH₂-[3-CH₃, 4-F-C₆H₃] | |
| A.240 | CH₂-[2-Cl, 4-CH₃-C₆H₃] | |
| A.241 | CH₂-[2-CH₃, 4-Cl-C₆H₃] | |
| A.242 | CH₂-[3-Cl, 4-CH₃-C₆H₃] | |
| A.243 | CH₂-[3-CH₃, 4-Cl-C₆H₃] | |
| A.244 | CH₂-[2,4-(CH₃)₂-C₆H₃] | |
| A.245 | CH₂-[3,4-(CH₃)₂-C₆H₃] | |
| A.246 | CH₂-[2,4,5-(CH₃)₃-C₆H₂] | |
| A.247 | CH₂-[2,4,6-(CH₃)₃-C₆H₂] | |
| A. 248 | CH₂-[2-CF₃-C₆H₄] | |
| A.249 | CH₂-[3-CF₃-C₆H₄] | |
| A.250 | CH₂-[4-CF₃-C₆H₄] | |
| A.251 | CH₂-[2-F, 4-CF₃-C₆H₃] | |
| A.252 | CH₂-[2-CF₃, 4-F-C₆H₃] | |
| A.253 | CH₂-[3-F, 4-CF₃-C₆H₃] | |
| A.254 | CH₂-[3-CF₃, 4-F-C₆H₃] | |
| A.255 | CH₂-[2-Cl, 4-CF₃-C₆H₃] | |
| A.256 | CH₂-[2-CF₃, 4-Cl-C₆H₃] | |
| A.257 | CH₂-[3-Cl, 4-CF₃-C₆H₃] | |
| A.258 | CH₂-[3-CF₃, 4-Cl-C₆H₃] | |
| A.259 | CH₂-[2,4-(CF₃)₂-C₆H₃] | |
| A.260 | CH₂-[3,4-(CF₃)₂-C₆H₃] | |
| A.261 | CH₂-[2,4,5-(CF₃)₃-C₆H₂] | |
| A.262 | CH₂-[2,4,6-(CF₃)₃-C₆H₂] | |
| A.263 | CH₂-[2-OCH₃-C₆H₄] | |
| A.264 | CH₂-[3-OCH₃-C₆H₄] | |
| A.265 | CH₂-[4-OCH₃-C₆H₄] | |
| A.266 | CH₂-[2-F, 4-OCH₃-C₆H₃] | |
| A.267 | CH₂-[2-OCH₃, 4-F-C₆H₃] | |
| A.268 | CH₂-[3-F, 4-OCH₃-C₆H₃] | |
| A.269 | CH₂-[3-OCH₃, 4-F-C₆H₃] | |
| A.270 | CH₂-[2-Cl, 4-OCH₃-C₆H₃] | |
| A.271 | CH₂-[2-OCH₃, 4-Cl-C₆H₃] | |
| A. 272 | CH₂-[3-Cl, 4-OCH₃-C₆H₃] | |
| A.273 | CH₂-[3-OCH₃, 4-Cl-C₆H₃] | |
| A.274 | CH₂-[2,4-(OCH₃)₂-C₆H₃] | |
| A.275 | CH₂-[3,4-(OCH₃)₂-C₆H₃] | |
| A.276 | CH₂-[2,4,5-(OCH₃)₃-C₆H₂] | |
| A.277 | CH₂-[2,4,6-(OCH₃)₃-C₆H₂] | |
| A.278 | CH₂-[2-OCHF₂-C₆H₄] | |
| A.279 | CH₂-[3-OCHF₂-C₆H₄] | |
| A.280 | CH₂-[4-OCHF₂-C₆H₄] | |
| A.281 | CH₂-[2-F, 4-OCHF₂-C₆H₃] | |
| A.282 | CH₂-[2-OCHF₂, 4-F-C₆H₃] | |
| A.283 | CH₂-[3-F, 4-OCHF₂-C₆H₃] | |
| A.284 | CH₂-[3-OCHF₂, 4-F-C₆H₃] | |
| A.285 | CH₂-[2-Cl, 4-OCHF₂-C₆H₃] | |
| A.286 | CH₂-[2-OCHF₂, 4-Cl-C₆H₃] | |
| A.287 | CH₂-[3-Cl, 4-OCHF₂-C₆H₃] | |
| A.288 | CH₂-[3-OCHF₂, 4-Cl-C₆H₃] | |
| A.289 | CH₂-[2,4-(OCHF₂)₂-C₆H₃] | |
| A.290 | CH₂-[3,4-(OCHF₂)₂-C₆H₃] | |
| A.291 | CH₂-[2,4,5-(OCHF₂)₃-C₆H₂] | |
| A.292 | CH₂-[2,4,6-(OCHF₂)₃-C₆H₂] | |
| A.293 | CH(CH₃)-C₆H₅ | |
| A.294 | CH(CH₃)-[2-CN-C₆H₄] | |
| A.295 | CH(CH₃)-[3-CN-C₆H₄] | |
| A.296 | CH(CH₃)-[4-CN-C₆H₄] | |
| A.297 | CH(CH₃)-[2-F-C₆H₄] | |
| A.298 | CH(CH₃)-[3-F-C₆H₄] | |
| A.299 | CH(CH₃)-[4-F-C₆H₄] | |
| A.300 | CH(CH₃)-[2,4-F₂-C₆H₃] | |
| A.301 | CH(CH₃)-[3,4-F₂-C₆H₃] | |
| A.302 | CH(CH₃)-[2,4,5-F₃-C₆H₂] | |
| A.303 | CH(CH₃)-[2,4,6-F₃-C₆H₂] | |
| A.304 | CH(CH₃)-[2-Cl-C₆H₄] | |
| A.305 | CH(CH₃)-[3-Cl-C₆H₄] | |
| A.306 | CH(CH₃)-[4-Cl-C₆H₄] | |
| A.307 | CH(CH₃)-[2,4-Cl₂-C₆H₃] | |
| A.308 | CH(CH₃)-[3,4-Cl₂-C₆H₃] | |
| A.309 | CH(CH₃)-[2,4,5-Cl₃-C₆H₂] | |
| A.310 | CH(CH₃)-[2,4,6-Cl₃-C₆H₂] | |
| A.311 | CH(CH₃)-[2-CH₃-C₆H₄] | |
| A.312 | CH(CH₃)-[3-CH₃-C₆H₄] | |
| A.313 | CH(CH₃)-[4-CH₃-C₆H₄] | |
| A.314 | CH(CH₃)-[2-F, 4-CH₃-C₆H₃] | |
| A.315 | CH(CH₃)-[2-CH₃, 4-F-C₆H₃] | |
| A.316 | CH(CH₃)-[3-F, 4-CH₃-C₆H₃] | |
| A.317 | CH(CH₃)-[3-CH₃, 4-F-C₆H₃] | |
| A.318 | CH(CH₃)-[2-Cl, 4-CH₃-C₆H₃] | |
| A.319 | CH(CH₃)-[2-CH₃, 4-Cl-C₆H₃] | |
| A.320 | CH(CH₃)-[3-Cl, 4-CH₃-C₆H₃] | |
| A.321 | CH(CH₃)-[3-CH₃, 4-Cl-C₆H₃] | |
| A.322 | CH(CH₃)-[2,4-(CH₃)₂-C₆H₃] | |
| A.323 | CH(CH₃)-[3,4-(CH₃)₂-C₆H₃] | |
| A.324 | CH(CH₃)-[2,4,5-(CH₃)₃-C₆H₂] | |
| A.325 | CH(CH₃)-[2,4,6-(CH₃)₃-C₆H₂] | |
| A.326 | CH(CH₃)-[2-CF₃-C₆H₄] | |
| A.327 | CH(CH₃)-[3-CF₃-C₆H₄] | |
| A.328 | CH(CH₃)-[4-CF₃-C₆H₄] | |
| A.329 | CH(CH₃)-[2-F, 4-CF₃-C₆H₃] | |
| A.330 | CH(CH₃)-[2-CF₃, 4-F-C₆H₃] | |
| A.331 | CH(CH₃)-[3-F, 4-CF₃-C₆H₃] | |
| A.332 | CH(CH₃)-[3-CF₃, 4-F-C₆H₃] | |
| A.333 | CH(CH₃)-[2-Cl, 4-CF₃-C₆H₃] | |
| A.334 | CH(CH₃)-[2-CF₃, 4-Cl-C₆H₃] | |
| A.335 | CH(CH₃)-[3-Cl, 4-CF₃-C₆H₃] | |
| A.336 | CH(CH₃)-[3-CF₃, 4-Cl-C₆H₃] | |
| A.337 | CH(CH₃)-[2,4-(CF₃)₂-C₆H₃] | |
| A.338 | CH(CH₃)-[3,4-(CF₃)₂-C₆H₃] | |
| A.339 | CH(CH₃)-[2,4,5-(CF₃)₃-C₆H₂] | |
| A.340 | CH(CH₃)-[2,4,6-(CF₃)₃-C₆H₂] | |
| A.341 | CH(CH₃)-[2-OCH₃-C₆H₄] | |
| A.342 | CH(CH₃)-[3-OCH₃-C₆H₄] | |
| A.343 | CH(CH₃)-[4-OCH₃-C₆H₄] | |
| A.344 | CH(CH₃)-[2-F, 4-OCH₃-C₆H₃] | |
| A.345 | CH(CH₃)-[2-OCH₃, 4-F-C₆H₃] | |
| A.346 | CH(CH₃)-[3-F, 4-OCH₃-C₆H₃] | |
| A.347 | CH(CH₃)-[3-OCH₃, 4-F-C₆H₃] | |
| A.348 | CH(CH₃)-[2-Cl, 4-OCH₃-C₆H₃] | |
| A.349 | CH(CH₃)-[2-OCH₃, 4-Cl-C₆H₃] | |
| A.350 | CH(CH₃)-[3-Cl, 4-OCH₃-C₆H₃] | |
| A.351 | CH(CH₃)-[3-OCH₃, 4-Cl-C₆H₃] | |
| A.352 | CH(CH₃)-[2,4-(OCH₃)₂-C₆H₃] | |
| A.353 | CH(CH₃)-[3,4-(OCH₃)₂-C₆H₃] | |
| A.354 | CH(CH₃)-[2,4,5-(OCH₃)₃-C₆H₂] | |
| A.355 | CH(CH₃)-[2,4,6-(OCH₃)₃-C₆H₂] | |
| A.356 | CH(CH₃)-[2-OCHF₂-C₆H₄] | |
| A.357 | CH (CH₃)-[3-OCHF₂-C₆H₄] | |
| A.358 | CH(CH₃)-[4-OCHF₂-C₆H₄] | |
| A.359 | CH(CH₃)-[2-F, 4-OCHF₂-C₆H₃] | |
| A.360 | CH(CH₃)-[2-OCHF₂, 4-F-C₆H₃] | |
| A.361 | CH(CH₃)-[3-F, 4-OCHF₂-C₆H₃] | |
| A.362 | CH(CH₃)-[3-OCHF₂, 4-F-C₆H₃] | |
| A.363 | CH(CH₃)-[2-Cl, 4-OCHF₂-C₆H₃] | |
| A.364 | CH(CH₃)-[2-OCHF₂, 4-Cl-C₆H₃] | |
| A.365 | CH(CH₃)-[3-Cl, 4-OCHF₂-C₆H₃] | |
| A.366 | CH(CH₃)-[3-OCHF₂, 4-Cl-C₆H₃] | |
| A.367 | CH(CH₃)-[2,4- (OCHF₂)₂-C₆H₃] | |
| A.368 | CH(CH₃)-[3,4-(OCHF₂)₂-C₆H₃] | |
| A.369 | CH(CH₃)-[2,4,5-(OCHF₂)₃-C₆H₂] | |
| A.370 | CH(CH₃)-[2,4,6-(OCHF₂)₃-C₆H₂] | |
| A.371 | CH₂F | |
| A.372 | CHF₂ | |
| A.373 | CF₃ | |
| A.374 | CH₂-CN | |
| A.375 | CH₂-OCH₃ | |
| A.376 | CH₂-OCH₂CH₃ | |
| A.377 | CH₂-OCH₂CH₂CH₃ | |
| A.378 | CH₂-OCH(CH₃)₂ | |
| A.379 | CH₂-OCH₂CH₂CH₂CH₃ | |
| A.380 | CH₂-OCH(CH₃)CH₂CH₃ | |
| A.381 | CH₂-OCH₂CH(CH₃)₂ | |
| A.382 | CH₂-OC(CH₃)₃ | |
| A.383 | CH₂-OCF₃ | |
| A.384 | CH₂-OCH₂CF₃ | |
| A.385 | CH₂CH₂-OCH₂CH₂CH₃ | |
| A.386 | CH₂CH₂-OCH(CH₃)₂ | |
| A.387 | CH₂CH₂-OCH₂CH₂CH₂CH₃ | |
| A.388 | CH₂CH₂-OCH(CH₃)CH₂CH₃ | |
| A.389 | CH₂CH₂-OCH₂CH(CH₃)₂ | |
| A.390 | CH₂CH₂-OC(CH₃)₃ | |
| A.391 | CH₂CH₂-OCF₃ | |
| A.392 | CH₂CH₂-OCH₂CF₃ | |
| A.393 | CH₂CH₂-[C₆H₅] | |
| A.394 | CH₂CH₂-[2-CN-C₆H₄] | |
| A.395 | CH₂CH₂-[3-CN-C₆H₄] | |
| A.396 | CH₂CH₂-[4-CN-C₆H₄] | |
| A.397 | CH₂CH₂-[2-F-C₆H₄] | |
| A.398 | CH₂CH₂-[3-F-C₆H₄] | |
| A.399 | CH₂CH₂-[4-F-C₆H₄] | |
| A.400 | CH₂CH₂-[2,4-F₂-C₆H₃] | |
| A.401 | CH₂CH₂-[3,4-F₂-C₆H₃] | |
| A.402 | CH₂CH₂-[2,4,5-F₃-C₆H₂] | |
| A.403 | CH₂CH₂-[2,4,6-F₃-C₆H₂] | |
| A.404 | CH₂CH₂-[2-Cl-C₆H₄] | |
| A.405 | CH₂CH₂-[3-Cl-C₆H₄] | |
| A.406 | CH₂CH₂-[4-Cl-C₆H₄] | |
| A.407 | CH₂CH₂-[2,4-Cl₂-C₆H₃] | |
| A.408 | CH₂CH₂- [3,4-Cl₂-C₆H₃] | |
| A.409 | CH₂CH₂-[2,4,5-Cl₃-C₆H₂] | |
| A.410 | CH₂CH₂-[2,4,6-Cl₃-C₆H₂] | |
| A.411 | CH₂CH₂- [2-CH₃-C₆H₄] | |
| A.412 | CH₂CH₂-[3-CH₃-C₆H₄] | |
| A.413 | CH₂CH₂-[4-CH₃-C₆H₄] | |
| A.414 | CH₂CH₂-[2-F, 4-CH₃-C₆H₃] | |
| A.415 | CH₂CH₂-[2-CH₃, 4-F-C₆H₃] | |
| A.416 | CH₂CH₂-[3-F, 4-CH₃-C₆H₃] | |
| A.417 | CH₂CH₂-[3-CH₃, 4-F-C₆H₃] | |
| A.418 | CH₂CH₂-[2-Cl, 4-CH₃-C₆H₃] | |
| A.419 | CH₂CH₂-[2-CH₃, 4-Cl-C₆H₃] | |
| A.420 | CH₂CH₂-[3-Cl, 4-CH₃-C₆H₃] | |
| A.421 | CH₂CH₂-[3-CH₃, 4-Cl-C₆H₃] | |
| A.422 | CH₂CH₂-[2,4-(CH₃)₂-C₆H₃] | |
| A.423 | CH₂CH₂-[3,4-(CH₃)₂-C₆H₃] | |
| A.424 | CH₂CH₂-[2,4,5-(CH₃)₃-C₆H₂] | |
| A.425 | CH₂CH₂-[2,4,6-(CH₃)₃-C₆H₂] | |
| A.426 | CH₂CH₂- [2-CF₃-C₆H₄] | |
| A.427 | CH₂CH₂-[3-CF₃-C₆H₄] | |
| A.428 | CH₂CH₂-[4-CF₃-C₆H₄] | |
| A.429 | CH₂CH₂-[2-F, 4-CF₃-C₆H₃] | |
| A.430 | CH₂CH₂-[2-CF₃, 4-F-C₆H₃] | |
| A.431 | CH₂CH₂-[3-F, 4-CF₃-C₆H₃] | |
| A.432 | CH₂CH₂-[3-CF₃, 4-F-C₆H₃] | |
| A.433 | CH₂CH₂-[2-Cl, 4-CF₃-C₆H₃] | |
| A.434 | CH₂CH₂-[2-CF₃, 4-Cl-C₆H₃] | |
| A.435 | CH₂CH₂-[3-Cl, 4-CF₃-C₆H₃] | |
| A.436 | CH₂CH₂-[3-CF₃, 4-Cl-C₆H₃] | |
| A.437 | CH₂CH₂-[2,4-(CF₃)₂-C₆H₃] | |
| A.438 | CH₂CH₂-[3,4-(CF₃)₂-C₆H₃] | |
| A.439 | CH₂CH₂-[2,4,5-(CF₃)₃-C₆H₂] | |
| A.440 | CH₂CH₂-[2,4,6-(CF₃)₃-C₆H₂] | |
| A.441 | CH₂CH₂-[2-OCH₃-C₆H₄] | |
| A.442 | CH₂CH₂-[3-OCH₃-C₆H₄] | |
| A.443 | CH₂CH₂-[4-OCH₃-C₆H₄] | |
| A.444 | CH₂CH₂-[2-F, 4-OCH₃-C₆H₃] | |
| A.445 | CH₂CH₂-[2-OCH₃, 4-F-C₆H₃] | |
| A.446 | CH₂CH₂-[3-F, 4-OCH₃-C₆H₃] | |
| A.447 | CH₂CH₂-[3-OCH₃, 4-F-C₆H₃] | |
| A.448 | CH₂CH₂-[2-Cl, 4-OCH₃-C₆H₃] | |
| A.449 | CH₂CH₂-[2-OCH₃, 4-Cl-C₆H₃] | |
| A.450 | CH₂CH₂-[3-Cl, 4-OCH₃-C₆H₃] | |
| A.451 | CH₂CH₂-[3-OCH₃, 4-Cl-C₆H₃] | |
| A.452 | CH₂CH₂-[2,4-(OCH₃)₂-C₆H₃] | |
| A.453 | CH₂CH₂-[3,4-(OCH₃)₂-C₆H₃] | |
| A.454 | CH₂CH₂-[2,4,5-(OCH₃)₃-C₆H₂] | |
| A.455 | CH₂CH₂-[2,4,6-(OCH₃)₃-C₆H₂] | |
| A.456 | CH₂CH₂-[2-OCHF₂-C₆H₄] | |
| A.457 | CH₂CH₂-[3-OCHF₂-C₆H₄] | |
| A.458 | CH₂CH₂-[4-OCHF₂-C₆H₄] | |
| A.459 | CH₂CH₂-[2-F, 4-OCHF₂-C₆H₃] | |
| A.460 | CH₂CH₂-[2-OCHF₂, 4-F-C₆H₃] | |
| A.461 | CH₂CH₂-[3-F, 4-OCHF₂-C₆H₃] | |
| A.462 | CH₂CH₂-[3-OCHF₂, 4-F-C₆H₃] | |
| A.463 | CH₂CH₂-[2-Cl, 4-OCHF₂-C₆H₃] | |
| A.464 | CH₂CH₂-[2-OCHF₂, 4-Cl-C₆H₃] | |
| A.465 | CH₂CH₂-[3-Cl, 4-OCHF₂-C₆H₃] | |
| A.466 | CH₂CH₂-[3-OCHF₂, 4-Cl-C₆H₃] | |
| A.467 | CH₂CH₂-[2,4-(OCHF₂)₂-C₆H₃] | |
| A.468 | CH₂CH₂-[3,4-(OCHF₂)₂-C₆H₃] | |
| A.469 | CH₂CH₂-[2,4,5-(OCHF₂)₃-C₆H₂] | |
| A.470 | CH₂CH₂-[2,4,6-(OCHF₂)₃-C₆H₂]) | |
| A.471 | CH(CH₃)-CN | |
| A.472 | CH(CH₃)-OCH₃ | |
| A.473 | CH(CH₃)-OCH₂CH₃ | |
| A.474 | CH(CH₃)-OCH₂CH₂CH₃ | |
| A.475 | CH(CH₃)-OCH(CH₃)₂ | |
| A.476 | CH(CH₃)-OCH₂CH₂CH₂CH₃ | |
| A.477 | CH(CH₃)-OCH(CH₃)CH₂CH₃ | |
| A.478 | CH(CH₃)-OCH₂CH(CH₃)₂ | |
| A.479 | CH(CH₃)-OC(CH₃)₃ | |
| A.480 | CH(CH₃)-OCF₃ | |
| A.481 | CH(CH₃)-OCH₂CF₃ | |
| A.482 | CH₂CH₂F | |
| A.483 | CH₂CHF₂ | |
| A.484 | CH₂CF₃ | |
| A.485 | CHFCH₃ | |
| A.486 | CF₂CH₃ | |
| A.487 | CHFCHF₂ | |
| A.488 | CHFCF₃ | |
| A.489 | CF₂CHF₂ | |
| A.490 | CF₂CF₃ | |
| A.491 | CF₂CHFCl | |
| A.492 | CH₂CH₂CH₂-CN | |
| A.493 | CH₂CH₂CH₂-OCH₃ | |
| A.494 | CH₂CH₂CH₂-OCH₂CH₃ | |
| A.495 | CH₂CH₂CH₂-OCH₂CH₂CH₃ | |
| A.496 | CH₂CH₂CH₂-OCH(CH₃)₂ | |
| A.497 | CH₂CH₂CH₂-OCF₃ | |
| A.498 | CH₂CH₂CH₂-OCH₂CF₃ | |
| A.499 | CH₂CH(CH₃)-CN | |
| A.500 | CH₂CH(CH₃)-OCH₃ | |
| A.501 | CH₂CH(CH₃)-OCH₂CH₃ | |
| A.502 | CH₂CH(CH₃)-OCH₂CH₂CH₃ | |
| A.503 | CH₂CH(CH₃)-OCH(CH₃)₂ | |
| A.504 | CH₂CH(CH₃)-OCF₃ | |
| A.505 | CH₂CH (CH₃)-OCH₂CF₃ | |
| A.506 | CH(CH₂CH₃)-CN | |
| A.507 | CH(CH₂CH₃)-OCH₃ | |
| A.508 | CH(CH₂CH₃)-OCH₂CH₃ | |
| A.509 | CH(CH₂CH₃)-OCH₂CH₂CH₃ | |
| A.510 | CH(CH₂CH₃)-OCH(CH₃)₂ | |
| A.511 | CH(CH₂CH₃)-OCF₃ | |
| A.512 | CH(CH₂CH₃)-OCH₂CF₃ | |
| A.513 | CH(CH₃)CH₂-CN | |
| A.514 | CH(CH₃)CH₂-OCH₃ | |
| A.515 | CH(CH₃)CH₂-OCH₂CH₃ | |
| A.516 | CH(CH₃)CH₂-OCH₂CH₂CH₃ | |
| A.517 | CH(CH₃)CH₂-OCH(CH₃)₂ | |
| A.518 | CH(CH₃)CH₂-OCF₃ | |
| A.519 | CH(CH₃)CH₂-OCH₂CF₃ | |
| A.520 | CH₂CH₂CF₃ | |
| A.521 | CH₂CF₃CF₃ | |
| A.522 | CHFCH₂CH₃ | |
| A.523 | CH(CF₃)CH₃ | |
| A.524 | CH(CF₃)₂ | |
| A.525 | CH₂CH₂CH₂CH₂-CN | |
| A.526 | CH₂CH₂CH₂CH₂-OCH₃ | |
| A.527 | CH₂CH₂CH₂CH₂-OCH₂CH₃ | |
| A.528 | CH₂CH₂CH₂CH₂-OCF₃ | |
| A.529 | CH (CN) -CH₂CH₂CH₃ | |
| A.530 | CH(OCH₃)-CH₂CH₂CH₃ | |
| A.531 | CH(OCH₂CH₃)-CH₂CH₂CH₃ | |
| A.532 | CH(OCF₃)-CH₂CH₂CH₃ | |
| A.533 | CH(OCH₂CF₃)-CH₂CH₂CH₃ | |
| A.534 | CH(CN)-CH₂CH(CH₃)₂ | |
| A.535 | CH(OCH₃)-CH₂CH(CH₃)₂ | |
| A.536 | CH(OCH₂CH₃)-CH₂CH(CH₃)₂ | |
| A.537 | CH(OCF₃)-CH₂CH(CH₃)₂ | |
| A.538 | CH(OCH₂CF₃)-CH₂CH(CH₃)₂ | |
| A.539 | CHFCH₂CH₂CH₃ | |
| A.540 | C(CH₃)=CH₂ | |
| A.541 | CH=CH-CH₃ | (Z) |
| A.542 | C(CH₂CH₃)=CH₂ | |
| A.543 | C(CH₃)=CH-CH₃ | (E) |
| A.544 | C(CH₃)=CH-CH₃ | (Z) |
| A.545 | C(CH₃)=C(CH₃)₂ | |
| A.546 | CH(CH₃)-CH=CH₂ | |
| A.547 | CH=C(CH₃)₂ | |
| A.548 | CH₂-C(CH₃)=CH₂ | |
| A.549 | CH(CH₃)-CH₂-CH=CH₂ | |
| A.550 | CH₂-CH (CH₃)-CH=CH₂ | |
| A.551 | CH₂-CCl=CH₂ | |
| A.552 | CH₂-CH=CH-Cl | (Z) |
| A.553 | CH₂-CCl=CH-Cl | (E) |
| A.554 | CH₂-CCl=CH-Cl | (Z) |
| A.555 | CH₂-CH=CCl₂ | |
| A.556 | CH₂-CCl=CCl₂ | |
| A.557 | CH₂-CBr=CH₂ | |
| A.558 | CH₂-CH=CH-Br | (Z) |
| A.559 | CH₂-CBr=CH-Br | (E) |
| A.560 | CH₂-CBr=CH-Br | (Z) |
| A.561 | CH₂-CH=CBr₂ | |
| A.562 | CH₂-CBr=CBr₂ | |
| A.563 | CH₂-CH=CH-CH₃ | (Z) |
| A.564 | CH₂-C(CH₃)=CH-CH₃ | (E) |
| A. 565 | CH₂-C(CH₃)=CH-CH₃ | (Z) |
| A.566 | CH₂-CH=C(CH₃)₂ | |
| A.567 | CH₂-CH₂-CH=CH₂ | |
| A.568 | CH₂-CCl=CH-CH₃ | (E) |
| A.569 | CH₂-CCl=CH-CH₃ | (Z) |
| A.570 | CH₂-CH=CCl-CH₃ | (E) |
| A.571 | CH₂-CH=CCl-CH₃ | (Z) |
| A.572 | CH₂-C(CH₃)=C(CH₃)₂ | |
| A.573 | CH₂-CBr=CH-CH₃ | (E) |
| A.574 | CH₂-CBr=CH-CH₃ | (Z) |
| A.575 | CH₂-CH=CBr-CH₃ | (E) |
| A.576 | CH₂-CH=CBr-CH₃ | (Z) |
| A.577 | CH₂-CH=CH-CH₂Cl | (E) |
| A.578 | CH₂-CH=CH-CH₂Cl | (Z) |
| A.579 | CH₂-CH=CH-CH₂CH₃ | (E) |
| A.580 | CH₂-CH=CH-CH₂CH₃ | (Z) |
| A.581 | CH₂-CH=CH-CH₂Br | (E) |
| A.582 | CH₂-CH=CH-CH₂Br | (Z) |
| A.583 | CH₂-CCl=CCl-CH₂Cl | (E) |
| A.584 | CH₂-CCl=CCl-CH₂Cl | (Z) |
| A.585 | CH₂-CF=CH₂ | |
| A.586 | CH₂-CH=CH-F | (E) |
| A.587 | CH₂-CH=CH-F | (Z) |
| A.588 | CH₂-CH=CF₂ | |
| A.589 | CH₂-CF=CH-F | (E) |
| A.590 | CH₂-CF=CH-F | (Z) |
| A.591 | CH(CH₃)-CH=CH₂ | |
| A.592 | CH(CH₃)-CCl=CH₂ | |
| A.593 | CH(CH₃)-CH=CH-Cl | (E) |
| A.594 | CH(CH₃)-CH=CH-Cl | (Z) |
| A.595 | CH(CH₃)-CCl=CH-Cl | (E) |
| A.596 | CH(CH₃)-CCl=CH-Cl | (Z) |
| A.597 | CH(CH₃)-CH=CCl₂ | |
| A.598 | CH(CH₃)-CCl=CCl₂ | |
| A.599 | CH(CH₃)-CBr=CH₂ | |
| A.600 | CH(CH₃)-CH=CH-Br | (E) |
| A.601 | CH(CH₃)-CH=CH-Br | (Z) |
| A.602 | CH(CH₃)-CBr=CH-Br | (E) |
| A.603 | CH(CH₃)-CBr-CH-Br | (Z) |
| A.604 | CH(CH₃)-CH=CBr₂ | |
| A.605 | CH(CH₃)-CBr=CBr₂ | |
| A.606 | CH(CH₃)-C(CH₃)=CH₂ | |
| A.607 | CH(CH₃)-CH=CH-CH₃ | (E) |
| A.608 | CH(CH₃)-CH=CH-CH₃ | (Z) |
| A.609 | CH(CH₃)-C(CH₃)=CH-CH₃ | (E) |
| A.610 | CH(CH₃)-C(CH₃)=CH-CH₃ | (Z) |
| A.611 | CH(CH₃)-CH=C(CH₃)₂ | |
| A.612 | CH(CH₃)-CCl=CH-CH₃ | (E) |
| A.613 | CH(CH₃)-CCl=CH-CH₃ | (Z) |
| A.614 | CH(CH₃)-CH=CCl-CH₃ | (E) |
| A.615 | CH(CH₃)-CH=CCl-CH₃ | (Z) |
| A.616 | CH(CH₃)-CBr=CH-CH₃ | (E) |
| A.617 | CH(CH₃)-CBr=CH-CH₃ | (Z) |
| A.618 | CH(CH₃)-CH=CBr-CH₃ | (E) |
| A.619 | CH(CH₃)-CH=CBr-CH₃ | (Z) |
| A.620 | CH(CH₃)-CH=CH-CH₂Cl | (E) |
| A.621 | CH(CH₃)-CH=CH-CH₂Cl | (Z) |
| A.622 | CH(CH₃)-CH=CH-CH₂CH₃ | (E) |
| A.623 | CH(CH₃)-CH=CH-CH₂CH₃ | (Z) |
| A.624 | CH(CH₃)-CH=CH-CH₂Br | (E) |
| A.625 | CH(CH₃)-CH=CH-CH₂Br | (Z) |
| A.626 | CH(CH₃)-CCl=CCl-CH₂Cl | (E) |
| A.627 | CH(CH₃)-CCl=CCl-CH₂Cl | (Z) |
| A.628 | CH(CH₃)-CF=CH₂ | |
| A.629 | CH(CH₃)-CH=CH-F | (E) |
| A.630 | CH(CH₃)-CH=CH-F | (Z) |
| A.631 | CH(CH₃)-CH=CF₂ | |
| A.632 | CH(CH₃)-CF=CH-F | (E) |
| A.633 | CH(CH₃)-CF=CH-F | (Z) |
| A.634 | CH₂CHCl-CH=CH₂ | |
| A.635 | CH₂CH₂-CH=C(CH₃)₂ | |
| A.636 | CH₂CH₂-C(CH₃)=CH-CH₃ | (E) |
| A.637 | CH₂CH₂-C(CH₃)=CH-CH₃ | (Z) |
| A.638 | C(CH₃)=CH-[C₆H₅] | (E) |
| A.639 | C(CH₃)=CH-[2-CN-C₆H₄] | (E) |
| A.640 | C(CH₃)=CH-[3-CN-C₆H₄] | (E) |
| A.641 | C(CH₃)=CH-[4-CN-C₆H₄] | (E) |
| A.642 | C(CH₃)=CH-[2-F-C₆H₄] | (E) |
| A.643 | C(CH₃)=CH-[3-F-C₆H₄] | (E) |
| A.644 | C(CH₃)=CH-[4-F-C₆H₄] | (E) |
| A.645 | C(CH₃)=CH-[2,4-F₂-C₆H₃] | (E) |
| A.646 | C(CH₃)=CH-[3,4-F₂-C₆H₃₃] | (E) |
| A.647 | C(CH₃)=CH-[2,4,5-F₃-C₆H₂] | (E) |
| A.648 | C(CH₃)=CH-[2,4,6-F₃-C₆H₂] | (E) |
| A.649 | C(CH₃)=CH-[2-Cl-C₆H₄] | (E) |
| A.650 | C(CH₃)=CH-[3-Cl-C₆H₄] | (E) |
| A.651 | C(CH₃)=CH-[4-Cl-C₆H₄] | (E) |
| A.652 | C(CH₃)=CH-[2,4-Cl₂-C₆H₃] | (E) |
| A.653 | C(CH₃)=CH-[3,4-Cl₂-C₆H₃] | (E) |
| A.654 | C(CH₃)=CH-[2,4,5-Cl₃-C₆H₂] | (E) |
| A.655 | C(CH₃)=CH-[2,4,6-Cl₃-C₆H₂] | (E) |
| A.656 | C(CH₃)=CH-[2-CH₃-C₆H₄] | (E) |
| A.657 | C(CH₃)=CH-[3-CH₃-C₆H₄] | (E) |
| A.658 | C(CH₃)=CH-[4-CH₃-C₆H₄] | (E) |
| A.659 | C(CH₃)=CH-[2-F, 4-CH₃-C₆H₃] | (E) |
| A.660 | C(CH₃)=CH-[2-CH₃, 4-F-C₆H₃] | (E) |
| A.661 | C(CH₃)=CH-[3-F, 4-CH₃-C₆H₃] | (E) |
| A.662 | C(CH₃)=CH-[3-CH₃, 4-F-C₆H₃] | (E) |
| A.663 | C(CH₃)=CH-[2-Cl, 4-CH₃-C₆H₃] | (E) |
| A.664 | C(CH₃)=CH-[2-CH₃, 4-Cl-C₆H₃] | (E) |
| A.665 | C(CH₃)=CH-[3-Cl, 4-CH₃-C₆H₃] | (E) |
| A.666 | C(CH₃)=CH-[3-CH₃, 4-Cl-C₆H₃] | (E) |
| A.667 | C(CH₃)=CH-[2,4-(CH₃)₂-C₆H₃] | (E) |
| A.668 | C(CH₃)=CH-[3,4-(CH₃)₂-C₆H₃] | (E) |
| A.669 | C(CH₃)=CH-[2,4,5-(CH₃)₃-C₆H₂] | (E) |
| A.670 | C(CH₃)=CH-[2,4,6-(CH₃)₃-C₆H₂] | (E) |
| A.671 | C(CH₃)=CH-[2-CF₃-C₆H₄] | (E) |
| A.672 | C(CH₃)=CH-[3-CF₃-C₆H₄] | (E) |
| A.673 | C(CH₃)=CH-[4-CF₃-C₆H₄] | (E) |
| A.674 | C(CH₃)=CH-[2-F, 4-CF₃-C₆H₃] | (E) |
| A.675 | C(CH₃)=CH-[2-CF₃, 4-F-C₆H₃] | (E) |
| A.676 | C(CH₃)=CH-[3-F, 4-CF₃-C₆H₃] | (E) |
| A.677 | C(CH₃)=CH-[3-CF₃, 4-F-C₆H₃] | (E) |
| A.678 | C(CH₃)=CH-[2-Cl, 4-CF₃-C₆H₃] | (E) |
| A.679 | C(CH₃)=CH-[2-CF₃, 4-Cl-C₆H₃] | (E) |
| A.680 | C(CH₃)=CH-[3-Cl, 4-CF₃-C₆H₃] | (E) |
| A.681 | C(CH₃)=CH-[3-CF₃, 4-Cl-C₆H₃] | (E) |
| A.682 | C(CH₃)=CH-[2,4-(CF₃)₂-C₆H₃] | (E) |
| A.683 | C(CH₃)=CH-[3,4-(CF₃)₂-C₆H₃] | (E) |
| A.684 | C(CH₃)=CH-[2,4,5-(CF₃)₃-C₆H₂] | (E) |
| A.685 | C(CH₃)=CH-[2,4,6-(CF₃)₃-C₆H₂] | (E) |
| A.686 | C(CH₃)=CH-[2-OCH₃-C₆H₄] | (E) |
| A.687 | C (CH₃)=CH-[3-OCH₃-C₆H₄] | (E) |
| A.688 | C(CH₃)=CH-[4-OCH₃-C₆H₄] | (E) |
| A.689 | C(CH₃)=CH-[2-F, 4-OCH₃-C₆H₃] | (E) |
| A.690 | C(CH₃)=CH-[2-OCH₃, 4-F-C₆H₃] | (E) |
| A.691 | C(CH₃)=CH-[3-F, 4-OCH₃-C₆H₃] | (E) |
| A.692 | C(CH₃)=CH-[3-OCH₃, 4-F-C₆H₃] | (E) |
| A.693 | C(CH₃)=CH-[2-Cl, 4-OCH₃-C₆H₃] | (E) |
| A.694 | C(CH₃)=CH-[2-OCH₃, 4-Cl-C₆H₃] | (E) |
| A.695 | C(CH₃)=CH-[3-Cl, 4-OCH₃-C₆H₃] | (E) |
| A.696 | C(CH₃)=CH-[3-OCH₃, 4-Cl-C₆H₃] | (E) |
| A.697 | C(CH₃)=CH-[2,4-(OCH₃)₂-C₆H₃] | (E) |
| A.698 | C(CH₃)=CH-[3,4-(OCH₃)₂-C₆H₃] | (E) |
| A.699 | C(CH₃)=CH-[2,4,5-(OCH₃)₃-C₆H₂] | (E) |
| A.700 | C(CH₃)=CH-[2,4,6-(OCH₃)₃-C₆H₂] | (E) |
| A.701 | C(CH₃)=CH-(2-OCHF₂-C₆H₄] | (E) |
| A.702 | C(CH₃)=CH-[3-OCHF₂-C₆H₄] | (E) |
| A.703 | C(CH₃)=CH-[4-OCHF₂-C₆H₄] | (E) |
| A.704 | C(CH₃)=CH-[2-F, 4-OCHF₂-C₆H₃] | (E) |
| A.705 | C(CH₃)=CH-[2-OCHF₂, 4-F-C₆H₃] | (E) |
| A.706 | C(CH₃)=CH-[3-F, 4-OCHF₂-C₆H₃] | (E) |
| A.707 | C(CH₃)=CH-[3-OCHF₂, 4-F-C₆H₃] | (E) |
| A.708 | C(CH₃)=CH-[2-Cl, 4-OCHF₂-C₆H₃] | (E) |
| A.709 | C(CH₃)=CH-[2-OCHF₂, 4-Cl-C₆H₃] | (E) |
| A.710 | C(CH₃)=CH-[3-Cl, 4-OCHF₂-C₆H₃] | (E) |
| A.711 | C(CH₃)=CH-[3-OCHF₂, 4-Cl-C₆H₃] | (E) |
| A.712 | C(CH₃)=CH-[2,4-(OCHF₂)₂-C₆H₃] | (E) |
| A.713 | C(CH₃)=CH-[3,4-(OCHF₂)₂-C₆H₃] | (E) |
| A. 714 | C(CH₃)=CH-[2,4,5-(OCHF₂)₃-C₆H₂] | (E) |
| A.715 | C(CH₃)=CH-[2,4,6-(OCHF₂)₃-C₆H₂] | (E) |
| A.716 | CH=C(CH₃)-[C₆H₅] | (E) |
| A.717 | CH=C(CH₃)-[2-CN-C₆H₄] | (E) |
| A.718 | CH=C(CH₃)-[3-CN-C₆H₄] | (E) |
| A.719 | CH=C(CH₃)-[4-CN-C₆H₄] | (E) |
| A.720 | CH=C(CH₃)-[2-F-C₆H₄] | (E) |
| A.721 | CH=C(CH₃)-[3-F-C₆H₄] | (E) |
| A.722 | CH=C(CH₃)-[4-F-C₆H₄] | (E) |
| A.723 | CH=C(CH₃)-[2,4-F₂-C₆H₃] | (E) |
| A.724 | CH=C(CH₃)-[3,4-F₂-C₆H₃] | (E) |
| A.725 | CH=C(CH₃)-[2,4,5-F₃-C₆H₂] | (E) |
| A.726 | CH=C(CH₃)-[2,4,6-F₃-C₆H₂] | (E) |
| A.727 | CH=C(CH₃)-[2-Cl-C₆H₄] | (E) |
| A.728 | CH=C(CH₃)-[3-Cl-C₆H₄] | (E) |
| A.729 | CH=C(CH₃)-[4-Cl-C₆H₄] | (E) |
| A.730 | CH=C(CH₃)-[2,4-Cl₂-C₆H₃] | (E) |
| A.731 | CH=C(CH₃)-[3,4-Cl₂-C₆H₃] | (E) |
| A.732 | CH=C(CH₃)-[2,4,5-Cl₃-C₆H₂] | (E) |
| A.733 | CH=C(CH₃)-[2,4,6-Cl₃-C₆H₂] | (E) |
| A.734 | CH=C(CH₃)-[2-CH₃-C₆H₄] | (E) |
| A.735 | CH=C(CH₃)-[3-CH₃-C₆H₄] | (E) |
| A.736 | CH=C(CH₃)-[4-CH₃-C₆H₄] | (E) |
| A.737 | CH=C(CH₃)-[2-F, 4-CH₃-C₆H₃] | (E) |
| A.738 | CH=C(CH₃)-[2-CH₃, 4-F-C₆H₃] | (E) |
| A.739 | CH=C(CH₃)-[3-F, 4-CH₃-C₆H₃] | (E) |
| A.740 | CH=C(CH₃)-[3-CH₃, 4-F-C₆H₃] | (E) |
| A.741 | CH=C(CH₃)-[2-Cl, 4-CH₃-C₆H₃] | (E) |
| A.742 | CH=C(CH₃)-[2-CH₃, 4-Cl-C₆H₃] | (E) |
| A.743 | CH=C(CH₃)-[3-Cl, 4-CH₃-C₆H₃] | (E) |
| A.744 | CH=C(CH₃)-[3-CH₃, 4-Cl-C₆H₃] | (E) |
| A.745 | CH=C(CH₃)-[2,4-(CH₃)₂-C₆H₃] | (E) |
| A.746 | CH=C(CH₃)-[3,4-(CH₃)₂-C₆H₃] | (E) |
| A.747 | CH=C (CH₃)-[2,4,5-(CH₃)₃-C₆H₂] | (E) |
| A.748 | CH=C(CH₃)-[2,4,6-(CH₃)₃-C₆H₂] | (E) |
| A.749 | CH=C(CH₃)-[2-CF₃-C₆H₄] | (E) |
| A.750 | CH=C(CH₃)-[3-CF₃-C₆H₄] | (E) |
| A.751 | CH=C(CH₃)-[4-CF₃-C₆H₄] | (E) |
| A.752 | CH=C(CH₃)-[2-F, 4-CF₃-C₆H₃] | (E) |
| A.753 | CH=C(CH₃)-[2-CF₃, 4-F-C₆H₃] | (E) |
| A.754 | CH=C(CH₃)-[3-F, 4-CF₃-C₆H₃] | (E) |
| A.755 | CH=C(CH₃)-[3-CF₃, 4-F-C₆H₃] | (E) |
| A.756 | CH=C(CH₃)-[2-Cl, 4-CF₃-C₆H₃] | (E) |
| A.757 | CH=C(CH₃)-[2-CF₃, 4-Cl-C₆H₃] | (E) |
| A.758 | CH=C(CH₃)-[3-Cl, 4-CF₃-C₆H₃] | (E) |
| A.759 | CH=C(CH₃)-[3-CF₃, 4-Cl-C₆H₃] | (E) |
| A.760 | CH=C(CH₃)-[2,4-(CF₃)₂-C₆H₃] | (E) |
| A.761 | CH=C(CH₃)-[2,4,5-(CF₃)₃-C₆H₃] | (E) |
| A.762 | CH=C(CH₃)-[2,4,5-(CF₃)₃-C₆H₂] | (E) |
| A.763 | CH=C(CH₃)-[2,4,6-(CF₃)₃-C₆H₂] | (E) |
| A.764 | CH=C(CH₃)-[2-OCH₃-C₆H₄] | (E) |
| A.765 | CH=C(CH₃)-[3-OCH₃-C₆H₄] | (E) |
| A.766 | CH=C(CH₃)-[4-OCH₃-C₆H₄] | (E) |
| A.767 | CH=C(CH₃)-[2-F, 4-OCH₃-C₆H₃] | (E) |
| A.768 | CH=C(CH₃)-[2-OCH₃, 4-F-C₆H₃] | (E) |
| A.769 | CH=C(CH₃)-[3-F, 4-OCH₃-C₆H₃] | (E) |
| A.770 | CH=C(CH₃)-[3-OCH₃, 4-F-C₆H₃] | (E) |
| A.771 | CH=C(CH₃)-[2-Cl, 4-OCH₃-C₆H₃] | (E) |
| A.772 | CH=C(CH₃)-[2-OCH₃, 4-Cl-C₆H₃] | (E) |
| A.773 | CH=C(CH₃)-[3-Cl, 4-OCH₃-C₆H₃] | (E) |
| A.774 | CH=C(CH₃)-[3-OCH₃, 4-Cl-C₆H₃] | (E) |
| A.775 | CH=C(CH₃)-[2,4-(OCH₃)₂-C₆H₃] | (E) |
| A.776 | CH=C(CH₃)-[3,4-(OCH₃)₂-C₆H₃] | (E) |
| A.777 | CH=C(CH₃]-[2,4,5-(OCH₃)₃-C₆H₂] | (E) |
| A.778 | CH=C(CH₃)-[2,4,6-(OCH₃)₃-C₆H₂] | (E) |
| A.779 | CH=C(CH₃)-[2-OCHF₂-C₆H₄] | (E) |
| A.780 | CH=C(CH₃)-[3-OCHF₂-C₆H₄] | (E) |
| A.781 | CH=C(CH₃)-[4-OCHF₂-C₆H₄] | ( E ) |
| A.782 | CH=C(CH₃)-[2-F, 4-OCHF₂-C₆H₃] | (E) |
| A.783 | CH=C(CH₃)-[2-OCHF₂, 4-F-C₆H₃] | (E) |
| A.784 | CH=C(CH₃)-[3-F, 4-OCHF₂-C₆H₃] | (E) |
| A.785 | CH=C(CH₃)-[3-OCHF₂, 4-F-C₆H₃] | (E) |
| A.786 | CH=C(CH₃)-[2-Cl, 4-OCHF₂-C₆H₃] | (E) |
| A.787 | CH=C(CH₃)-[2-OCHF₂, 4-Cl-C₆H₃] | (E) |
| A.788 | CH=C(CH₃)-[3-Cl, 4-OCHF₂-C₆H₃] | (E) |
| A.789 | CH=C(CH₃)-[3-OCHF₂, 4-Cl-C₆H₃] | (E) |
| A.790 | CH=C(CH₃)-[2,4-(OCHF₂)₂-C₆H₃] | (E) |
| A.791 | CH=C(CH₃)-[3,4-(OCHF₂)₂-C₆H₃] | (E) |
| A.792 | CH=C(CH₃)-[2,4,5-(OCHF₂)₃-C₆H₂] | (E) |
| A.793 | CH=C(CH₃)-[2,4,6-(OCHF₂)₃-C₆H₂] | (E) |
| A.794 | C≡C-I | |
| A.795 | CH₂-C≡C-H | |
| A.796 | CH₂-C≡C-Cl | |
| A.797 | CH₂-C≡C-Br | |
| A.798 | CH₂-C≡C-J | |
| A.799 | CH₂-C≡C-CH₃ | |
| A.800 | CH₂-C≡C-CH₂CH₃ | |
| A.801 | CH₂CH₂-C≡C-H | |
| A.802 | CH₂CH₂-C≡C-Cl | |
| A.803 | CH₂CH₂-C≡C-Br | |
| A.804 | CH₂CH₂-C≡C-J | |
| A.805 | CH₂CH₂-C≡C-CH₃ | |
| A.806 | CH₂CH₂CH₂-C≡C-H | |
| A.807 | CH₂CH₂CH₂-C≡C-Cl | |
| A.808 | CH₂CH₂CH₂-C≡C-Br | |
| A.809 | CH₂CH₂CH₂-C≡C-J | |
| A.810 | CH₂CH₂CH₂-C≡C-CH₃ | |
| A.811 | CH(CH₃)-C≡C-H | |
| A.812 | CH(CH₃)-C≡C-Cl | |
| A.813 | CH(CH₃)-C≡C-Br | |
| A.814 | CH(CH₃)-C≡C-J | |
| A.815 | CH(CH₃)-C≡C-CH₃ | |
| A.816 | C≡C-[4-F-C₆H₄] | |

**Tabelle B**

| **Nr.** | **R**^{**2**} | |
|---|---|---|
| B.1 | CH₂CH₃ | |
| B.2 | CH₂CH₂-CN | |
| B.3 | CH₂CH₂-O-CH₃ | |
| B.4 | CH₂CH₂-O-CH₂CH₃ | |
| B.5 | CH₂CH₂CH₃ | |
| B.6 | CH(CH₃)₂ | |
| B.7 | CH₂CH₂CH₂CH₃ | |
| B.8 | CH(CH₃)CH₂CH₃ | |
| B.9 | CH₂CH(CH₃)₂ | |
| B.10 | CH=CH₂ | |
| B.11 | CH=CH-CH₃ | (E) |
| B.12 | CH=CH-[C₆H₅] | (E) |
| B.13 | CH=CH- [2-CN-C₆H₄] | (E) |
| B.14 | CH=CH-[3-CN-C₆H₄] | (E) |
| B.15 | CH=CH-[4-CN-C₆H₄] | (E) |
| B.16 | CH=CH-[2-F-C₆H₄] | (E) |
| B.17 | CH=CH-[3-F-C₆H₄] | (E) |
| B.18 | CH=CH-[4-F-C₆H₄] | (E) |
| B.19 | CH=CH-[2,4-F₂-C₆H₃] | (E) |
| B.20 | CH=CH-(3,4-F₂-C₆H₃] | (E) |
| B.21 | CH=CH-[2,4,5-F₃-C₆H₂] | (E) |
| B.22 | CH=CH-[2,4,6-F₃-C₆H₂] | (E) |
| B.23 | CH=CH-[2-Cl-C₆H₄] | (E) |
| B.24 | CH=CH-[3-Cl-C₆H₄] | (E) |
| B.25 | CH=CH-[4-Cl-C₆H₄] | (E) |
| B.26 | CH=CH-(2,4-Cl₂-C₆H₃] | (E) |
| B.27 | CH=CH-[3,4-Cl₂-C₆H₃] | (E) |
| B.28 | CH=CH-[2,4,5-Cl₃-C₆H₂] | (E) |
| B.29 | CH=CH-[2,4,6-Cl₃-C₆H₂] | (E) |
| B.30 | CH=CH-[2-CH₃-C₆H₄] | (E) |
| B.31 | CH=CH-[3-CH₃-C₆H₄] | (E) |
| B.32 | CH=CH-[4-CH₃-C₆H₄] | (E) |
| B.33 | CH=CH-[2-F, 4-CH₃-C₆H₃] | (E) |
| B.34 | CH=CH-[2-CH₃, 4-F-C₆H₃] | (E) |
| B.35 | CH=CH-[3-F, 4-CH₃-C₆H₃] | (E) |
| B.36 | CH=CH-[3-CH₃, 4-F-C₆H₃] | (E) |
| B.37 | CH=CH-[2-Cl, 4-CH₃-C₆H₃] | (E) |
| B.38 | CH=CH-[2-CH₃, 4-Cl-C₆H₃] | (E) |
| B.39 | CH=CH-[3-Cl, 4-CH₃-C₆H₃] | (E) |
| B.40 | CH=CH-[3-CH₃, 4-Cl-C₆H₃] | (E) |
| B.41 | CH=CH-[2-CF₃-C₆H₄] | (E) |
| B.42 | CH=CH-[3-CF₃-C₆H₄] | (E) |
| B.43 | CH=CH-[4-CF₃-C₆H₄] | (E) |
| B.44 | CH=CH-[2-OCH₃-C₆H₄] | (E) |
| B.45 | CH=CH-[3-OCH₃-C₆H₄] | (E) |
| B.46 | CH=CH-[4-OCH₃-C₆H₄] | (E) |
| B.47 | CH=CH-[2-OCHF₂-C₆H₄] | (E) |
| B.48 | CH=CH-[3-OCHF₂-C₆H₄] | (E) |
| B.49 | CH=CH-[4-OCHF₂-C₆H₄] | (E) |
| B.50 | CH₂CH=CH₂ | |
| B.51 | CH₂CH=CH-Cl | (E) |
| B.52 | CH₂CH=CH-CH₃ | (E) |
| B.53 | C≡CH | |
| B.54 | C≡C-Cl | |
| B.55 | C≡C-Br | |
| B.56 | C≡C-CH₃ | |
| B.57 | C≡C-C₆H₅ | |
| B.58 | C≡C-[2-Cl-C₆H₄] | |
| B.59 | C≡C-[4-Cl-C₆H₄] | |
| B.60 | C≡C-[2,4-Cl₂-C₆H₃] | |
| B.61 | C≡C-[2-CH₃-C₆H₄] | |
| B.62 | C≡C-[4-CH₃-C₆H₄] | |
| B.63 | C≡C-[2,4-(CH₃)₂-C₆H₃] | |
| B.64 | C≡C-[2-Cl, 4-CH₃-C₆H₃] | |
| B.65 | C≡C-[2-CH₃, 4-Cl-C₆H₃] | |
| B.66 | C≡C-[3-CF₃-C₆H₄] | |
| B.67 | C≡C-[3-Cl, 5-CF₃-C₆H₃] | |
| B.68 | C≡C-[2-OCH₃-C₆H₄] | |
| B.69 | C≡C-[4-OCH₃-C₆H₄] | |
| B.70 | C≡C-[2,4-(OCH₃)₂-C₆H₃] | |
| B.71 | C≡C-[2-Cl, 4-OCH₃-C₆H₃] | |
| B.72 | C≡C-[2-OCH₃, 4-Cl-C₆H₃] | |
| B.73 | C≡C-[3-OCHF₂-C₆H₄] | |
| B.74 | C≡C-[3-Cl, 5-OCHF₂-C₆H₃] | |
| B.75 | cyclopentyl | |
| B.76 | 1-CH₃-cyclopentyl | |
| B.77 | 2-CH₃-cyclopentyl | |
| B.78 | 3-CH₃-cyclopentyl | |
| B.79 | 2,3-(CH₃)₂-cyclopentyl | |
| B.80 | 1-Cl-cyclopentyl | |
| B.81 | 2-Cl-cyclopentyl | |
| B.82 | 3-Cl-cyclopentyl | |
| B.83 | 2-CH₃, 3-Cl-cyclopentyl | |
| B.84 | 2,3-Cl₂-cyclopentyl | |
| B.85 | cyclohexyl | |
| B.86 | 1-CH₃-cyclohexyl | |
| B.87 | 2-CH₃-cyclohexyl | |
| B.88 | 3-CH₃-cyclohexyl | |
| B.89 | 2,3-(CH₃)₂-cyclohexyl | |
| B.90 | 3,3-(CH₃)₂-cyclohexyl | |
| B.91 | 1-Cl-cyclohexyl | |
| B.92 | 2-Cl-cyclohexyl | |
| B.93 | 3-Cl-cyclohexyl | |
| B.94 | 2-CH₃, 3-Cl-cyclohexyl | |
| B.95 | 2,3-Cl₂-cyclohexyl | |
| B.96 | C₆H₅ | |
| B.97 | 2-CN-C₆H₄ | |
| B.98 | 3-CN-C₆H₄ | |
| B.99 | 4-CN-C₆H₄ | |
| B.100 | 2-F-C₆H₄ | |
| B.101 | 3-F-C₆H₄ | |
| B.102 | 4-F-C₆H₄ | |
| B.103 | 2,4-F₂-C₆H₃ | |
| B.104 | 3,4-F₂-C₆H₃ | |
| B.105 | 2,4,5-F₃-C₆H₂ | |
| B.106 | 2,4,6-F₃-C₆H₂ | |
| B.107 | 2-Cl-C₆H₄ | |
| B.108 | 3-Cl-C₆H₄ | |
| B.109 | 4-Cl-C₆H₄ | |
| B.110 | 2,4-Cl₂-C₆H₃ | |
| B.111 | 3,4-Cl₂-C₆H₃ | |
| B.112 | 2,4,5-Cl₃-C₆H₂ | |
| B.113 | 2,4,6-Cl₃-C₆H₂ | |
| B.114 | 2-CH₃-C₆H₄ | |
| B.115 | 3-CH₃-C₆H₄ | |
| B.116 | 4-CH₃-C₆H₄ | |
| B.117 | 2-F, 4-CH₃-C₆H₃ | |
| B.118 | 2-CH₃, 4-F-C₆H₃ | |
| B.119 | 3-F, 4-CH₃-C₆H₃ | |
| B.120 | 3-CH₃, 4-F-C₆H₃ | |
| B.121 | 2-Cl, 4-CH₃-C₆H₃ | |
| B.122 | 2-CH₃, 4-Cl-C₆H₃ | |
| B.123 | 3-Cl, 4-CH₃-C₆H₃ | |
| B.124 | 3-CH₃, 4-Cl-C₆H₃ | |
| B.125 | 2,4-(CH₃)₂-C₆H₃ | |
| B.126 | 3,4-(CH₃)₂-C₆H₃ | |
| B.127 | 2,4,5-(CH₃)₃-C₆H₂ | |
| B.128 | 2,4,6-(CH₃)₃-C₆H₂ | |
| B.129 | 2-CF₃-C₆H₄ | |
| B.130 | 3-CF₃-C₆H₄ | |
| B.131 | 4-CF₃-C₆H₄ | |
| B.132 | 2-F, 4-CF₃-C₆H₃ | |
| B.133 | 2-CF₃, 4-F-C₆H₃ | |
| B.134 | 3-F, 4-CF₃-C₆H₃ | |
| B.135 | 3-CF₃, 4-F-C₆H₃ | |
| B.136 | 2-Cl, 4-CF₃-C₆H₃ | |
| B.137 | 2-CF₃, 4-Cl-C₆H₃ | |
| B.138 | 3-Cl, 4-CF₃-C₆H₃ | |
| B.139 | 3-CF₃, 4-Cl-C₆H₃ | |
| B.140 | 2,4-(CF₃)₂-C₆H₃ | |
| B.141 | 3,4-(CF₃)₂-C₆H₃ | |
| B.142 | 2,4,5-(CF₃)₃-C₆H₂ | |
| B.143 | 2,4,6-(CF₃)₃-C₆H₂ | |
| B.144 | 2-OCH₃-C₆H₄ | |
| B.145 | 3-OCH₃-C₆H₄ | |
| B.146 | 4-OCH₃-C₆H₄ | |
| B.147 | 2-F, 4-OCH₃-C₆H₃ | |
| B.148 | 2-OCH₃, 4-F-C₆H₃ | |
| B.149 | 3-F, 4-OCH₃-C₆H₃ | |
| B.150 | 3-OCH₃, 4-F-C₆H₃ | |
| B.151 | 2-Cl, 4-OCH₃-C₆H₃ | |
| B.152 | 2-OCH₃, 4-Cl-C₆H₃ | |
| B.153 | 3-Cl, 4-OCH₃-C₆H₃ | |
| B.154 | 3-OCH₃, 4-Cl-C₆H₃ | |
| B.155 | 2,4-(OCH₃)₂-C₆H₃ | |
| B.156 | 3,4-(OCH₃)₂-C₆H₃ | |
| B.157 | 2,4,5-(OCH₃)₃-C₆H₂ | |
| B.158 | 2,4,6-(OCH₃)₃-C₆H₂ | |
| B.159 | 2-OCHF₂-C₆H₄ | |
| B.160 | 3-OCHF₂-C₆H₄ | |
| B.161 | 4-OCHF₂-C₆H₄ | |
| B.162 | 2-F, 4-OCHF₂-C₆H₃ | |
| B.163 | 2-OCHF₂, 4-F-C₆H₃ | |
| B.164 | 3-F, 4-OCHF₂-C₆H₃ | |
| B.165 | 3-OCHF₂, 4-F-C₆H₃ | |
| B.166 | 2-Cl, 4-OCHF₂-C₆H₃ | |
| B.167 | 2-OCHF₂, 4-Cl-C₆H₃ | |
| B.168 | 3-Cl, 4-OCHF₂-C₆H₃ | |
| B.169 | 3-OCHF₂, 4-Cl-C₆H₃ | |
| B.170 | 2,4-(OCHF₂)₂-C₆H₃ | |
| B.171 | 3,4-(OCHF₂)₂-C₆H₃ | |
| B.172 | 2,4,5-(OCHF₂)₃-C₆H₂ | |
| B.173 | 2,4,6-(OCHF₂)₃-C₆H₂ | |
| B.174 | CH₂-C₆H₅ | |
| B.175 | CH₂-[2-CN-C₆H₄] | |
| B.176 | CH₂-[3-CN-C₆H₄] | |
| B.177 | CH₃-[4-CN-C₆H₄] | |
| B.178 | CH₂-[2-F-C₆H₄] | |
| B.179 | CH₂-[3-F-C₆H₄] | |
| B.180 | CH₂-[4-F-C₆H₄] | |
| B.181 | CH₂-[2,4-F₂-C₆H₃] | |
| B.182 | CH₂-[3,4-F₂-C₆H₃] | |
| B.183 | CH₂-[2,4,5-F₃-C₆H₂] | |
| B.184 | CH₂-[2,4,6-F₃-C₆H₂] | |
| B.185 | CH₂-[2-Cl-C₆H₄] | |
| B.186 | CH₂-[3-Cl-C₆H₄] | |
| B.187 | CH₂-[4-Cl-C₆H₄] | |
| B.188 | CH₂-[2,4-Cl₂-C₆H₃] | |
| B.189 | CH₂-[3,4-Cl₂-C₆H₃] | |
| B.190 | CH₂-[2,4,5-Cl₃-C₆H₂] | |
| B.191 | CH₂-[2,4,6-Cl₃-C₆H₂] | |
| B.192 | CH₂-[2-CH₃-C₆H₄] | |
| B.193 | CH₂-[3-CH₃-C₆H₄] | |
| B.194 | CH₂-[4-CH₃-C₆H₄] | |
| B.195 | CH₂-[2-F, 4-CH₃-C₆H₃] | |
| B.196 | CH₂-[2-CH₃, 4-F-C₆H₃] | |
| B.197 | CH₂-[3-F, 4-CH₃-C₆H₃] | |
| B.198 | CH₂-[3-CH₃, 4-F-C₆H₃] | |
| B.199 | CH₂-[2-Cl, 4-CH₃-C₆H₃] | |
| B.200 | CH₂-[2-CH₃, 4-Cl-C₆H₃] | |
| B.201 | CH₂-[3-Cl, 4-CH₃-C₆H₃] | |
| B.202 | CH₂-[3-CH₃, 4-Cl-C₆H₃] | |
| B.203 | CH₂-[2-CF₃-C₆H₄] | |
| B.204 | CH₂-[3-CF₃-C₆H₄] | |
| B.205 | CH₂-[4-CF₃-C₆H₄] | |
| B.206 | CH₂-[2-OCH₃-C₆H₄] | |
| B.207 | CH₂-[3-OCH₃-C₆H₄] | |
| B.208 | CH₂-[4-OCH₃-C₆H₄] | |
| B.209 | CH₂-[2-OCHF₂-C₆H₄] | |
| B.210 | CH₂-[3-OCHF₂-C₆H₄] | |
| B.211 | CH₂-[4-OCHF₂-C₆H₄] | |
| B.212 | CH(CH₃)-C₆H₅ | |
| B.213 | CH(CH₃)-[2-CN-C₆H₄] | |
| B.214 | CH(CH₃)-[3-CN-C₆H₄] | |
| B.215 | CH(CH₃)-[4-CN-C₆H₄] | |
| B.216 | CH(CH₃)-[2-F-C₆H₄] | |
| B.217 | CH(CH₃)-[3-F-C₆H₄] | |
| B.218 | CH(CH₃)-[4-F-C₆H₄] | |
| B.219 | CH(CH₃)-[2,4-F₂-C₆H₃] | |
| B.220 | CH(CH₃)-[3,4-F₂-C₆H₃] | |
| B.221 | CH(CH₃)-[2,4,5-F₃-C₆H₂] | |
| B.222 | CH(CH₃)-[2,4,6-F₃-C₆H₂] | |
| B.223 | CH(CH₃)-[2-Cl-C₆H₄] | |
| B.224 | CH(CH₃)-[3-Cl-C₆H₄] | |
| B.225 | CH(CH₃)-[4-Cl-C₆H₄] | |
| B.226 | CH(CH₃)-[2,4-Cl₂-C₆H₃] | |
| B.227 | CH(CH₃)-[3,4-Cl₂-C₆H₃] | |
| B.228 | CH(CH₃)-[2,4,5-Cl₃-C₆H₂] | |
| B.229 | CH(CH₃)-[2,4,6-Cl₃-C₆H₂] | |
| B.230 | CH(CH₃)-[2-CH₃-C₆H₄] | |
| B.231 | CH(CH₃)-[3-CH₃-C₆H₄] | |
| B.232 | CH(CH₃)-[4-CH₃-C₆H₄] | |
| B.233 | CH (CH₃)-[2-F, 4-CH₃-C₆H₃] | |
| B.234 | CH(CH₃)-[2-CH₃, 4-F-C₆H₃] | |
| B.235 | CH(CH₃)-[3-F, 4-CH₃-C₆H₃] | |
| B.236 | CH(CH₃)-[3-CH₃, 4-F-C₆H₃] | |
| B.237 | CH(CH₃)-[2-Cl, 4-CH₃-C₆H₃] | |
| B.238 | CH(CH₃)-[2-CH₃, 4-Cl-C₆H₃] | |
| B.239 | CH(CH₃)-[3-Cl, 4-CH₃-C₆H₃] | |
| B.240 | CH(CH₃)-[3-CH₃, 4-Cl-C₆H₃] | |
| B.241 | CH(CH₃)-[2-CF₃-C₆H₄] | |
| B.242 | CH(CH₃)-[3-CF₃-C₆H₄] | |
| B.243 | CH(CH₃)-[4-CF₃-C₆H₄] | |
| B.244 | CH(CH₃)-[2-OCH₃-C₆H₄] | |
| B.245 | CH(CH₃)-[3-OCH₃-C₆H₄] | |
| B.246 | CH(CH₃)-[4-OCH₃-C₆H₄] | |
| B.247 | CH(CH₃)-[2-OCHF₂-C₆H₄] | |
| B.248 | CH(CH₃)-[3-OCHF₂-C₆H₄] | |
| B.249 | CH(CH₃)-[4-OCHF₂-C₆H₄] | |
| B.250 | CH₂F | |
| B.251 | CHF₂ | |
| B.252 | CF₃ | |
| B.253 | CH₂-CN | |
| B.254 | CH₂-OCH₃ | |
| B.255 | CH₂-OCH₂CH₃ | |
| B.256 | CH₂-OCH₂CH₂CH₃ | |
| B.257 | CH₂-OCH(CH₃)₂ | |
| B.258 | CH₂-OCH₂CH₂CH₂CH₃ | |
| B.259 | CH₂-OCH(CH₃)CH₂CH₃ | |
| B.260 | CH₂-OCH₂CH(CH₃)₂ | |
| B.261 | CH₂-OC(CH₃)₃ | |
| B.262 | CH₂-OCF₃ | |
| B.263 | CH₂-OCH₂CF₃ | |
| B.264 | CH₂CH₂-OCH₂CH₂CH₃ | |
| B.265 | CH₂CH₂-OCH(CH₃)₂ | |
| B.266 | CH₂CH₂-OCH₂CH₂CH₂CH₃ | |
| B.267 | CH₂CH₂-OCH (CH₃)CH₂CH₃ | |
| B.268 | CH₂CH₂-OCH₂CH(CH₃)₂ | |
| B.269 | CH₂CH₂-OC(CH₃)₃ | |
| B.270 | CH₂CH₂-OCF₃ | |
| B.271 | CH₂CH₂-OCH₂CF₃ | |
| B.272 | CH₂CH₂-[C₆H₅] | |
| B.273 | CH₂CH₂-[2-CN-C₆H₄] | |
| B.274 | CH₂CH₂-[3-CN-C₆H₄] | |
| B.275 | CH₂CH₂-[4-CN-C₆H₄] | |
| B.276 | CH₂CH₂-[2-F-C₆H₄] | |
| B.277 | CH₂CH₂-[3-F-C₆H₄] | |
| B.278 | CH₂CH₂-[4-F-C₆H₄] | |
| B.279 | CH₂CH₂-[2,4-F₂-C₆H₃] | |
| B.280 | CH₂CH₂-[3,4-F₂-C₆H₃] | |
| B.281 | CH₂CH₂-[2,4,5-F₃-C₆H₂] | |
| B.282 | CH₂CH₂-[2,4,6-F₃-C₆H₂] | |
| B.283 | CH₂CH₂-[2-Cl-C₆H₄] | |
| B.284 | CH₂CH₂-[3-Cl-C₆H₄] | |
| B.285 | CH₂CH₂-[4-Cl-C₆H₄] | |
| B.286 | CH₂CH₂-[2,4-Cl₂-C₆H₃] | |
| B.287 | CH₂CH₂-[3,4-Cl₂-C₆H₃] | |
| B.288 | CH₂CH₂-[2,4,5-Cl₃-C₆H₂] | |
| B.289 | CH₂CH₂-[2,4,6-Cl₃-C₆H₂] | |
| B.290 | CH₂CH₂-[2-CH₃-C₆H₄] | |
| B.291 | CH₂CH₂-[3-CH₃-C₆H₄] | |
| B.292 | CH₂CH₂- [4-CH₃-C₆H₄] | |
| B.293 | CH₂CH₂-[2-F, 4-CH₃-C₆H₃] | |
| B. 294 | CH₂CH₂-[2-CH₃, 4-F-C₆H₃] | |
| B.295 | CH₂CH₂-[3-F, 4-CH₃-C₆H₃] | |
| B.296 | CH₂CH₂- [3-CH₃, 4-F-C₆H₃] | |
| B.297 | CH₂CH₂-[2-Cl, 4-CH₃-C₆H₃] | |
| B.298 | CH₂CH₂-[2-CH₃, 4-Cl-C₆H₃] | |
| B.299 | CH₂CH₂-[3-Cl, 4-CH₃-C₆H₃] | |
| B.300 | CH₂CH₂-[3-CH₃, 4-Cl-C₆H₃] | |
| B.301 | CH₂CH₂-[2-CF₃-C₆H₄] | |
| B.302 | CH₂CH₂- [3-CF₃-C₆H₄] | |
| B.303 | CH₂CH₂-[4-CF₃-C₆H₄] | |
| B.304 | CH₂CH₂- [2-OCH₃-C₆H₄] | |
| B.305 | CH₂CH₂-[3-OCH₃-C₆H₄] | |
| B.306 | CH₂CH₂-[4-OCH₃-C₆H₄] | |
| B.307 | CH₂CH₂-[2-OCHF₂-C₆H₄] | |
| B.308 | CH₂CH₂- [3-OCHF₂-C₆H₄] | |
| B.309 | CH₂CH₂- [4-OCHF₂-C₆H₄] | |
| B.310 | CH(CH₃)-CN | |
| B.311 | CH(CH₃)-OCH₃ | |
| B.312 | CH(CH₃)-OCH₂CH₃ | |
| B.313 | CH(CH₃)-OCH₂CH₂CH₃ | |
| B.314 | CH(CH₃)-OCH(CH₃)₂ | |
| B.315 | CH(CH₃)-OCH₂CH₂CH₂CH₃ | |
| B.316 | CH(CH₃)-OCH(CH₃)CH₂CH₃ | |
| B.317 | CH(CH₃)-OCH₂CH(CH₃)₂ | |
| B.318 | CH(CH₃)-OC(CH₃)₃ | |
| B.319 | CH(CH₃)-OCF₃ | |
| B.320 | CH(CH₃)-OCH₂CF₃ | |
| B.321 | CH₂CH₂F | |
| B.322 | CH₂CHF₂ | |
| B.323 | CH₂CF₃ | |
| B.324 | CHFCH₃ | |
| B.325 | CF₂CH₃ | |
| B.326 | CHFCHF₂ | |
| B.327 | CHFCF₃ | |
| B.328 | CF₂CHF₂ | |
| B.329 | CF₂CF₃ | |
| B.330 | CF₂CHFCl | |
| B.331 | CH₂CH₂CH₂-CN | |
| B.332 | CH₂CH₂CH₂-OCH₃ | |
| B.333 | CH₂CH₂CH₂-OCH₂CH₃ | |
| B.334 | CH₂CH₂CH₂-OCH₂CH₂CH₃ | |
| B.335 | CH₂CH₂CH₂-OCH(CH₃)₂ | |
| B.336 | CH₂CH₂CH₂-OCF₃ | |
| B.337 | CH₂CH₂CH₂-OCH₂CF₃ | |
| B.338 | CH₂CH(CH₃)-CN | |
| B.339 | CH₂CH(CH₃)-OCH₃ | |
| B.340 | CH₂CH(CH₃)-OCH₂CH₃ | |
| B.341 | CH₂CH(CH₃)-OCH₂CH₂CH₃ | |
| B.342 | CH₂CH(CH₃)-OCH(CH₃)₂ | |
| B.343 | CH₂CH(CH₃)-OCF₃ | |
| B.344 | CH₂CH(CH₃)-OCH₂CF₃ | |
| B.345 | CH(CH₂CH₃)-CN | |
| B.346 | CH(CH₂CH₃)-OCH₃ | |
| B.347 | CH(CH₂CH₃)-OCH₂CH₃ | |
| B.348 | CH(CH₂CH₃)-OCH₂CH₂CH₃ | |
| B.349 | CH(CH₂CH₃)-OCH(CH₃)₂ | |
| B.350 | CH(CH₂CH₃)-OCF₃ | |
| B.351 | CH(CH₂CH₃)-OCH₂CF₃ | |
| B.352 | CH(CH₃)CH₂-CN | |
| B.353 | CH(CH₃)CH₂-OCH₃ | |
| B.354 | CH(CH₃)CH₂-OCH₂CH₃ | |
| B.355 | CH(CH₃)CH₂-OCH₂CH₂CH₃ | |
| B.356 | CH(CH₃)CH₂-OCH(CH₃)₂ | |
| B.357 | CH(CH₃)CH₂-OCF₃ | |
| B.358 | CH(CH₃)CH₂-OCH₂CF₃ | |
| B.359 | CH₂CH₂CF₃ | |
| B.360 | CH₂CF₃CF₃ | |
| B.361 | CHFCH₂CH₃ | |
| B.362 | CH(CF₃)CH₃ | |
| B.363 | CH(CF₃)₂ | |
| B.364 | CH₂CH₂CH₂CH₂-CN | |
| B.365 | CH₂CH₂CH₂CH₂-OCH₃ | |
| B.366 | CH₂CH₂CH₂CH₂-OCH₂CH₃ | |
| B.367 | CH₂CH₂CH₂CH₂-OCF₃ | |
| B.368 | CH(CN)-CH₂CH₂CH₃ | |
| B.369 | CH(OCH₃)-CH₂CH₂CH₃ | |
| B.370 | CH(OCH₂CH₃)-CH₂CH₂CH₃ | |
| B.371 | CH(OCF₃)-CH₂CH₂CH₃ | |
| B.372 | CH(OCH₂CF₃)-CH₂CH₂CH₃ | |
| B.373 | CH(CN)-CH₂CH(CH₃)₂ | |
| B.374 | CH(OCH₃)-CH₂CH(CH₃)₂ | |
| B.375 | CH(OCH₂CH₃)-CH₂CH(CH₃)₂ | |
| B.376 | CH(OCF₃)-CH₂CH(CH₃)₂ | |
| B.377 | CH(OCH₂CF₃) -CH₂CH(CH₃)₂ | |
| B.378 | CHFCH₂CH₂CH₃ | |
| B.379 | C(CH₃)=CH₂ | |
| B.380 | CH=CH-CH₃ | (Z) |
| B.381 | C(CH₂CH₃)=CH₂ | |
| B.382 | C(CH₃)=CH-CH₃ | (E) |
| B.383 | C(CH₃)=CH-CH₃ | (Z) |
| B.384 | C(CH₃)=C(CH₃)₂ | |
| B.385 | CH(CH₃)-CH=CH₂ | |
| B.386 | CH=C(CH₃)₂ | |
| B.387 | CH₂-C(CH₃)=CH₂ | |
| B.388 | CH (CH₃)-CH₂-CH=CH₂ | |
| B.389 | CH₂-CH(CH₃)-CH=CH₂ | |
| B.390 | CH₂-CCl=CH₂ | |
| B.391 | CH₂-CH=CH-Cl | (Z) |
| B.392 | CH₂-CCl=CH-Cl | (E) |
| B.393 | CH₂-CCl=CH-Cl | (Z) |
| B.394 | CH₂-CH=CCl₂ | |
| B.395 | CH₂-CCl=CCl₂ | |
| B.396 | CH₂-CBr=CH₂ | |
| B.397 | CH₂-CH=CH-Br | (Z) |
| B.398 | CH₂-CBr=CH-Br | (E) |
| B.399 | CH₂-CBr=CH-Br | (Z) |
| B.400 | CH₂-CH=CBr₂ | |
| B.401 | CH₂-CBr=CBr₂ | |
| B.402 | CH₂-CH=CH-CH₃ | (Z) |
| B.403 | CH₂-C(CH₃)=CH-CH₃ | (E) |
| B.404 | CH₂-C(CH₃)=CH-CH₃ | (Z) |
| B.405 | CH₂-CH=C(CH₃)₂ | |
| B.406 | CH₂-CH₂-CH=CH₂ | |
| B.407 | CH₂-CCl=CH-CH₃ | (E) |
| B.408 | CH₂-CCl=CH-CH₃ | (Z) |
| B.409 | CH₂-CH=CCl-CH₃ | (E) |
| B.410 | CH₂-CH=CCl-CH₃ | (Z) |
| B.411 | CH₂-C(CH₃)=C(CH₃)₂ | |
| B.412 | CH₂-CBr=CH-CH₃ | (E) |
| B.413 | CH₂-CBr=CH-CH₃ | (Z) |
| B.414 | CH₂-CH=CBr-CH₃ | (E) |
| B.415 | CH₂-CH=CBr-CH₃ | (Z) |
| B.416 | CH₂-CH=CH-CH₂Cl | (E) |
| B.417 | CH₂-CH=CH-CH₂Cl | (Z) |
| B.418 | CH₂-CH=CH-CH₂CH₃ | (E) |
| B.419 | CH₂-CH=CH-CH₂CH₃ | (Z) |
| B.420 | CH₂-CH=CH-CH₂Br | (E) |
| B.421 | CH₂-CH=CH-CH₂Br | (Z) |
| B.422 | CH₂-CCl=CCl-CH₂Cl | (E) |
| B.423 | CH₂-CCl=CCl-CH₂Cl | (Z) |
| B.424 | CH₂-CF=CH₂ | |
| B.425 | CH₂-CH=CH-F | (E) |
| B.426 | CH₂-CH=CH-F | (Z) |
| B.427 | CH₂-CH=CF₂ | |
| B.428 | CH₂-CF=CH-F | (E) |
| B.429 | CH₂-CF=CH-F | (Z) |
| B.430 | CH(CH₃)-CH=CH₂ | |
| B.431 | CH(CH₃)-CCl=CH₂ | |
| B.432 | CH(CH₃)-CH=CH-Cl | (E) |
| B.433 | CH(CH₃)-CH=CH-Cl | (Z) |
| B.434 | CH(CH₃)-CCl=CH-Cl | (E) |
| B.435 | CH(CH₃)-CCl=CH-Cl | (Z) |
| B.436 | CH(CH₃)-CH=CCl₂ | |
| B.437 | CH(CH₃)-CCl=CCl₂ | |
| B.438 | CH(CH₃)-CBr=CH₂ | |
| B.493 | CH(CH₃)-CH=CH-Br | (E) |
| B.440 | CH(CH₃)-CH=CH-Br | (Z) |
| B.441 | CH(CH₃)-CBr=CH-Br | (E) |
| B.442 | CH(CH₃)-CBr=CH-Br | (Z) |
| B.443 | CH(CH₃)-CH=CBr₂ | |
| B.444 | CH(CH₃)-CBr=CBr₂ | |
| B.445 | CH(CH₃)-C(CH₃)=CH₂ | |
| B.446 | CH(CH₃)-C(CH₃)=CH₂ | (E) |
| B.447 | CH(CH₃)-CH=CH-CH₃ | (Z) |
| B.448 | CH(CH₃)-C(CH₃)=CH-CH₃ | (E) |
| B.449 | CH(CH₃)-C(CH₃)=CH-CH₃ | (Z) |
| B.450 | CH(CH₃)-CH=C(CH₃)₂ | |
| B.451 | CH(CH₃)-CCl=CH-CH₃ | (E) |
| B.452 | CH(CH₃)-CCl=CH-CH₃ | (Z) |
| B.453 | CH(CH₃)-CH=CCl-CH₃ | (E) |
| B.454 | CH(CH₃)-CH=CCl-CH₃ | (Z) |
| B.455 | CH(CH₃)-CBr=CH-CH₃ | (E) |
| B.456 | CH(CH₃)-CBr=CH-CH₃ | (Z) |
| B.457 | CH(CH₃)-CH=CBr-CH₃ | (E) |
| B.458 | CH(CH₃)-CH=CBr-CH₃ | (Z) |
| B.459 | CH(CH₃)-CH=CH-CH₂Cl | (E) |
| B.460 | CH(CH₃)-CH=CH-CH₂Cl | (Z) |
| B.461 | CH(CH₃)-CH=CH-CH₂CH₃ | (E) |
| B.462 | CH(CH₃)-CH=CH-CH₂CH₃ | (Z) |
| B.463 | CH(CH₃)-CH=CH-CH₂Br | (E) |
| B.464 | CH(CH₃)-CH=CH-CH₂Br | (Z) |
| B.465 | CH(CH₃)-CCl=CCl-CH₂Cl | (E) |
| B.466 | CH(CH₃)-CCl=CCl-CH₂Cl | (Z) |
| B.467 | CH(CH₃)-CF=CH₂ | |
| B.468 | CH(CH₃)-CH=CH-F | (E) |
| B.469 | CH(CH₃)-CH=CH-F | (Z) |
| B.470 | CH(CH₃)-CH=CF₂ | |
| B.471 | CH(CH₃)-CF=CH-F | (E) |
| B.472 | CH(CH₃)-CF=CH-F | (Z) |
| B.473 | CH₂CHCl-CH=CH₂ | |
| B.474 | CH₂CH₂-CH=C(CH₃)₂ | |
| B.475 | CH₂CH₂-C(CH₃)=CH-CH₃ | (E) |
| B.476 | CH₂CH₂-C(CH₃)=CH-CH₃ | (Z) |
| B.477 | C(CH₃)=CH-[C₅H₅] | (E) |
| B.478 | C(CH₃)=CH-[2-CN-C₆H₄] | (E) |
| B.479 | C(CH₃)=CH-[3-CN-C₆H₄] | (E) |
| B.480 | C(CH₃)=CH-[4-CN-C₆H₄] | (E) |
| B.481 | C(CH₃)=CH-[2-F-C₆H₄] | (E) |
| B.482 | C(CH₃)=CH-[3-F-C₆H₄] | (E) |
| B.483 | C(CH₃)=CH-[4-F-C₆H₄] | (E) |
| B.484 | C(CH₃)=CH-[2,4-F₂-C₆H₃] | (E) |
| B.485 | C(CH₃)=CH-[3,4-F₂-C₆H₃] | (E) |
| B.486 | C(CH₃)=CH-[2,4,5-F₃-C₆H₂] | (E) |
| B.487 | C(CH₃)=CH-[2,4,6-F₃-C₆H₂] | (E) |
| B.488 | C(CH₃)=CH-[2-Cl-C₆H₄] | (E) |
| B.489 | C(CH₃)=CH-[3-Cl-C₆H₄] | (E) |
| B.490 | C(CH₃)=CH-[4-Cl-C₆H₄] | (E) |
| B.491 | C(CH₃)=CH-[2,4-Cl₂-C₆H₃] | (E) |
| B.492 | C(CH₃)=CH-[3,4-Cl₂-C₆H₃] | (E) |
| B.493 | C(CH₃)=CH-[2,4,5-Cl₃-C₆H₂] | (E) |
| B.494 | C(CH₃)=CH-[2,4,6-Cl₃-C₆H₂] | (E) |
| B.495 | C(CH₃)=CH-[2-CH₃-C₆H₄] | (E) |
| B.496 | C(CH₃)=CH-[3-CH₃-C₆H₄] | (E) |
| B.497 | C(CH₃)=CH-[4-CH₃-C₆H₄] | (E) |
| B.498 | C(CH₃)=CH-[2-F, 4-CH₃-C₆H₃] | (E) |
| B.499 | C(CH₃)=CH-[2-CH₃, 4-F-C₆H₃] | (E) |
| B.500 | C(CH₃)=CH-[3-F, 4-CH₃-C₆H₃] | (E) |
| B.501 | C(CH₃)=CH-[3-CH₃, 4-F-C₆H₃] | (E) |
| B.502 | C(CH₃)=CH-[2-Cl, 4-CH₃-C₆H₃] | (E) |
| B.503 | C(CH₃)=CH-[2-CH₃, 4-Cl-C₆H₃] | (E) |
| B.504 | C(CH₃)=CH-[3-Cl, 4-CH₃-C₆H₃] | (E) |
| B.505 | C(CH₃)=CH-[3-CH₃, 4-Cl-C₆H₃] | (E) |
| B.506 | C(CH₃)=CH-[2-CF₃-C₆H₄] | (E) |
| B.507 | C(CH₃)=CH-[3-CF₃-C₆H₄] | (E) |
| B.508 | C(CH₃)=CH-[4-CF₃-C₆H₄] | (E) |
| B.509 | C(CH₃)=CH-[2-OCH₃-C₆H₄] | (E) |
| B.510 | C(CH₃)=CH-[3-OCH₃-C₆H₄] | (E) |
| B.511 | C(CH₃)=CH-[4-OCH₃-C₆H₄] | (E) |
| B.512 | C(CH₃)=CH-[2-OCHF₂-C₆H₄] | (E) |
| B.513 | C(CH₃)=CH-[3-OCHF₂-C₆H₄] | (E) |
| B.514 | C(CH₃)=CH-[4-OCHF₂-C₆H₄] | (E) |
| B.515 | CH=C(CH₃)-[C₆H₅] | (E) |
| B.516 | CH=C(CH₃)-[2-CN-C₆H₄] | (E) |
| B.517 | CH=C(CH₃)-[3-CN-C₆H₄] | (E) |
| B.518 | CH=C(CH₃)-[4-CN-C₆H₄] | (E) |
| B.519 | CH=C(CH₃)-[2-F-C₆H₄] | (E) |
| B.520 | CH=C(CH₃)-[3-F-C₆H₄] | (E) |
| B.521 | CH=C(CH₃)-[4-F-C₆H₄] | (E) |
| B.522 | CH=C(CH₃)-[2,4-F₂-C₆H₃] | (E) |
| B.523 | CH=C(CH₃)-[3,4-F₂-C₆H₃] | (E) |
| B.524 | CH=C(CH₃)-[2,4,5-F₃-C₆H₂] | (E) |
| B.525 | CH=C(CH₃)-[2,4,6-F₃-C₆H₂] | (E) |
| B.526 | CH=C(CH₃)-[2-Cl-C₆H₄] | (E) |
| B.527 | CH=C(CH₃)-[3-Cl-C₆H₄] | (E) |
| B.528 | CH=C(CH₃)-[4-Cl-C₆H₄] | (E) |
| B.529 | CH=C(CH₃)-[2,4-Cl₂-C₆H₃] | (E) |
| B.530 | CH=C(CH₃)-[3,4-Cl₂-C₆H₃] | (E) |
| B.531 | CH=C(CH₃)-[2,4,5-Cl₃-C₆H₂] | (E) |
| B.532 | CH=C(CH₃)-[2,4,6-Cl₃-C₆H₂] | (E) |
| B.533 | CH=C(CH₃)-[2-CH₃-C₆H₄] | (E) |
| B.534 | CH=C(CH₃)-[3-CH₃-C₆H₄] | (E) |
| B.535 | CH=C(CH₃)-[4-CH₃-C₆H₄] | (E) |
| B.536 | CH=C(CH₃)-[2-F, 4-CH₃-C₆H₃] | (E) |
| B.537 | CH=C(CH₃)-[2-CH₃, 4-F-C₆H₃] | (E) |
| B.538 | CH=C(CH₃)-[3-F, 4-CH₃-C₆H₃] | (E) |
| B.539 | CH=C(CH₃)-[3-CH₃, 4-F-C₆H₃] | (E) |
| B.540 | CH=C(CH₃)-[2-Cl, 4-CH₃-C₆H₃] | (E) |
| B.541 | CH=C(CH₃)-[2-CH₃, 4-Cl-C₆H₃] | (E) |
| B.542 | CH=C(CH₃)-[3-Cl, 4-CH₃-C₆H₃] | (E) |
| B.543 | CH=C(CH₃)-[3-CH₃, 4-Cl-C₆H₃] | (E) |
| B.544 | CH=C(CH₃)-[2-CF₃-C₆H₄] | (E) |
| B.545 | CH=C(CH₃)-[3-CF₃-C₆H₄] | (E) |
| B.546 | CH=C(CH₃)-[4-CF₃-C₆H₄] | (E) |
| B.547 | CH=C(CH₃)-[2-OCH₃-C₆H₄] | (E) |
| B.548 | CH=C(CH₃)-[3-OCH₃-C₆H₄] | (E) |
| B.549 | CH=C(CH₃)-[4-OCH₃-C₆H₄] | (E) |
| B.550 | CH=C(CH₃)-[2-OCHF₂-C₆H₄] | (E) |
| B.551 | CH=C(CH₃)-[3-OCHF₂-C₆H₄] | (E) |
| B.552 | CH=C(CH₃)-[4-OCHF₂-C₆H₄] | (E) |
| B.553 | C≡C-I | |
| B.554 | CH₂-C≡C-H | |
| B.555 | CH₂-C≡C-Cl | |
| B.556 | CH₂-C≡C-Br | |
| B.557 | CH₂-C≡C-J | |
| B.558 | CH₂-C≡C-CH₃ | |
| B.559 | CH₂-C≡C-CH₂CH₃ | |
| B.560 | CH₂CH₂-C≡C-H | |
| B.561 | CH₂CH₂-C≡C-Cl | |
| B.562 | CH₂CH₂-C≡C-Br | |
| B.563 | CH₂CH₂-C≡C-J | |
| B.564 | CH₂CH₂-C≡C-CH₃ | |
| B.565 | CH₂CH₂CH₂-C≡C-H | |
| B.566 | CH₂CH₂CH₂-C≡C-Cl | |
| B.567 | CH₂CH₂CH₂-C≡C-Br | |
| B.568 | CH₂CH₂CH₂-C≡C-J | |
| B.569 | CH₂CH₂CH₂-C≡C-CH₃ | |
| B.570 | CH(CH₃)-C≡C-H | |
| B.571 | CH(CH₃)-C≡C-Cl | |
| B.572 | CH(CH₃)-C≡C-Br | |
| B.573 | CH(CH₃)-C≡C-J | |
| B.574 | CH(CH₃)-C≡C-CH₃ | |
| B.575 | C≡C-[4-F-C₆H₄] | |

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der Ascomyceten, Deuteromyceten, Phycomyceten und Basidiomycecen, aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- Alternaria-Arten an Gemüse und Obst,
- Botrytis cinerea (Grauschimmel) an Erdbeeren, Gemüse, Zierpflanzen und Reben,
- Cercospora arachidicola an Erdnüssen,
- Erysiphe cichoracearum und Sphaerotheca fuliginea an Kürbisgewächsen,
- Erysiphe graminis (echter Mehltau) an Getreide,
- Fusarium- und Verticillium-Arten an verschiedenen Pflanzen,
- Helminthosporium-Arten an Getreide,
- Mycosphaerella-Arten an Bananen,
- Phytophthora infestans an Kartoffeln und Tomaten,
- Plasmopara viticola an Reben,
- Podosphaera leucotricha an Äpfeln,
- Pseudocercosporella herpotrichoides an Weizen und Gerste,
- Pseudocercosporella-Arten an Hopfen und Gurken,
- Puccinia-Arten an Getreide,
- Pyricularia oryzae an Reis,
- Rhizoctonia-Arten an Baumwolle, Reis und Rasen,
- Septoria nodorum an Weizen,
- Uncinula necator an Reben,
- Ustilago-Arten an Getreide und Zuckerrohr, sowie
- Venturia inaequalis (Schorf) an Äpfeln.

Die Verbindungen I eignen sich außerdem zur Bekämpfung von Schadpilzen im Materialschutz (z.B. Holz, Papier, Dispersionen für den Ansprich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen der Formel I sind außerdem geeignet, tierische Schädlinge aus der Klasse der Insekten, Spinnentiere und Nematoden wirksam zu bekämpfen. Sie können im Pflanzenschutz sowie auf dem Hygiene-, vorratsschutz- und Veterinärsektor zur Bekämpfung tierischer Schädlinge eingesetzt werden. Insbesondere eignen sie sich zur Bekämpfung der folgenden tierischen Schädlinge:
- Insekten aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni und Zeiraphera canadensis,
- Käfer (Coleoptera), z.B. Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus und Sitophilus granaria,
- Zweiflügler (Diptera), z.B. Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea und Tipula paludosa,
- Thripse (Thysanoptera), z.B. Frankliniella fusca, Frankliniella occidentalis, Frankliniella tritici, Scirtothrips citri, Thrips oryzae, Thrips palmi und Thrips tabaci,
- Hautflügler (Hymenoptera), z.B. Athalia rosae, Atta cephalotes, Atta sexdens, Atta texana, Hoplocampa minuta, Hoplocampa testudinea, Monomorium pharaonis, Solenopsis geminata und Solenopsis invicta,
- Wanzen (Heteroptera), z.B. Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis und Thyanta perditor,
- Pflanzensauger (Homoptera), z.B. Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum und Viteus vitifolii.
- Termiten (Isoptera), z.B. Calotermes flavicollis, Leucotermes flavipes, Reticulitermes lucifugus und Termes natalensis,
- Geradflügler (Orthoptera), z.B. Acheta domestica, Blatta orientalis. Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus und Tachycines asynamorus,
- Arachnoidea wie Spinnentiere (Acarina), z.B. Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius und Tetranychus urticae,
- Nematoden wie Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus und Pratylenchus goodeyi.

Die Aufwandmenge an Wirkstoff zur Bekämpfung von tierischen Schädlingen beträgt unter Freilandbedingungen 0,1 bis 2,0, vorzugsweise 0,2 bis 1,0 kg/ha.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, Z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt.
   Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalinalpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält maneine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Hilfsstoffe und Mittel können zu den erfindungsgemäßen Wirkstoffen im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfid, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, 0,0-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
- N-Dichlorfluormethylthio-N', N'-dimethyl-N-phenyl-schwefelsäure- diamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-ylharnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis-(pchlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[α-(o-tolyloxy)-o-tolyl]acetat, Methyl-E-2-{2-[6-(2-cyanophenoxy)-pyrimidin-4-yloxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl)-acetamid,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid,
- sowie verschiedene Fungizide, wie Dodecylguanidinacetat, 3-[3-(3,5-Dimethyl-2-oxycyclohexyl)-2-hydroxyethyl)-glutarimid, Hexachlorbenzol, DL-Methyl-N-(2,6-dimethyl-phenyl)-N-furoyl(2)-alaninat, DL-N-(2, 6-Dimethyl-phenyl)-N-(2'-methoxyacetyl)-alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl-(5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano- [N- (ethylaminocarbonyl) -2-methoximino] -acetamid, 1- [2(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1: Herstellung von

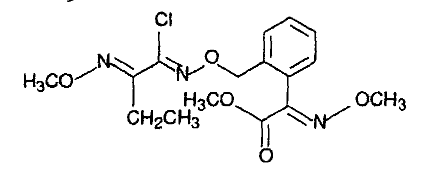

### Stufe 1:

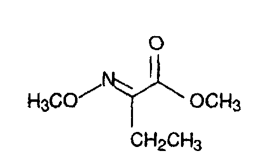

Eine Lösung von 50g (0,49 mol) 2-Oxobutansäure in 300 ml Methanol wurde portionsweise mit 123g (1,47 mol) Methoxyaminhydrochlorid versetzt. Nach 16 h Rühren bei Raumtemperatur (ca. 25°C) wurde die Reaktionsmischung bei vermindertem Druck vom Lösungsmittel befreit. Der so erhaltene Rückstand wurde in Wasser und tert.-Butylmethylether aufgenommen. Die Ether-Phase wurde mit gesättigter NaHCO₃-Lösung und mit Wasser gewaschen, über Na₂SO₄ getrocknet und anschließend bei vermindertem Druck eingeengt. Man erhielt so 47 g der Titelverbindung als farblose Flüssigkeit.
¹H-NMR (CDCl₃; δ in ppm): 1,06 (t, 3H); 2,57 (q, 2H); 3,85 (s, 3H); 4,04 (s, 3H)

### Stufe 2:

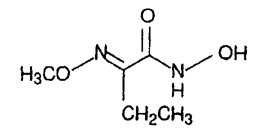

Eine Lösung aus 33 g (0,48 mol) Hydroxylamin-hydrochlorid und 400 ml Methanol wurde bei 10°C tropfenweise mit 46 g (0,32 mol) der Verbindung aus Beispiel 1, Stufe 1, versetzt. Zu der so erhaltenen Mischung wurden anschließend unter Eiskühlung innerhalb von 15 min. 171 g Natriummethylat-Lösung (30%-ig in Methanol, 0,96 mol) gegeben. Die Reaktionsmischung wurde dann für 60h bei Raumtemperatur (ca. 25°C) gerührt, wobei sich eine Suspension bildete. Die Suspension wurde in Wasser gegeben; die so erhaltene Mischung wurde unter Eiskühlung mit konz. Salzsäure bis zu einem pH Wert von 6,5 angesäuert und anschließend mit Methylenchlorid mehrere Male extrahiert. Die organischen Phasen wurden vereinigt, mit Wasser gewaschen, über Na₂SO₄ getrocknet und bei vermindertem Druck und 30°C vom Lösungsmittel befreit. Man erhielt 34,1 g der Titelverbindung als weißes Pulver; Fp.: 56-59°C.
- ACHTUNG:: *Hydroxamsäuren vom Typ der Verbindung gemäß Beispiel 1*., *Stufe 2,* können *thermolabil sein und sich bei höheren Temperaturen explosionsartig zersetzen!*
¹H-NMR (CDCl₃; δ in ppm): 1,03 (t, 3H); 2,53 (q, 2H); 3,93 (s, 3H); 8,95 (s, br, 2H)

### Stufe 3:

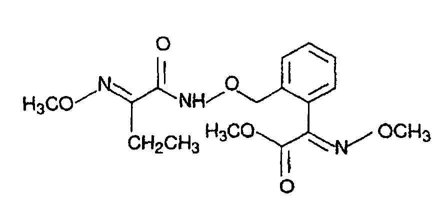

Eine Lösung aus 28,5 g (0,195 mol) der Verbindung gemäß Beispiel 1, Stufe 2, 55,8 g (0,195 mol) E-2-Methoxyimino-2-[2'-brommethyl-phenyl]-essigsäure-methylester (gem. EP-A 400 417) und 600 ml N,N-Dimethylformamid wurde unter Eiskühlung tropfenweise mit 38,6 g Natriummethylat-Lösung (30%-ig in Methanol; 0,215 mol) versetzt. Nach 16h Rühren bei Raumtemperatur (ca. 25°C) wurde das Reaktionsgemisch in gekühlte verdünnte Salzsäure eingerührt und die so erhaltene Mischung wurde mit tert.-Butylmethylether extrahiert. Die organische Phase wurde mit Wasser gewaschen, über Na₂SO₄ getrocknet und bei vermindertem Druck und ca. 35°C vom Lösungsmittel befreit. Der erhaltene Rückstand wurde chromatographisch (Kieselgel; Eluent: tert.-Butylmethylether/Cyclohexan) gereinigt. Nach Waschen des isolierten Feststoffs mit Pentan erhielt man 50 g der Titelverbindung als hellgelbes Pulver; Fp.: 63-65°C.
¹H-NMR (CDCl₃; δ in ppm): 1,03 (t, 3H); 2,51 (q, 2H); 3,87 (s, 6H); 4,03 (s, 3H); 4,86 (s, 2H); 7,14-7,45 (m, 4H); 8,92 (s, 1H)

### Stufe 4:

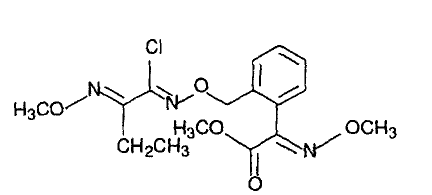

Eine Mischung aus 6,0 g (17,1 mmol) der Verbindung aus Beispiel 1, Stufe 3, 22,4 g (85,5 mmol) Triphenylphosphin und 250 ml Acetonitril wurde portionsweise mit 13,2 g (85,5 mmol) Tetrachlormethan versetzt. Die so erhaltene Reaktionsmischung wurde 60 h unter Rückfluß gekocht und anschließend auf Raumtemperatur (ca. 25°C) abgekühlt. Das Lösungsmittel wurde bei vermindertem Druck abdestilliert und der verbleibende Rückstand wurde chromatographisch (Kieselgel, tert.-Butylmethylether/Cyclohexan) gereinigt. Man erhielt 2,3 g der Titelverbindung als beige-farbenes Pulver; Fp.: 56-59°C.
¹H-NMR (CDCl₃; δ in ppm): 0,97 (t, 3H); 2,55 (q, 2H); 3,85 (s/ 3H); 3,99 (s, 3H); 4,04 (s, 3H); 5,13 (s, 2H); 7,15-7,50 (m, 4H)

### Beispiel 2: Herstellung von

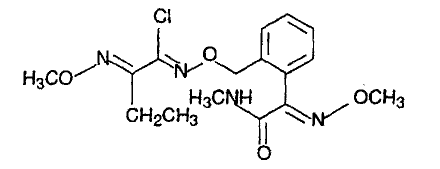

Eine Lösung aus 15,5 g (42 mmol) der Verbindung aus Beispiel 1, Sufe 4, und 100 ml Tetrahydrofuran wurde mit 33 g Methylamin-Lösung (40%-ig in Wasser) versetzt. Nach 2 h bei Raumtemperatur (ca. 25°C) wurde die Reaktionsmischung in gekühlte verdünnte Saltzsäure gegeben. Die saure Lösung wurde mit tert.-Butylmethylether extrahiert. Anschließend wurde die organische Phase mit Wasser gewaschen, über Na₂SO₄ getrocknet und bei vermindertem Druck vom Lösungsmittel befreit. Nach Verreiben des Rückstands mit Hexan/tert.-Butylmethylether erhielt man 11,5 g der Titelverbindung als beige-farbenes Pulver; Fp.: 66-69°C.
¹H-NMR (CDCl₃; δ in ppm): 0,97 (t, 3H); 2,54 (q, 2H); 2,85 (d, 3H); 3,91 (s, 3H); 3,97 (s, 3H); 5,15 (s, 2H); 6,80 (s, br, 1H); 7,18-7,43 (m, 4H)

### Beispiel 3: Herstellung von

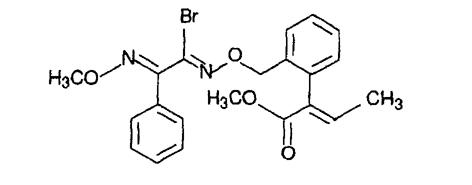

### Stufe 1:

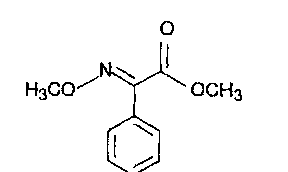

Eine Lösung aus 388 g (4,65 mol) Methoxyaminhydrochlorid und 1,5 l Methanol wurde zunächst mit 60 g Molekularsieb (3Å) und anschließend bei 60°C mit 254 g (1,55 mol) Phenylglyoxylsäure-methylester versetzt. Nach 6 h bei 60°C und weiteren 60 h bei Raumtemperatur (ca. 25°C) wurde die Reaktionsmischung mit Wasser versetzt. Die wäßrige Lösung wurde mit tert.-Butylmethylether extrahiert. Die Etherphase wurde mit Wasser gewaschen, über Na₂SO₄ getrocknet und bei vermindertem Druck vom Lösungsmittel befreit. Der Rückstand wurde chromatographisch (Kieselgel; tert.-Butylmethylether/Cyclohexan) gereinigt. Man erhielt so 66 g der Titelverbindung als hellgelbes Pulver.
¹H-NMR (CDCl₃; δ in ppm): 3,88 (s, 3H); 4,07 (s, 3H); 7,40 (s, 5H)

### Stufe 2:

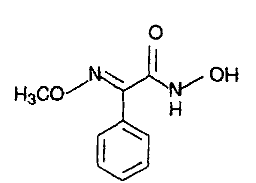

Eine Lösung aus 58,2 g (1,04 mol) Kaliumhydroxid in 750 ml Methanol wurde tropfenweise mit einer Lösung aus 36,1 g (0,52 mol) Hydroxylamin-hydrochlorid in 250 ml Methanol versetzt. Nach ca. 10 min. wurde der gebildete Niederschlag abfiltriert und die erhaltene Lösung wurde mit einer Lösung aus 100 g der Verbindung aus Beispiel 3, Stufe 1, in 500 ml Methanol versetzt. Nach 24 h bei Raumtemperatur (ca. 25°C) wurde das Reaktionsgemisch auf Wasser gegeben und mit tert.-Butylmethylether extrahiert. Die wäßrige Phase wurde unter Eiskühlung mit verdünnter Salzusäure bis zu einem pH von 6,5 angesäuert, wobei sich ein Niederschlag bildete. Der Niederschlag wurde isoliert und bei vermindertem Druck getrocknet. Man erhielt so 57 g der Titelverbindung als beige-farbenes Pulver; Fp.: 142-144°C.
¹H-NMR (d₆-DMSO; δ in ppm): 3,89 (s, 3H); 7,33-7,55 (m, 5H); 9,20 (s, br, 1H); 11,14 (s, br, 1H)

### Stufe 3:

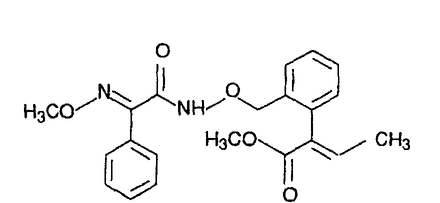

Eine Lösung aus 20 g (0,1 mol) der Verbindung gemäß Beispiel 3, Stufe 2, 27 g (0,1 mol) E-3-Methyl-2-[2'-brommethyl-phenyl]-acrylsäure-methylester (gem. EP-A 513 580) und 300 ml N,N-Dimethylformamid wurde unter Eiskühlung tropfenweise mit 18 g Natriummethylat-Lösung (30%-ig in Methanol; 0,1 mol) versetzt. Nach 12 h Rühren bei Raumtemperatur (ca. 25°C) wurde das Reaktionsgemisch in Eiswasser eingerührt und die so erhaltene Mischung wurde mit Methylenchlorid extrahiert. Die organische Phase wurde mit Wasser gewaschen, über Na₂SO₄ getrocknet und bei vermindertem Druck und ca. 35°C vom Lösungsmittel befreit. Der erhaltene Rückstand wurde aus tert.-Butylmethylether/Hexan/Isopropanol kristallisiert. Man erhielt so 23,8 g der Titelverbindung als farblose Kristalle; Fp.: 81-84°C.
¹H-NMR (CDCl₃; δ in ppm): 1,63 (d, 3H); 3,70 (s, 3H); 3,92 (s, 3H); 4,83-4,92 (m, 2H); 7,08-7,53 (m, 10H); 9,07 (s, br, 1H)

### Stufe 4:

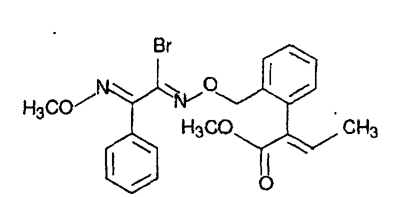

Eine Mischung aus 4,8 g (12,6 mmol) der Verbindung aus Beispiel 3, Stufe 3, 10 g (38 mmol) Triphenylphosphin und 100 ml Acetonitril wurde portionsweise mit 12,6 g (38 mmol) Tetrabrommethan versetzt. Die so erhaltene Reaktionsmischung wurde 100 h unter Rückfluß gekocht und anschließend auf Raumtemperatur (ca. 25°C) abgekühlt. Das Lösungsmittel wurde bei vermindertem Druck abdestilliert und der verbleibende Rückstand wurde chromatographisch (Kieselgel, tert.-Butylmethylether/Cyclohexan) gereinigt. Man erhielt 2,4 g der Titelverbindung als farbloses Pulver; Fp.: 60-61°C.
¹H-NMR (CDCl₃; δ in ppm): 1,54 (d, 3H); 3,66 (s, 3H); 3,99 (s, 3H); 5,02 (s, 2H); 7,01-7,38 (m, 10H)

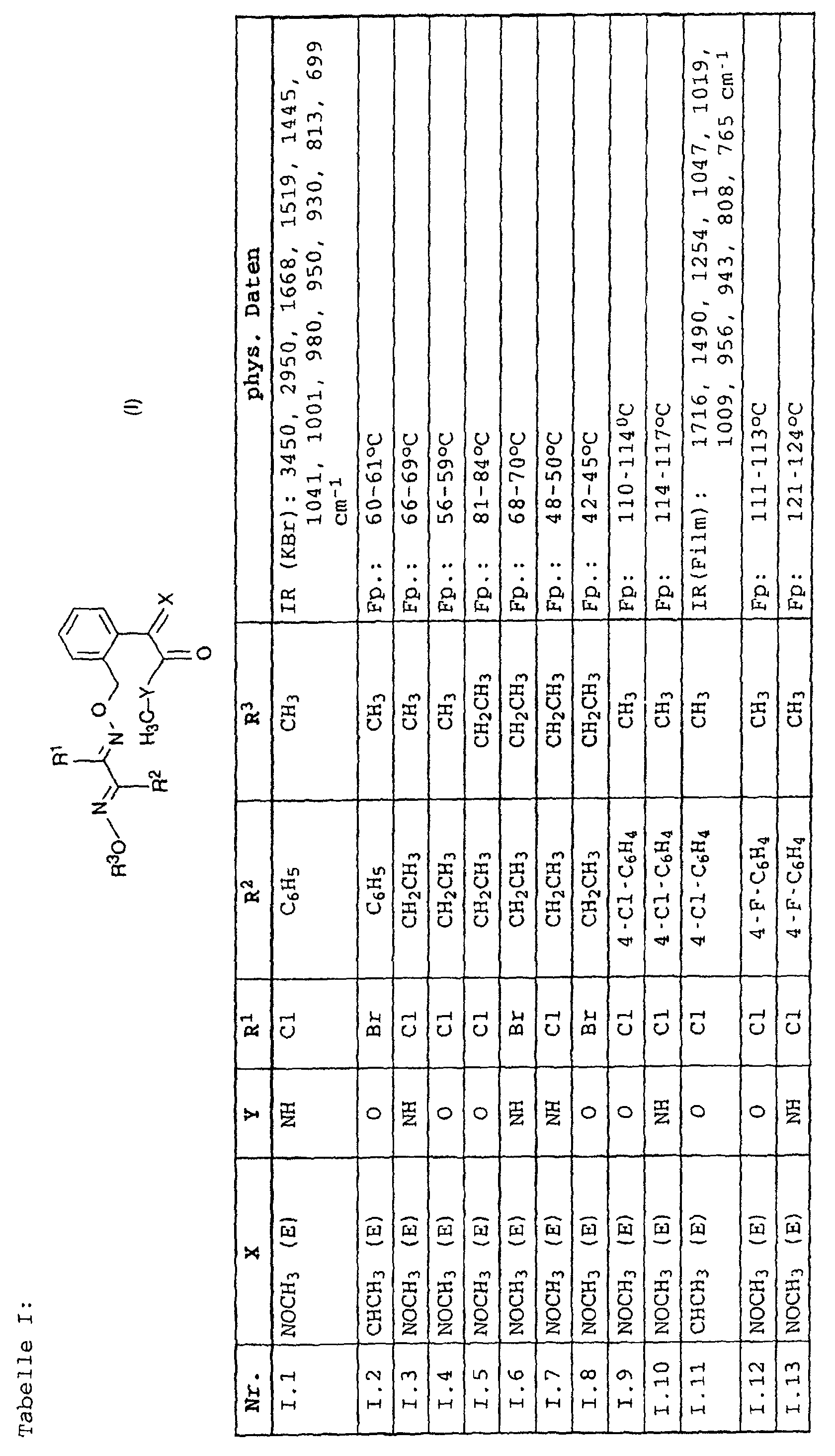

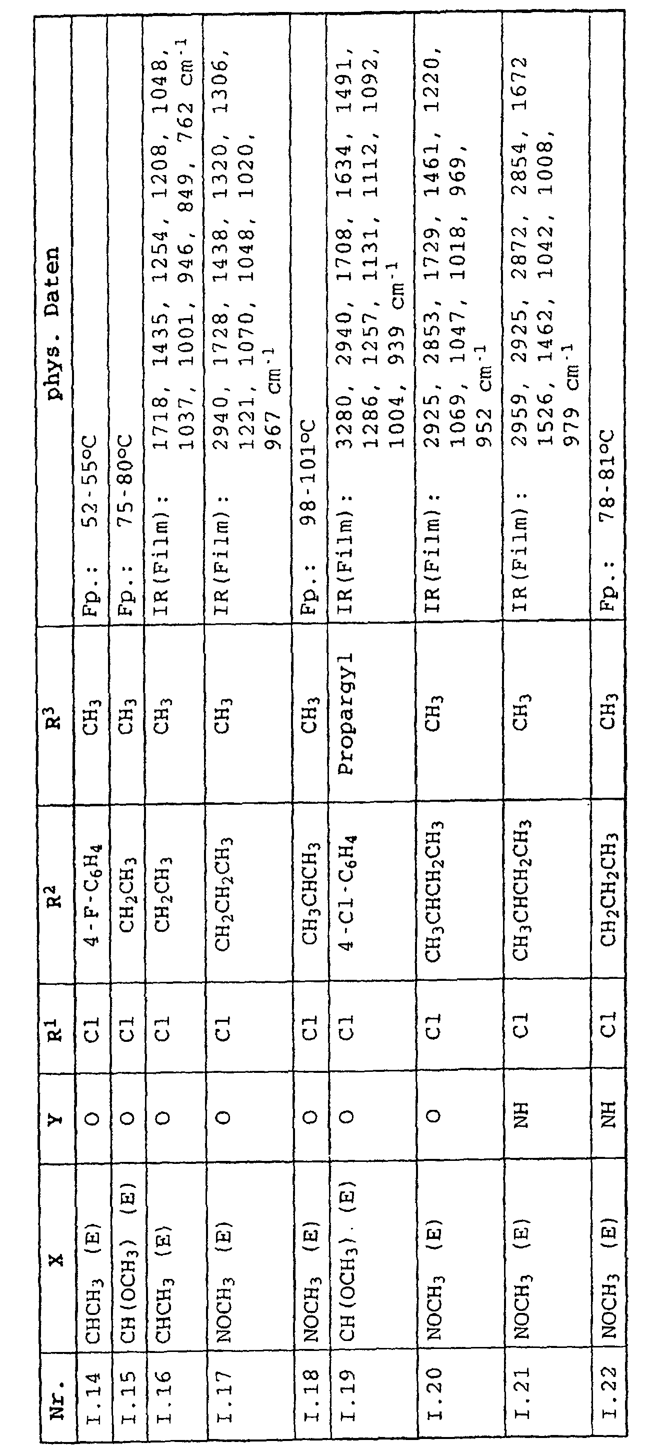

### Beispiele für die Wirkung gegen Schadpilze

Die verbesserte fungizide Wirkung der Verbindungen der allgemeinen Formel I ließ sich durch die folgenden Versuche zeigen. Als Vergleichsverbindungen des Standes der Technik gemäß WO-A 95/21,153 und WO-A 95/21,154 dienten die bekannten Wirkstoffe A.1 und A.2:

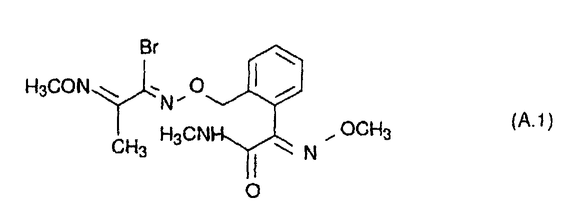

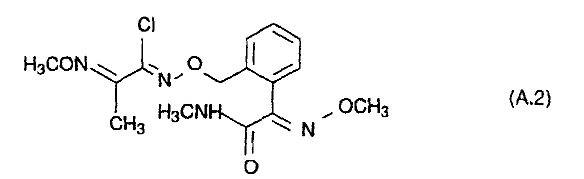

Die Wirkstoffe wurden getrennt als 10%ige Emulsion in einem Gemisch aus 63 Gew.-% Cyclohexanon, 27 Gew.-% Emulgator aufbereitet und entsprechend der gewünschten Konzentration mit Wasser verdünnt.

### Wirksamkeit gegen Puccinia recondita an Weizen (Weizenbraunrost)

Blätter von in Töpfen gewachsenen Weizensämlingen der Sorte "Frühgold" wurden mit Sporen des Braunrostes (*Puccinia recondita)* bestäubt. Danach wurden die Töpfe für 24 Stunden in eine Kammer mit hoher Luftfeuchtigkeit (90 bis 95.%) und 20 bis 22°C gestellt. Während dieser Zeit keimten die Sporen aus und die Keimschläuche drangen in das Blattgewebe ein. Die infizierten Pflanzen wurden am nächsten Tag mit einer wäßrigen Wirkstoffaufbereitung tropfnaß besprüht. Nach dem Antrocknen des Spritzbelages wurden die Versuchspflanzen im Gewächshaus bei Temperaturen zwischen 20 und 22°C und 65 bis 70 % relativer Luftfeuchte für 7 Tage kultiviert. Dann wurde das Ausmaß der Rostpilzentwicklung auf den Blättern ermittelt.

In diesem Test zeigten die mit 16 ppm der erfindungsgemäßen Verbidnungen I.3, I.6, I.7, I.10 bis I.17, I.21 und I.22 behandelten Pflanzen keinen Befall oder einen Befall von maximal 5% während die mit der gleichen Menge der Vergleichsverbindungen A.1 bzw. A.2 behandelten Pflanzen zu 15 bzw. 80% befallen waren. Die unbehandelten (Kontroll-) Pflanzen waren zu 80% befallen.

In einem analogen Test zeigten die mit 4 ppm der erfindungsgemäßen Verbidnungen I.3, I.6, I.7, I.10 bis I.17, I.21 und I.22 behandelten Pflanzen einen Befall von 5 bis 40%, während die mit der gleichen Menge der Vergleichsverbindungen A.1 bzw. A.2 be-handelten Pflanzen zu 60 bzw. 80% befallen waren. Die unbehandelten (Kontroll-) Pflanzen waren zu 80% befallen.

### Wirksamkeit gegen Pyricularia oryzae (protektiv)

Blätter von in Töpfen gewachsenen Reiskeimlingen der Sorte "Tai-Nong 67" wurden mit wäßriger Wirkstoffaufbereitung bis zur Tropfnäße besprüht. Am folgenden Tag wurden die Pflanzen einer wäßrigen Sporensuspension von *Pyricularia oryzae* inokuliert. Anschließend wurden die Versuchspflanzen in Klimakammern bei 22 - 24°C und 95 - 99% relativer Luftfeuchtigkeit für 6 Tage aufgestellt. Dann wurde das Ausmaß der Befallsentwicklung auf den Blättern visuell ermittelt.

In diesem Test zeigten die mit 250 ppm der erfindungsgemäßen Verbidnungen I.1, I.2, I.3, I.4, I.5, I.6, I.7, I.8, I.10, I.11, I.14 bis I.17 und I.20 bis I.22 behandelten Pflanzen einen Befall von maximal 15% während die mit der gleichen Menge der Vergleichsverbindungen A.1 bzw. A.2 behandelten Pflanzen zu 80 bzw. 25% befallen waren. Die unbehandelten (Kontroll-) Pflanzen waren zu 80% befallen.

### Beispiele für die Wirkung gegen tierische Schädlinge

Die verbesserte Wirkung der Verbindungen der allgemeinen Formel I gegen tierische Schädlinge ließ sich durch folgende Versuche zeigen.

Die Wirkstoffe wurden
a. als 0,1%-ige Lösung in Aceton oder
b. als 10%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl)
aufbereitet und entsprechend der gewünschten Konzentration mit Aceton im Fall von a. bzw. mit Wasser im Fall von b. verdünnt.

Nach Abschluß der Versuche wurde die jeweils niedrigste Konzentration ermittelt, bei der die Verbindungen im Vergleich zu unbehandelten Kontrollen noch eine 80 bis 100%-ige Hemmung bzw. Mortalität hervorriefen (Wirkschwelle bzw. Minimalkonzentration).

## Patentansprüche

1. Hydroximsäurehalogenide der Formel I 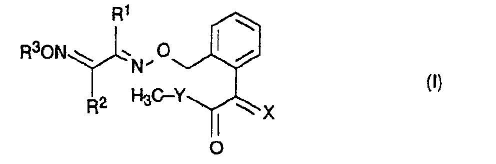 in der die Substituenten die folgende Bedeutung haben:
X NOCH₃, CHOCH₃ oder CHCH₃;
Y O oder NH;
R¹ Halogen;
R² C₂-C₆-Alkyl, C₂-C₆-Alkenyl und C₂-C₆-Alkinyl, wobei diese Gruppen partiell oder vollständig halogeniert sein können und/oder einen oder zwei der folgenden Reste tragen können: Cyano, C₁-C₄-Alkoxy, C₁-C₄-Halogenalkoxy und Phenyl, welches seinerseits partiell oder vollständig halogeniert sein kann und/oder eine bis drei der folgenden Gruppen tragen kann: Cyano, Nitro, C₁-C₄-Alkyl, C₁-C₄-Halogenalkyl, C₁-C₄-Alkoxy und C₁-C₄-Halogenalkoxy;
Methyl, welches partiell oder vollständig halogeniert ist und/oder einen der folgenden Reste trägt: Cyano, C₁-C₄-Alkoxy oder C₁-C₄-Halogenalkoxy;
C₅-C₆-Cycloalkyl, welches partiell oder vollständig halogeniert sein kann und/oder eine bis drei C₁-C₄-Alkylgruppen tragen kann;
Aryl oder Arylmethylen, welches im Arylteil partiell oder vollständig halogeniert sein kann und/oder einen bis drei der folgenden Reste tragen kann: Cyano, Nitro, C₁-C₄-Alkyl, C₁-C₄-Halogenalkyl, C₁-C₄-Alkoxy und C₁-C₄-Halogenalkoxy;
R³ C₁-C₆-Alkyl, C₁-C₃-Alkoxy-C₁-C₆-alkyl, C₃-C₆-Cycloalkyl-C₁-C₆-alkyl, C₃-C₆-Alkenyl und C₃-C₆-Alkinyl, wobei diese Gruppen partiell oder vollständig halogeniert sein können und wobei die Cycloalkylgruppen außerdem eine bis drei C₁-C₄-Alkylreste tragen können,
sowie ihre Salze.

2. Verfahren zur Herstellung der Verbindungen I gemäß Anspruch 1, **dadurch gekennzeichnet, daß** man einen Carbonsäureester der Formel IIa 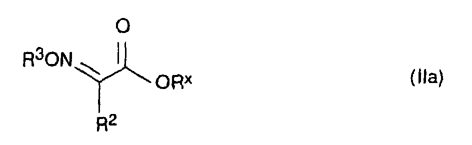 in der R^{x} für einen C₁-C₆-Alkylrest oder einen Phenylrest steht, zunächst mit Hydroxylamin in die entsprechende Hydroxamsäure der Formel IIc 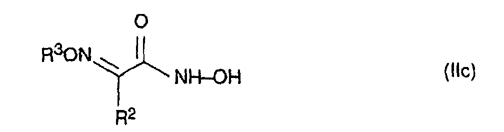 überführt, IIc anschließend mit einer Benzylverbindung der Formel IIIa 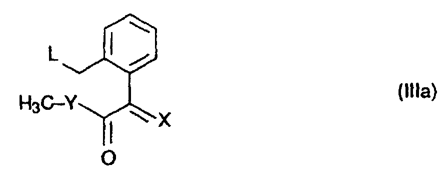 in der L für eine nucleofuge Abgangsgruppe steht, zum entsprechenden Hydroxamsäureester der Formel IV 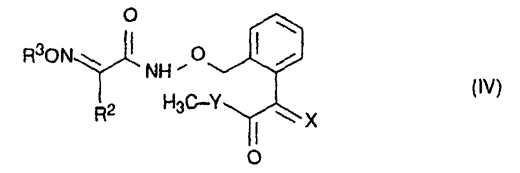 umsetzt und IV mit einem Halogenierungsmittel in I überführt.

3. Verfahren zur Herstellung der Verbindungen IV gemäß Anspruch 2, **dadurch gekennzeichnet, daß** man eine Carbonsäure der Formel IIb 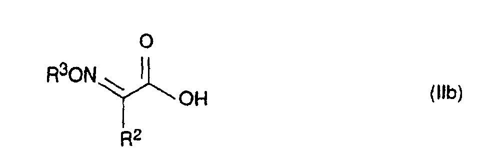 mit einem Benzylhydroxylamin der Formel IIIb 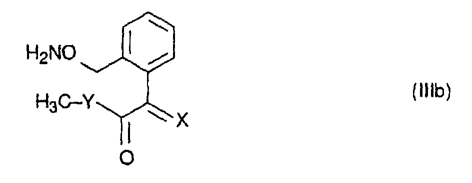 umsetzt.

4. Verfahren zur Herstellung der Verbindungen I gemäß Anspruch 1, **dadurch gekennzeichnet, daß** man ein Amidoxim der Formel IId 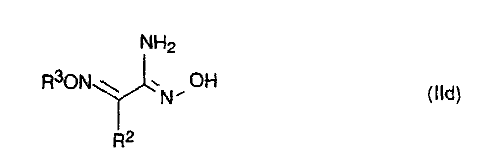 mit einer Benzylverbindung der Formel IIIa gemäß Anspruch 2 in die entsprechende Verbindung der Formel V 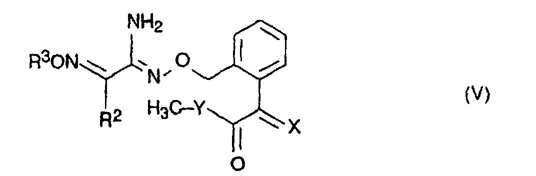 überführt und die Aminogruppe von v im Wege einer Diazotierung gegen Halogen austauscht.

5. Verwendung von Verbindungen der Formel V gemäß Anspruch 4 zur Herstellung der Verbindungen I gemäß Anspruch 1.

6. Zur Bekämpfung von tierischen Schädlingen oder Schadpilzen geeignetes Mittel, enthaltend einen festen oder flüssigen Trägerstoff und eine Verbindung der allgemeinen Formel I gemäß Anspruch 1.

7. Verfahren zur Bekämpfung von Schadpilzen, **dadurch gekennzeichnet, daß** man die Pilze oder die vor Pilzbefall zu schützenden Materialien, Pflanzen, den Boden oder Saatgüter mit einer wirksamen Menge einer Verbindung der allgemeinen Formel I gemäß Anspruch 1 behandelt.

8. Verfahren zur Bekämpfung von tierischen Schädlingen, **dadurch gekennzeichnet, daß** man die Schädlinge oder die vor ihnen zu schützenden Materialien, Pflanzen, den Boden oder Saatgüter mit einer wirksamen Menge einer Verbindung der allgemeinen Formel I gemäß Anspruch 1 behandelt.

9. Verwendung der Verbindungen I gemäß Anspruch 1 zur Herstellung eines zur Bekämpfung von tierischen Schädlingen oder Schadpilzen geeigneten Mittels.

10. Verwendung der Verbindungen I gemäß Anspruch 1 zur Bekämpfung von tierischen Schädlingen oder Schadpilzen.

## Claims

1. A hydroximic acid halide of the formula I 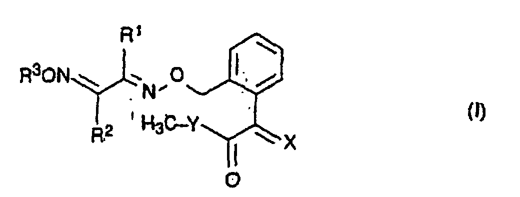 where the substituents have the following meanings:
X is NOCH₃, CHOCH₃ or CHCH₃;
Y is O or NH;
R¹ is halogen;
R² is C₂-C₆-alkyl, C₂-C₆-alkenyl and C₂-C₆-alkynyl, it being possible for these groups to be partially or fully halogenated and/or to have attached to them one or two of the following radicals: cyano, C₁-C₄-alkoxy, C₁-C₄-haloalkoxy and phenyl, it being possible for the phenyl, in turn, to be partially or fully halogenated and/or to have attached to it one to three of the following groups: cyano, nitro, C₁-C₄-alkyl, C₁-C₄-haloalkyl, C₁-C₄-alkoxy and C₁-C₄-haloalkoxy;
methyl which is partially or fully halogenated and/or has attached to it one of the following radicals: cyano, C₁-C₄-alkoxy or C₁-C₄-haloalkoxy;
C₅-C₆-cycloalkyl which can be partially or fully halogenated and/or can have attached to it one to three C₁-C₄-alkyl groups;
aryl or arylmethylene which can be partially or fully halogenated in the aryl moiety and/or can have attached to it one to three of the following radicals: cyano, nitro, C₁-C₄-alkyl, C₁-C₄-haloalkyl, C₁-C₄-alkoxy and C₁-C₄-haloalkoxy;
R³ is C₁-C₆-alkyl, C₁-C₃-alkoxy-C₁-C₆-alkyl, C₃-C₆-cycloalkyl-C₁-C₆-alkyl, C₃-C₆-alkenyl and C₃-C₆-alkynyl, it being possible for these groups to be partially or fully halogenated and it being possible for the cycloalkyl groups additionally to have attached to them one to three C₁-C₄-alkyl radicals,
or a salt thereof.

2. A process for the preparation of a compound I as claimed in claim 1, which comprises first converting a carboxylic ester of the formula IIa 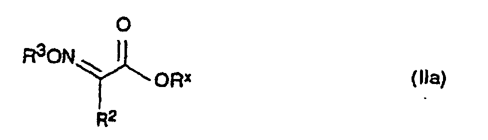 where R^{x} is a C₁-C₆-alkyl radical or a phenyl radical group, with hydroxylamine to give the corresponding hydroxamic acid of the formula IIc 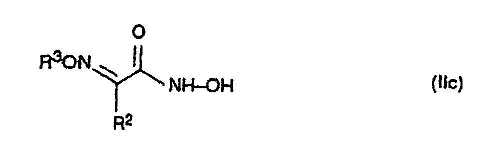 subsequently reacting IIc with a benzyl compound of the formula IIIa 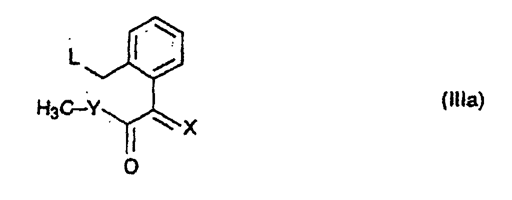 where L is a nutleofugic leaving group, to give the corresponding hydroxamic ester of the formula IV 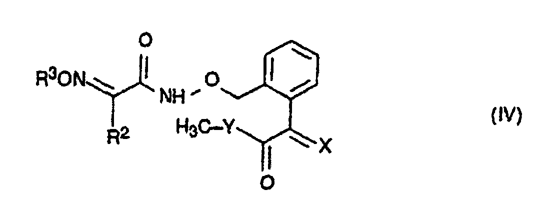 and converting IV with a halogenating agent to give I.

3. A process for the preparation of a compound IV as set forth in claim 2, which comprises reacting a carboxylic acid of the formula IIb 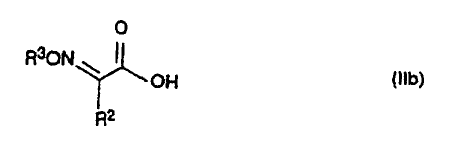 with a benzylhydroxylamine of the formula IIIb

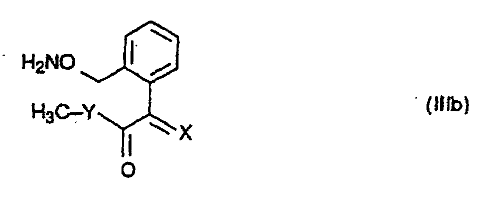

4. A process for the preparation of a compound I as claimed in claim 1, which comprises converting an amidoxime of the formula IId 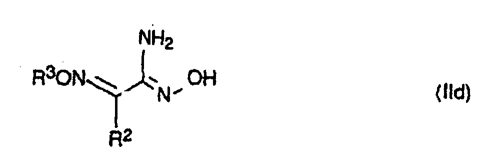 with a benzyl compound of the formula IIIa as set forth in claim 2 to give the corresponding compound of the formula V 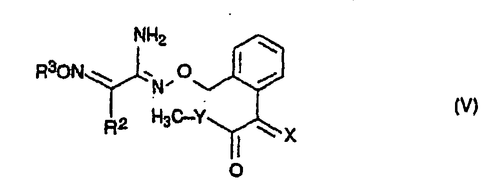 and exchanging the amino group of V for halogen via a diazotization reaction.

5. The use of a compound of the formula V as, set forth in claim 4 for the preparation of a compound I as claimed in claim 1.

6. A composition which is suitable for controlling animal pests or harmful fungi, comprising a solid or liquid carrier and a compound of the formula I as claimed in claim 1.

7. A method of controlling harmful fungi, which comprises treating the fungi, or the materials, plants, soil or seeds to be protected against. fungal infection with an effective amount of a compound of the formula I as claimed in claim 1.

8. A method of controlling animal pests, which comprises treating the pests or the materials, plants, soil or seeds to be protected against the animal pests with an effective amount of a compound of the formula I as claimed in claim 1.

9. The use of a compound I as claimed in claim 1 for the preparation of a composition which is suitable for controlling animal pests or harmful fungi.

10. The use of a compound I as claimed in claim 1 for controlling animal pests or harmful fungi.

## Revendications

1. Halogénures de l'acid hydroximique de formule I 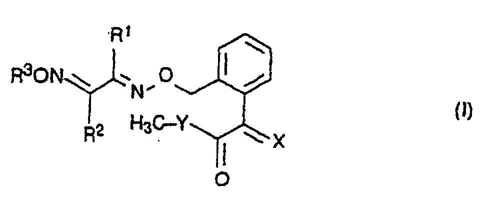 dans laquelle les substituants présentent la signification suivante :
X représente NOCH₃, CHOCH₃ ou CHCH₃ ;
Y représente O ou NH ;
R¹ représente un halogène ;
R² représente un alkyle en C₂-C₆, un alcényle en C₂-C₆ et un alcynyle en C₂-C₆, où ces groupes peuvent être partiellement ou totalement halogènes et/ou peuvent porter un ou deux des radicaux suivants : un cyano, un alcoxy en C₁-C₄, un halogénoalcoxy en C₁-C₄ et un phényle, qui peut être, à son tour, partiellement ou totalement halogéné et/ou peut porter un à trois des groupes suivants : un cyano, un nitro, un alkyle en C₁-C₄, un halogénoalkyle en C₁-C₄, un alcoxy en C₁-C₄ et un halogénoalcoxy en C₁-C₄ ;
un méthyle, qui est partiellement ou totalement halogéné et/ou porte l'un des radicaux suivants : un cyano, un alcoxy en C₁-C₄ ou un halogénoalcoxy en C₁-C₄ ;
un cycloalkyle en C₅-C₆, qui peut être partiellement ou totalement halogéné et/ou peut porter un à trois groupes alkyle en C₁-C₄ ;
un aryle ou un arylméthylène, qui peut être partiellement ou totalement halogéné dans la partie aryle et/ou peut porter un à trois des radicaux suivants : un cyano, un nitro, un alkyle en C₁-C₄, un halogénoalkyle en C₁-C₄, un alcoxy en C₁-C₄ et un halogénoalcoxy en C₁-C₄ ;
R³ représente un alkyle en C₁-C₆, un C₁-C₃-alcoxy-C₁-C₆-alkyle, un C₃-C₆-cycloalkyl-C₁-C₆-alkyle, un alcényle en C₃-C₆ et un alcynyle en C₃-C₆, où ces groupes peuvent être partiellement ou totalement halogénés et où les groupes cycloalkyle peuvent en outre porter un à trois radicaux alkyle en C₁-C₄,
ainsi que leurs sels.

2. Procédé de préparation des composés I selon la revendication 1, **caractérisé en ce qu'**un ester carboxylique de formule IIa 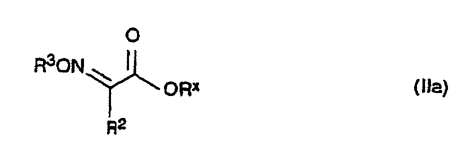 dans laquelle R^{x} représente un radical alkyle en C₁-C₆ ou un radical phényle, est d'abord transformé avec de l'hydroxylamine en l'acide hydroxamique correspondant de formule IIc 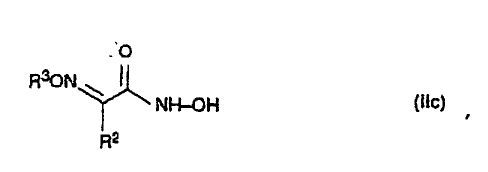 IIc est ensuite mis à réagir avec un composé benzylique de formule IIIa 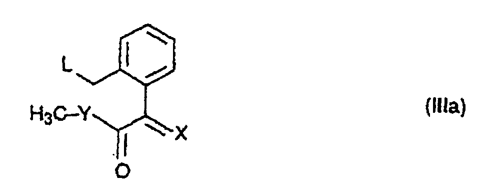 dans laquelle L représente un groupe partant nucléofuge, pour donner lieu à l'ester hydroxamique correspondant de formule IV 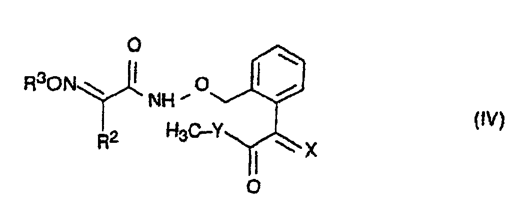 et Iv est transformé en I avec un agent d'halogénation.

3. Procédé de préparation des composés IV selon la revendication 2, **caractérisé en ce qu'**un acide carboxylique de formule IIb 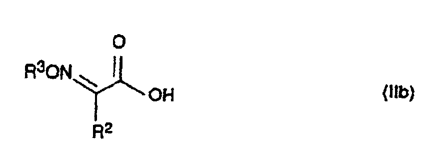 est mis à réagir avec une benzylhydroxylamine de formule IIIb

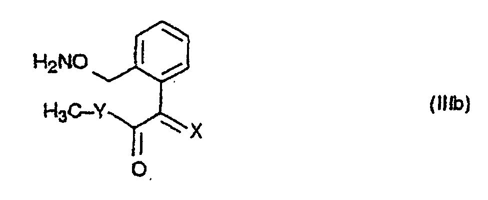

4. Procédé de préparation des composés I selon la revendication 1, **caractérisé en ce qu'**un amide-oxime de formule IId 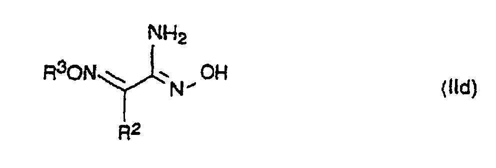 est transformé avec un composé benzylique de formule IIIa selon la revendication 2 en le composé correspondant de formule V 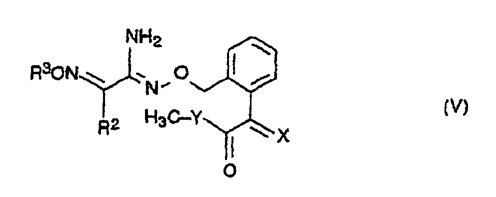 et le groupe amino de V est échangé contre un halogène par l'intermédiaire d'une diazotation.

5. Utilisation de composés de formule V selon la revendication 4 pour la préparation des composés I selon la revendication 1.

6. Composition appropriée pour la lutte contre des parasites animaux ou des champignons nuisibles, comprenant un support solide ou liquide et un composé de formule générale I selon la revendication 1.

7. Procédé de lutte contre des champignons nuisibles, **caractérisé en ce que** les champignons ou les matières, les plantes, les sols ou les semences à protéger vis-à-vis d'une infection fongique sont traités par une quantité efficace d'un composé de formule générale I selon la revendication 1.

8. Procédé de lutte contre des parasites animaux, **caractérisé en ce que** les parasites ou les matières, les plantes, les sols ou les semences à protéger vis-à-vis de ceux-ci sont traités par une quantité efficace d'un composé de formule générale I selon la revendication 1.

9. Utilisation des composés I selon la revendication 1 pour la préparation d'une composition appropriée pour la lutte contre des parasites animaux ou des champignons nuisibles.

10. Utilisation des composés I selon la revendication 1 pour la lutte contre des parasites animaux ou des champignons nuisibles.
